# EUROPEAN PATENT APPLICATION

(11) **EP 4 282 968 A1**
(43) Date of publication of application: **29.11.2023**
(21) Application number: 21920753.7
(22) Date of filing: 01.12.2021
(51) Int. Cl.: C12N 15/85, C12N 15/63, A61K 48/00, A61P 25/16, A61P 25/28, A61P 35/00, A61P 35/02, A61P 43/00

(54) **GENE TRANSCRIPTION FRAMEWORK, VECTOR SYSTEM, GENOME SEQUENCE EDITING METHOD AND APPLICATION**

(30) Priority: 22.01.2021 CN 202110089068
(71) Applicant: Peng, Shuanghong, Beijing 100078 (CN)
(72) Inventor: SUI, Yunpeng, Beijing 100078 (CN); PENG, Shuanghong, Beijing 100078 (CN)
(74) Representative: Grünecker Patent- und Rechtsanwälte PartG mbB
(86) International application number: PCT/CN2021/134710
(87) International publication number: WO 2022/156378

(57) **Abstract**

The invention provides a gene transcription framework, a vector system, a genome sequence editing method and the application thereof. The gene transcription framework is based on a eukaryotic retrotransposition mechanism and can be mediated by a DNA, RNA or RNP pathway, and comprises the transcription production sequence comprising one or more upstream sequence of target site, a sequence to be inserted and a downstream sequence of target site, one or more SINE (element(s)), one or more LINE (element(s)), one or more ORF1p coding sequence(s) and/or one or more ORF2p coding sequence(s). According to the gene editing method, on the premise that foreign systems or substances are not introduced as much as possible and double-strand breaks are not generated, by transferring the gene editing system into nucleus or cytoplasm through a DNA, RNA or RNP pathway (RNA or RNP is transferred from cytoplasm to nucleus through ORF1p and/or ORF2p mediation), the target fragment is inserted into a designated site in a genome or the designated fragment in the genome is deleted or replaced, and high targeting accuracy is achieved.

## Description

### TECHNICAL FIELD

The invention belongs to the technical field of biology, and relates to a gene editing technique, in particular to a gene editing technique mediated by DNA, RNA or RNP pathways and the application thereof.

### BACKGROUND ART

At present, in the field of biotechnique, gene editing techniques mainly include ZFN, TALEN, CRISPR/Cas9 and Targetron techniques. Among them, ZFN technique was first developed in history. However, due to the fact that its DNA binding domain can only recognize sequences with a length of 9bp, its targeting accuracy in practical application is greatly limited. Moreover, the actual design of this technique is tedious, and it is unable to knock out sequence with unknown upstream and downstream sequences. In addition, its cytotoxicity and off-target rate are high. Compared with ZFN, TALEN technique is simpler in design and can recognize 17-18bp sequences with higher specificity. However, since the core technique is controlled by individual commercial companies, most laboratories cannot complete it on their own, which limits its dissemination and application to a certain extent. At the same time, its construction process is still relatively complex. The CRISPR/Cas9 technique is the simplest and most operational of the three. It was first found in archaea and bacteria, and can specifically recognize sequences of about 20bp, causing double-strand breaks at specific sites under the action of Cas9 endonuclease, and repair through the system's own DNA repair function, thereby performing gene editing operations.

However, all three techniques have the so-called off-target problem, and even the CRISPR/Cas9 technique with the highest recognition length is no exception. Studies have shown that the specificity of CRISPR/Cas9 recognition to the target site mainly depends on the pairing of 10-12 bases near the gRNA PAM site, which makes it tend to produce non-specific cleavage. In addition, after the generation of double-strand breaks by these three techniques, there is a high probability that double-strand breaks will be repaired by non-homologous end joining instead of homologous recombination, and random sequences will be generated, whose uncertainty greatly impairs their practical application, especially in human body and clinical application. At the same time, these three techniques bring in genetic materials and proteins that are not part of the acceptance system, which may also have unexpected influences.

Meanwhile, the updated Targetron technique uses group II introns to insert sequences at specific sites in the genome to mutate the corresponding genes. However, this technique will inevitably cause genome double-strand break and introduce exogenous group II introns into the genome to produce "scars". Moreover, since this technique originates from prokaryotes, the RNA generated by itself for reverse transcription has no transmembrane transport function, limiting the application of its RNA to solely perform its functions. In addition, and most importantly, the technique performed well in the field of bacterial gene editing but poorly in higher organisms. All four gene editing techniques must introduce protein and nucleic acid that do not belong to the receiving system, which increases the uncertainty of their effect and greatly hinders their clinical application.

### SUMMARY OF THE INVENTION

In order to solve the above problems, it is an object of the present invention to provide a gene transcription framework that can be transferred into the nucleus or cytoplasm mediated by the DNA, RNA, and/or RNP pathways, insert a target fragment into a specific site in a genome, or delete or replace a specific fragment in a genome, with high targeting accuracy at the same time.

Another object of the present invention is to provide a vector system that can be mediated by the DNA, RNA and/or RNP pathway.

A third object of the present invention is to provide a gene editing method which uses DNA, RNP or RNA (which can be prepared and produced in vitro) and related protein to transfer a target fragment into nucleus or cytoplasm by the DNA, RNA or RNP pathway, insert the target fragment into a designated site in a genome or delete or replace a designated fragment in the genome, and at the same time has high targeting accuracy, on the premise of not introducing a foreign system or substance as much as possible (to human) and not generating double-stranded break.

In order to achieve the above object, the present invention provides a gene transcription framework, characterized in comprising a upstream sequence of target site, a sequence to be inserted, a downstream sequence of target site along a 5'→3' direction;
wherein the gene transcription framework is a segment of DNA sequence which can be transcribed by RNA polymerase I, RNA polymerase II or RNA polymerase III, and
in the transcription product of the gene transcription framework or the transformation product of the transcription product, the upstream sequence of target site or the complementary sequence thereof can hybridize with the upstream sequence or the complementary sequence thereof of the corresponding target site in the cell genome, and the downstream sequence of target site or the complementary sequence thereof can hybridize with the downstream sequence or the complementary sequence thereof of the corresponding target site in the cell genome, and
the upstream sequence of target site and the downstream sequence of target site are directly connected in the corresponding target gene sequence in the genome, and the site between the upstream sequence of target site and the downstream sequence of target site in the target gene sequence in the genome is the target site of the sequence to be inserted.

The above gene transcription framework is use for inserting a sequence to be inserted into a target site of a genome.

Preferably, the cell is a eukaryotic cell.

The invention also provides a vector system comprising one or more vector(s) comprising:
one or more 1) the gene transcription framework according to claim 1, and/or
one or more 2) short interspersed element(s) and/or partial short interspersed element(s) and/or short interspersed-like element(s), and/or
one or more 3) long interspersed element(s) and/or ORF1p coding sequence(s) and/or ORF2p coding sequence(s);
wherein the components 1), 2) and/or 3) are located on the same or different vectors of the vector system; and
the components 1), when more than one exists, are on the same or different vectors of the vector system; and
the components 2), when more than one exists, are on the same or different vectors of the vector system; and
the components 3), when more than one exists, are on the same or different vectors of the vector system,
the vector is provided with one or more promoter(s) which is/are an RNA polymerase I promoter, an RNA polymerase II promoter or an RNA polymerase III promoter, and is/are located upstream of the components 1), 2) and/or 3); and
the vector system is mediated by the DNA, RNA and/or RNP pathway.

Further, the vector is a eukaryotic expression vector, a prokaryotic expression vector, a viral vector, a plasmid vector, an artificial chromosome, a phage vector, or a cosmid vector.

Further, the vector is an expression vector, a cloning vector, a sequencing vector, a transformation vector, a shuttle vector or a multifunctional vector.

Further, when 1) and 2) are located on the same vector, the short interspersed element, and/or the partial short interspersed element, and/or the short interspersed-like element are located downstream of the gene transcription framework, and the gene transcription framework is connected directly or indirectly to the short interspersed element, and/or the partial short interspersed element, and/or the short interspersed-like element; when directly connected, the gene transcription framework shares a promotor with the short interspersed element, and/or the partial short interspersed element, and/or the short interspersed-like element; when indirectly connected, the gene transcription framework shares or does not share a promotor with the short interspersed element, and/or the partial short interspersed element, and/or the short interspersed-like element.

Further, when 1) and 3) are located on the same vector, the one or more long interspersed element(s) and/or one or more ORF1p coding sequence(s), and/or the one or more ORF2p coding sequence(s) are located upstream and/or downstream of the gene transcription framework, and the gene transcription framework is linked directly or indirectly to the one or more long interspersed element(s), and/or the one or more ORF1p coding sequence(s), and/or the one or more ORF2p coding sequence(s); when directly connected, the gene transcription framework shares a promotor with the one or more long interspersed element(s), and/or one or more ORF1p coding sequence(s), and/or one or more ORF2p coding sequence(s); when indirectly connected, the gene transcription framework shares or does not share a promotor with the one or more long interspersed element(s), and/or one or more ORF1p coding sequence(s), and/or one or more ORF2p coding sequence(s).

Further, when 1), 2) and 3) are located on the same vector, the short interspersed element and/or the partial short interspersed element and/or the short interspersed-like element are located downstream of the gene transcription framework and/or downstream of the long interspersed element and/or the ORF1p coding sequence and/or the ORF2p coding sequence; when the short interspersed element and/or the partial short interspersed element and/or the short interspersed-like element are located downstream of the gene transcription framework, the long interspersed element and/or the ORF1p coding sequence and/or the ORF2p coding sequence are located upstream of the gene transcription framework, and/or the long interspersed element and/or the ORF1p coding sequence and/or the ORF2p coding sequence are located downstream of the short interspersed element and/or the partial short interspersed element and/or the short interspersed-like element; when the short interspersed element and/or the partial short interspersed element and/or the short interspersed-like element are located downstream of the long interspersed element and/or the ORF1p coding sequence and/or the ORF2p coding sequence, the long interspersed element and/or the ORF1p coding sequence and/or the ORF2p coding sequence are located downstream of the gene transcription framework; and the gene transcription framework, the short interspersed element and/or the partial short interspersed element and/or the short interspersed-like element, and the long interspersed element and/or the ORF1p coding sequence and/or the ORF2p coding sequence are directly connected or indirectly connected;
when directly connected, the gene transcription framework, the short interspersed element and/or the partial short interspersed element and/or the short interspersed-like element, and the long interspersed element and/or the ORF1p coding sequence and/or the ORF2p coding sequence share a promoter; when indirectly connected, the gene transcription framework, the short interspersed element and/or the partial short interspersed element and/or the short interspersed-like element, and the long interspersed element and/or the ORF1p coding sequence and/or the ORF2p coding sequence share or do not share a promoter.

Further, when 2) and 3) are located on the same vector, the long interspersed element and/or the ORF1p coding sequence and/or the ORF2p coding sequence are located upstream and/or downstream of the short interspersed element and/or the partial short interspersed element and/or the short interspersed-like element, and the short interspersed element and/or the partial short interspersed element and/or the short interspersed-like element are directly connected or indirectly connected to the long interspersed element and/or the ORF1p coding sequence and/or the ORF2p coding sequence, when directly connected, the short interspersed element and/or partially short interspersed element and/or short interspersed-like element share a promoter with the long interspersed element and/or the ORF1p coding sequence and/or the ORF2p coding sequence; when indirectly connected, the short interspersed element and/or partially short interspersed element and/or short interspersed-like element share or do not share a promoter with the long interspersed element and/or the ORF1p coding sequence and/or the ORF2p coding sequence.

In order to improve transcription efficiency, the number of gene transcription framework(s), short interspersed element(s) and/or partial short interspersed element(s) and/or short interspersed-like element(s), long interspersed element(s) and/or ORF1p coding sequence(s) and/or ORF2p coding sequence(s) in a vector system is increased. Short interspersed element(s) and long interspersed element(s) are also naturally present in different species. However, due to the poor activity of the native promoter, transcription with an additional promoter and/or additional transcription of short interspersed element(s) and/or partial short interspersed element(s) and/or short interspersed-like element(s), and/or long interspersed element(s) and/or ORF1p coding sequence(s) and/or ORF2p coding sequence(s) are applied to increase the expression and gene editing efficiency.

The invention also provides a genome sequence editing method, characterized in comprising the steps of:
1) selecting a site to be inserted of a target gene to be edited in a genome, and determining an upstream sequence (upstream sequence of target site) and a downstream sequence (downstream sequence of target site) of the site to be inserted of the target gene on two sides of the site to be inserted;
2) preparing the vector system as described above;
3) transforming or transfecting the vector system into cells, tissues, organisms for transcription.

The invention also provides the use of the vector system in the insertion, deletion and substitution of DNA sequence(s) in any region of the genome.

Preferably, the DNA sequence is one or more CNV sequence(s), CNV terminal sequence(s), short interspersed element(s), partial short interspersed element(s), long interspersed element(s), partial long interspersed element(s), ORF1p coding sequence and/or the ORF2p coding sequence.

When the DNA sequence is a CNV sequence, or a CNV terminal sequence, the CNV terminal (CNV end) can be edited (i.e., between a gene part (gene portion) and a portion of the partial SINE (partial SINE part) in the CNV terminal) to insert a sequence that is not homologous to the genome or partial genome to block gene copy number changes and expression changes thereof; or the partial sequence of the gene portion at the end of the CNV (CNV end, CNV terminal) is deleted to change the expression of the corresponding cell. The CNV terminal (CNV end) consists of a gene part (gene portion) and a portion of the partial SINE (partial SINE part).

The invention also provides the use of the vector system as a medicament for preventing and/or treating cancer, gene related genetic disorder or neurodegenerative diseases.

Preferably, the cancer is a glioma, breast cancer, cervical cancer, lung cancer, stomach cancer, colorectal cancer, duodenal cancer, leukemia, prostate cancer, endometrial cancer, thyroid cancer, lymphoma, pancreatic cancer, liver cancer, melanoma, skin cancer, pituitary tumor, germ cell tumor, meningioma, meningeal carcinoma, glioblastoma, various astrocytomas, various oligodendrogliomas, anaplastic oligodendrogliomas, various ependymomas, choroid plexus papilloma, choroid plexus carcinoma, chordoma, various ganglioneuroma, olfactory neuroblastoma, sympathetic nervous system neuroblastoma, pineal cell tumor, pineal blastoma, medulloblastoma, trigeminal schwannoma, acoustic neuroma, glomus jugular, angioreticuloma, craniopharyngioma, and/or granulosa cell tumor.

Preferably, the gene related genetic disorder is Huntington's disease, fragile X syndrome, phenylketonuria, pseudohypertrophic progressive muscular dystrophy, mitochondrial encephalomyopathy, spinal muscular atrophy, Parkinson plus syndrome, albinism, red-green color blindness, achondroplasia, alkaptonuria, congenital deafness, thalassemia, sickle cell anemia, hemophilia, epilepsy associated with genetic alterations, myoclonus, dystonia, stroke and schizophrenia, vitamin D-resistant rickets, familial colon polyposis, and/or hereditary nephritis.

Preferably, the neurodegenerative disease is Parkinson's disease, Alzheimer's disease, Huntington's disease, amyotrophic lateral sclerosis, spinocerebellar ataxia, multiple system atrophy, primary lateral sclerosis, Pick's disease, frontotemporal dementia, dementia with Lewy bodies and/or progressive supranuclear palsy.

The present invention can prevent the occurrence of the above cancers and their metastatic cancers, inhibit their proliferation and prevent their elevation and progression, or reverse their nature and cure them; prevent, delay or improve drug resistance to insulin, levodopa, various tumor chemotherapy drugs and targeted drugs, delay or stop gene changes and state changes of cells, tissues, organs, embryos or organisms, tissue and organ regeneration and biological regeneration.

By applying the related technique of the invention, the copy number variation (CNV) in each gene and the terminal part thereof (CNV terminal) can be edited to change the terminal position or stabilize the terminal. Since the copy number variation in each gene and the terminal part thereof (CNV terminal) determines the gene expression, changes of the terminal position or stabilization of the terminal can stabilize or change each state of the cells, tissues, organs, embryos and organisms, it can be used in the technical field for modifying genes and states of cells, tissues, organs, embryos and organisms, modifying the genome of organisms such as human to improve functions, modifying the genome of organisms such as human to treat various genetic diseases related to genes such as Huntington's disease and fragile X syndrome, delaying or stopping gene and state changes of cells, tissues, organs, embryos and organisms (such as senescence), changing genes and states of cells, tissues, organs, embryos or organisms, tissue, embryo or organ reconstruction and biological regeneration, assisting reproduction by introducing transcription factor to convert somatic cells into germ cells, preventing or delaying neurodegenerative diseases such as Parkinson's disease, Alzheimer's disease, Huntington's disease, amyotrophic lateral sclerosis, multiple system atrophy, primary lateral sclerosis, spinocerebellar ataxia, Pick's disease, frontotemporal dementia, Lewy body dementia and progressive supranuclear palsy, inhibiting the metabolic activity and proliferation rate and production of tumor cells, delaying their progression and improving their malignancy degree, as well as researches and treatments of all other diseases related to genetic and CNVs changes, such as diabetes, and other physiological, pathological and pathophysiological research.

In the present invention, the sequence to be inserted in the gene transcription framework can be an exogenous sequence or an endogenous sequence, and the length of the one-time insertion sequence is 1 bp to 2000 bp. Insertion of a DNA sequence of any length into genome can be achieved when multiple insertions are performed. The nucleotide sequence length of the upstream sequence of the target site may be between 10 bp and 2000 bp, and the nucleotide sequence length of the downstream sequence of the target site may be between 10 bp and 2000 bp.

The invention is characterized in that,
The invention can position the sequence to be inserted which needs to be inserted into a selected site of the genome on the site to be inserted (target site) of the genome by the upstream and downstream sequences of target sites at two sides of the sequence to be inserted on a vector, and insert the sequence to be inserted into the selected site of the genome under the assistance of the short interspersed element, the long interspersed element and the expressed protein thereof. Furthermore, the ORF2p expressed in cells or by a vector can smoothly slide from the 3' end of the nucleic acid vector to a cleavage site for single-strand cleavage on the genome only under the condition that the upstream sequence of the target site or the complementary sequence thereof on the vector is completely matched with the upstream sequence of the target site or the complementary sequence thereof of the corresponding target site in the cell genome, such that the targeting accuracy is greatly improved, the occurrence of unexpected cleavage is avoided, and the targeting accuracy is theoretically higher than that of the existing gene editing technique.

In addition, the required RNA and the corresponding endogenous proteins such as ORF1p and ORF2p can be produced in vitro, and the target sequence and gene on genome can be edited by RNA or RNP pathway unnecessarily transfecting into nuclei and without introducing DNA fragments. RNA or RNP-mediated pathway can minimize the impact on the receiving system and improve the targeting accuracy while reducing non-specific effect. By virtue of the nuclear localization function of ORF1p and ORF2p and the protection effect of ORF1p on nucleic acid, RNA and protein transfected into cells can be guided into the nucleus, which is conducive to the gene editing of cells difficult to operate due to the difficulty of the vector into the nucleus.

The invention has better targeting, can carry out more accurate target sequence identification and cleavage on the premise of not generating double-strand breaks, can directionally insert desired sequences through homologous recombination, and can delete and substitute corresponding fragments. However, existing gene editing techniques such as CRISPR generate double-strand breaks and unpredictable random mutations, and the techniques are in low probability of target sequence introduction through extremely inefficient homologous recombination. The present technique does not generate double-strand breaks without fear of danger of DNA double-strand breaks and introduction of unexpected random sequences.

The present invention can introduce a long sequence into the genome in a progressive manner by constantly designing a vector for further insertion according to a new site generated after previous insertion, which is also difficult to achieve by the currently known gene editing technique. Similarly, targeted and accurate sequence deletions and sequence substitutions on the genome are difficult to achieve by the prior art but can also be achieved by the present invention. Moreover, operations such as editing or stabilizing CNV or its terminal to change or stabilize gene expression and/or state of cells or organisms cannot be realized by the existing gene editing techniques, but can be realized by the present invention.

The invention has the beneficial effects that:
The invention provides a gene editing method mediated by a DNA, RNA or RNP pathway based on a eukaryotic retrotransposition mechanism. According to the method, through the inherent mechanism of eukaryotes, on the premise of not introducing an exogenous system or substance as far as possible and not generating double-strand breaks, DNA, RNP or RNA (which can be prepared and generated in vitro) and related protein are used as media, and the target fragment (sequence to be inserted) is transferred into nucleus or cytoplasm through a DNA, RNA or RNP pathway, and the target fragment (sequence to be inserted) is inserted into a selected site in a genome or a selected sequence is deleted or substituted in the genome, and high targeting accuracy is achieved simultaneously. Compared with the existing gene editing technique, the invention is easier to apply to clinical practice due to no introduction of external systems such as protein derived from prokaryotes and the like and no generation of double-strand breaks.

### BRIEF DESCRIPTION OF THE DRAWINGS

Fig. 1 is a basic schematic diagram for gene editing based on the retrotransposition mechanisms of eukaryotes.
Fig. 2 is a schematic diagram of DNA-mediated genomic insertion or deletion.
Fig. 3 is a schematic diagram of RNA- and RNP-mediated genomic insertion and deletion.
Fig. 4 is a schematic diagram of blocking CNV terminal changes by inserting a non-homologous sequence at the CNV terminal.
Fig. 5 is a structural schematic diagram of a gene transcription framework provided by the present invention.
Fig. 6 is a structural schematic diagram of a gene transcription framework connecting promoter provided by the present invention.
Fig. 7 is a structural schematic diagram of a gene transcription framework connecting promoter and the short interspersed element, partial short interspersed element or short interspersed-like element provided by the present invention.
Fig. 8 is a structural schematic diagram of an upstream connecting promoter of the gene transcription framework provided by the present invention, and a downstream connecting the long interspersed element or ORF1p coding sequence or ORF2p coding sequence of the gene transcription framework provided by the present invention.
Fig. 9 is a structural schematic diagram of a gene transcription framework provided by the present invention, wherein the gene transcription framework is connected with a long interspersed element or an ORF1p coding sequence or an ORF2p coding sequence in the upstream, and a promotor is connected upstream of the long interspersed element or ORF1p coding sequence or ORF2p coding sequence.
Fig. 10 is a structural schematic diagram of a gene transcription framework provided by the present invention, wherein a promoter is connected upstream of the gene transcription framework, a short interspersed element or a partial short interspersed element or a short interspersed-like element is connected downstream of the gene transcription framework, and then a long interspersed element or an ORF1p coding sequence or an ORF2p coding sequence is connected downstream of the short interspersed element or partial short interspersed element or short interspersed-like element.
Fig. 11 is a structural schematic diagram of a gene transcription framework provided by the present invention, wherein the gene transcription framework is connected with a short interspersed element, a partial short interspersed element or a short interspersed-like element in the downstream, the gene transcription framework is connected with a long interspersed element or an ORF1p coding sequence or an ORF2p coding sequence in the upstream, and the upstream of the long interspersed element or the ORF1p coding sequence or the ORF2p coding sequence is connected with a promoter.
Fig. 12 is a structural schematic diagram of a gene transcription framework provided by the present invention, the gene transcription framework does not share a promoter with the short interspersed element, partial short interspersed element or short interspersed-like element.
Fig. 13 is a structural schematic diagram of a gene transcription framework provided by the present invention, the gene transcription framework does not share a promoter with a long interspersed element or an ORF1p coding sequence or an ORF2p coding sequence.
Fig. 14 is a structural schematic diagram of a gene transcription framework provided by the present invention, the gene transcription framework does not share a promoter with a short interspersed element, a partial short interspersed element or a short interspersed-like element, and a long interspersed element or an ORF1p coding sequence or an ORF2p coding sequence, while the short interspersed element, the partial short interspersed element or the short interspersed-like element, and the long interspersed element or ORF1p coding sequence or ORF2p coding sequence share a promoter.
Fig. 15 is a structural schematic diagram of a gene transcription framework provided by the present invention, the gene transcription framework does not share a promoter with a short interspersed element, a partial short interspersed element or a short interspersed-like element, and a long interspersed element or an ORF1p coding sequence or an ORF2p coding sequence, while the short interspersed element, the partial short interspersed element or the short interspersed-like element, and the long interspersed element or the ORF1p coding sequence or the ORF2p coding sequence share a promoter.
Fig. 16 is a plasmid map of the plasmid pSIL-eGFP-VEGFA1-Alu1 constructed in Example 1 in which the gene transcription framework VEGFA1 was inserted into the vector in Example 1.
Fig. 17 is a plasmid map of plasmid pBS-L1PA1-CH-mneo-IT15-1 constructed in Example 6 in which the gene transcription framework IT15-1 was inserted into the vector in Example 6.

### DETAILED DESCRIPTION OF THE INVENTION

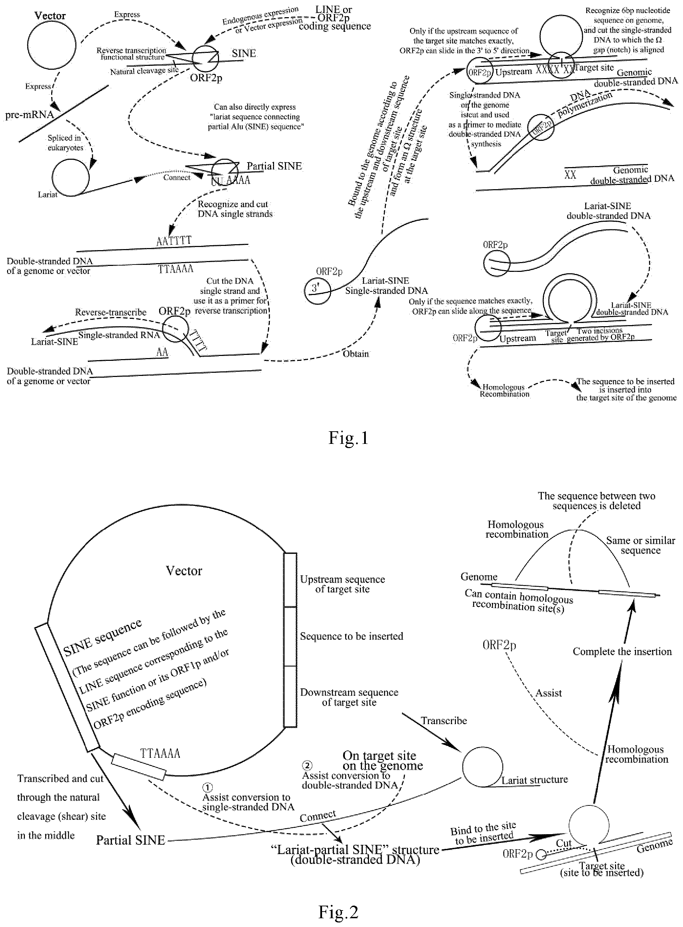

The invention is based on a genome reconstruction mechanism which universally exists in eukaryotes and modifies gene copy numbers, repeat sequences and the like on a genome by transposons. This mechanism may cause deletion or addition of the pathogenic triple nucleotide repeat in some neurodegenerative diseases such as Huntington's disease and fragile X syndrome, and it is consistent with homologous recombination, such as high homology of sequence(s) and being inhibited by methylation, and is related to expression level of corresponding gene(s). As shown in Fig. 1, the invention is related to the short interspersed element (SINE, short interspersed nuclear element), the long interspersed element (LINE, long interspersed nuclear element) and related proteins generated by the long interspersed element such as open reading frame 1 protein (ORF1p), open reading frame 2 protein (ORF2p), and other kinds of open reading frame protein (ORFp). The short interspersed element (SINE) mainly includes Alu element and SVA element in primates, and mammalian-wide interspersed repeat element (MIRs) common to various mammals in mammals, such as MIR and MIR3, Mon-1 in monotremes, B1 element and B2 element in rodents, HE1 family in zebrafish, Anolis SINE2 and Sauria SINE in reptiles, IdioSINE1, IdioSINE2, SepiaSINE, Sepioth-SINE1, Sepioth-SINE2A, Sepioth-SINE2B and OegopSINE in invertebrates such as cuttlefish, and p-SINE1 in plants such as rice. The long interspersed element mainly includes various LINE-1(L1), various LINE-2(L2) and various LINE-3(L3), Ta element(s) and other LINEs in R2, RandI, L1, RTE, I and Jockey of various organisms. These structures widely exist in all kinds of animals and plants and are dispersed throughout the genome. Each organism has its own specific SINE and LINE corresponding to its complementary function. SINE is mainly characterized by a relatively short transposon distributed on the genome, containing an internal RNA polymerase III promoter and ending in an A-or T-rich tail or short simple repeat sequence, and reverse transcription by means of LINE, the right half of whose transcript contains a reverse transcription functional structure. LINE, on the other hand, is characterized by transposons that contain RT (reverse transcriptase)-encoding sequences and are widely distributed in the genome. Both SINE and its corresponding LINE in the corresponding species continuously reconstruct the genome through a similar mechanism. The basic principle of this mechanism is to connect the lariat structure produced by process of pre-mRNA with the right half of the SINE transcript remaining after cleavage carrying the reverse transcription functional structure (the right half with reverse transcription functional structure remaining after cleavage of the complete SINE transcript at the intermediate site is called a partial SINE sequence). The cleavage site of different SINEs in different species is different. The natural cleavage site of SINE is generally located in the middle and front of the whole length. For SINE with a general full length of about 100-400 nt, the natural cleavage site is generally located in the 100th-250th nt. For Alu element with a full length of about 300bp, for example, the cleavage site is located in the 118th nt. For various types of MIRs with a total length of about 260nt, a cleavage site could be observed within the range of 100th to 150th nt. In fact, no matter where the cleavage site is located, as long as after cleavage, the remaining right part contains a complete reverse transcription functional structure (The secondary structure of which forms a special structure, usually in Ω shape. The primary structure of which is characterized by comprising two sequences separated by an intermediate spacer sequence between the two sequences, wherein the two sequences can be combined with complementary sequences of the two sequences on the genome which do not contain the intermediate spacer sequence and the two sequences are directly connected on the genome. LINE-encoded ORF2p can be combined with the sequence located at 3' among the two sequences in the transcript and cleave the genomic single strand at the genomic site corresponding to the gap between the two sequences to initiate reverse transcription), i.e., a partial SINE sequence. The partial SINE produced by a particular SINE, such as an Alu element, is designated as a partial Alu, specifically its intermediate adenylate repeat sequence or together with the 2-3 bases followed by its upstream, and Alu right monomers and the 3' polyA repeat sequence thereafter. In addition, sequences that contain a reverse transcription functional structure and are capable of initiating reverse transcription but are different in sequence from conventional various types of SINEs are called short interspersed-like element (SINE-like). By the protein (i.e., ORF1p and ORF2p) expressed by corresponding types of LINEs (such as LINE-1 corresponding to that function of Alu elements and LINE-2 corresponding to various types of MIR elements, and the like), it can convert RNA into double-stranded DNA and bind to sequences complementary to the double-stranded DNA on a genome (wherein single-stranded DNA generated by reverse transcription of RNA generated by transcription and double-stranded DNA generated by using genomic sequences as primers are transformation products of transcription products), and the insertion into genome is completed through homologous recombination mechanism facilitated by a specific Ω secondary structure of nucleic acid. In addition, LINE can also achieve the above-mentioned similar transformation of RNA into double-stranded DNA and genomic insertion by transcribing its downstream sequence (i.e., 3' transduction) and combining with complementary sequences on the genome to form the Ω structure. Taking Alu element and its corresponding LINE-1 that assists its function as an example: pre-mRNA generated after gene expression can be cut to generate lariats that overlap in sequence, which can occur in any region of pre-mRNA. The difference lies in the strength of the cuts that generate these lariats. Because the cleavage strength (based on sequence differences) of the upstream and downstream lariats of exons is higher than that of other surrounding lariats, the exons can be easily and completely cut off in the process of pre-mRNA, and the production of other lariats is inhibited. At the same time, the ORF1p generated by LINE-1 can protect the nucleic acid bound thereto, which together with the ORF2p generated by LINE (LINE-1) can position the bound nucleic acid to the nucleus and transport it into the nucleus. In addition, ORF2p can bind to the special Ω secondary structure of Alu element transcript and mediate subsequent genomic single-strand cleavage, reverse transcription, and integration into the genome. The transcript of the Alu element can be cleaved at a specific site (multiple displaced loci produced small cytoplast element RNA) (i.e., the scAlu cleavage site or natural cleavage site below, which is generally located in front of the intermediate polyA sequence of the Alu transcript, and may be varied depending on the actual condition), and a small cytoplasmic Alu (scAlu) RNA and the remaining portion containing the right monomer (including a reverse transcription functional structure that can bind to ORF2p) are produced. The remaining portion containing the right monomer is referred to as a partial Alu RNA, the DNA sequence from which the partial Alu RNA is transcribed is referred to as partial Alu sequence (partial Alu). Thereafter, the resultant lariat structure can be ligated from its 3' end to the remainder of the cleaved Alu sequence transcript containing the reverse transcription functional structure, and ORF2p can be recruited via a sequence rich in A (adenylate) and combined with the 3' foot of the Ω structure bipod formed by the partial Alu RNA secondary structure, and identify a sequence on the genome that matches the sequence on the Ω bipod (mainly UU/AAAA on partial Alu RNA, discontinuity between U and A, i.e., where the gap is located). The single strand of the genomic site opposite to the Ω gap is cleaved and the genomic sequence complementary to the sequence of Ω bipod is melted as a primer for reverse transcription. Such process is called target-primed reverse transcription (TPRT). The ORF2p then moves to the 3' end of the generated single-stranded DNA with the reverse transcription, and the generated single-stranded DNA sequence can be combined with a complementary sequence on the genome and form an Ω secondary structure at a corresponding site to be inserted (target site) on the genome (since the sequence to be inserted does not exist at the corresponding site to be inserted (target site) on the genome, while sequences on two sides of the sequence to be inserted on the single-stranded DNA exist at two sides of the site to be inserted (target site) on the genome). The ORF2p can slide along the matched sequence in the direction of 3' to 5' to the Ω structure, identify the genomic sequence with 6 nucleotides complementary to the bottom bipod of Ω structure (mainly four nucleotides of 3' and two nucleotides of 5') and generate double-stranded DNA through the similar process mentioned above. It is noted that only perfectly matched sequences (the upstream sequence of target site or the complementary sequence thereof on generated nucleaic acid with the upstream sequence of target site or the complementary sequence thereof on genome) can slide ORF2p to the cleavage site (i.e., the site on genome corresponding to the gap of Ω structure on generated DNA), which guarantees its targeting accuracy. The finally produced double-stranded DNA is combined with both sides of the corresponding insertion site (target site) (both sequences on two sides are matched) in an Ω shape (Ω structure) again. When the middle of 6 nucleotides (mainly between 4 nucleotides of 3' and 2 nucleotides of 5' recognized by ORF2p) is discontinuous (at the gap of Ω structure of double-stranded DNA), two single-stranded DNA incisions can be created on the gene (genome) corresponding to the gap and the other strand of generated double-stranded DNA through endonuclease action of ORF2p, and the middle circular part of Ω structure is inserted into the genome by means of homologous recombination mechanism. By changing the inserted sequence (the sequence to be inserted), other effects such as deletion or substitution on genome can be achieved through homologous sequence recombination. In the above process, the annealing and deconstruction functions of the ORF1p encoded by LINE can play an auxiliary role in stabilizing the secondary structure generated by the nucleic acid and its binding to the genome in the above genome reconstruction process, and promoting the separation of the nucleic acid from the genome after its binding and action. In addition, ORF1p has a high RNA affinity and a nuclear localization function. Since ORF2p can only cleave one strand of the genomic double strand and cannot produce double-strand break, it has high safety. Similar mechanisms apply to other SINEs and LINEs combinations. Changes in local copy number variations during physiological and pathological processes such as embryonic development and tumorigenesis and insertion of the HIV-1 genome with deletions into the human genome has a preference for short interspersed element sequences are manifestations of this mechanism in nature. It has been reported that the transcribed mRNA sequences could be integrated into the genome with the assistance of ORF1p and ORF2p. But since the transcription template was a purely exogenous non-homologous sequence, it could not be targeted to a specific site in the genome and fragment with a reverse transcription functional structure was not attached, resulting in inefficient and random processes that were difficult to control. According to the invention, the transcription sequence is redesigned, and is connected with sequence(s) containing a reverse transcription functional structure such as various types of SINEs or partial SINEs by various active or passive means, such that a more accurate and efficient gene editing effect is achieved.

Under physiological conditions, copy number variation (CNV) is similar to a copy of a intact gene original. Through the above-mentioned mechanism, CNV that can be continuously extended as a copy according to the intact gene original can make the protein expression and various states of cells, tissues and organisms constantly change. The CNV terminal (i.e., the CNV end) consists of an upstream genetic (gene) part and a downstream partial SINE sequence part, and short sequence fragment(s) contained by lariat is/are continuously inserted between the two parts of CNV terminal through sequence(s) of lariat(s) connecting with partial SINE sequence(s) to extend the CNV. In the early embryonic development, the transcription of LINE is significantly increased, while the genomic SINE such as Alu element sequence shows significant demethylation. While the LINE-mediated 3' transduction (based on the right monomer deletion of the SINE upstream of the corresponding (associated) gene's promoter and the complete SINE structure downstream) initiated the extension of associated gene copy number variation (CNVs), the homologous recombination of the demethylated SINE sequences with each other deletes most of the previously extended CNVs (initialization). Thereafter, the completely initialized embryonic cells regain the hypermethylation state, and the partial SINE sequence at the CNV terminal mediates the gradual extension of the CNV terminal, thereby changing the expression status and state of each cell. In turn, the gene expression status of each cell affects the change of CNVs through the lariats, thus leading to changes in the genome and gradual induction of differentiation. This is consistent with the prevalent changes in CNVs in the embryo and the differences in CNVs in various tissues.

The prolongation of different genes' CNVs (such as oncogenes' CNVs) commonly exists in all kinds of tumor cells and is positively correlated with clinical grade. At the same time, the expression levels of proto-oncogene and tumor suppressor gene are also in direct proportion to the length of CNVs, so the formation and progression of tumors should be related to the disorder of CNVs in proto-oncogene or tumor suppressor gene. In addition, some irreversible diseases related to external stimuli such as diabetes mellitus are also related to the disorders of CNVs. Since most of the drug resistance is associated with changes in the expression of corresponding proteins due to long-term external stimulation, CNV changes of their corresponding genes can be involved, the drug resistance can be improved or hindered by this technique.
**I.** DNA-mediated sequence insertion into genome technique (when gene editing is mediated by DNA, 1-40 TTAAAA or TTTTAA sequences can be added to the plasmid to assist in the transformation of RNA into DNA) (as shown in Fig. 2).
   1. Lariat structure-mediated approach: Upstream and downstream sequences (within 2000bp) of the site to be inserted (i.e., target site) are selected, and the sequence to be inserted (within 2000bp) is added at the intermediate insertion site of the upstream and downstream sequences. The designed sequence is synthesized and integrated into a vector and started to be transcribed by RNA polymerase II. The other regions of the vector are inserted with SINE sequence(s) (0-20, which can be SINEs in corresponding species according to the species of the receiving system to reduce the impact on the receiving system. For example: the SINE sequence can be Alu sequence in primates and Mon-1 sequence in monotremes, and the number is proportional to the efficiency within a certain range. It is also possible to use a non-native SINE, after which LINE or its protein-encoding sequences corresponding to the function of the SINE used must be introduced. See below) and initiated by RNA polymerase II or III alone. After the SINE sequence, a termination signal of a corresponding RNA polymerase is selectively connected (the corresponding LINE sequence or a protein sequence coded by the LINE sequence can be selectively added before the termination signal after the SINE sequence, such as a LINE-1 sequence corresponding to an Alu element in the SINE, or an ORF1p and ORF2p sequence coded by the LINE -1 sequence, a LINE-2 sequence corresponding to an MIR element, or an ORF1p and ORF2p sequence coded by the LINE-2 sequence, and the like, to express protein needed for gene editing so as to realize gene editing or increase editing efficiency) (when transcription is initiated by RNA polymerase II, the corresponding termination signal is a polyA sequence, and the length thereof can be appropriately extended (within 200bp) to increase ORF2p recruitment. Otherwise, polyA sequence with an appropriate length can be added after the ORF1p and ORF2p sequences before the termination signal (within 200bp), and the polyA sequence at the end of the SINE sequence can be appropriately extended (within 200bp)). Thereafter, the vector (other vectors expressing the LINE sequence corresponding to the SINE sequence contained in the vector or the protein sequence encoded by the LINE sequence, such as LINE-1 or ORF2p and/or ORF1p sequence corresponding to Alu sequence, can be simultaneously transfected into the receiving system to increase gene editing efficiency. When the receiving system does not express the above-mentioned protein, such as ORF1P and ORF2p of corresponding LINE, additional expression of the protein is required. In addition, a vector expressing SINE can also be simultaneously transfected into the receiving system, so as to improve the gene editing efficiency) is transferred into cells and tissues cultured in vitro through conventional means such as liposome or virus transfection, and the like, or is administered to organisms through pathways such as blood, lymph, cerebrospinal fluid and the like or local tissues, such that the constructed vector enters the nucleus for expression, and the purpose of inserting a corresponding sequence to be inserted into a corresponding site to be inserted on a genome is completed (If the expected efficiency is not achieved, it might be due to the formation efficiency of the lariat structure comprising the upstream and downstream sequences of the site to be inserted (target site) and the intermediate sequence to be inserted is not high, the length of the upstream and downstream sequences or the sequence to be inserted can be increased or decreased to promote the formation of the lariat structure. Alternatively, a lariat structure containing a site to be inserted (target site) can be detected according to the following detection method, and the sequence or partial sequence of the lariat structure is taken as an upstream sequence and a downstream sequence of target site, the upstream sequence and downstream sequence of target site are appropriately prolonged according to a genome sequence, a sequence to be inserted is placed at the intermediate insertion site (target site) between upstream sequence and downstream sequence of target site forming a gene transcription framework, which can be constructed into a vector, the transfection of this vector can also improve gene editing efficiency.) (The constructed vector can be optionally incubated in a physiological liquid containing ORF1p and/or ORF2p for a short time (at an appropriate temperature, at normal temperature or 37°C, and within 48h) to improve the nuclear localization and import efficiency of the vector). If insertion according to the above method is continued based on the new site generated after insertion before, the sequence insertion can be continuous and a long fragment insertion without significant length limitation into genome can be achieved.
   **2. SINE sequence direct connection approach:** The approach does not need to connect a generated lariat with a cleaved SINE transcript, but directly connects an upstream and downstream sequence of a site to be inserted and a sequence to be inserted in the middle with a SINE related sequence during vector construction, such that the method is suitable for prokaryotes such as bacteria and the like which cannot generate a lariat structure without a eukaryotic pre-mRNA splicing mechanism, and is also suitable for eukaryotes with a pre-mRNA splicing mechanism. This also applies to the following LINE mediated approach. The specific steps are as follows: An upstream sequence and an downstream sequence of site to be inserted (target site) (within 2000bp) and a sequence to be inserted (within 2000bp) between the upstream sequence and downstream sequence form a gene transcription framework, which is started by a promoter of an RNA polymerase II or III, followed by a SINE sequence, a partial SINE sequence or a SINE-like sequence (optionally adding a LINE sequence or a protein coding sequence thereof which can assist the corresponding SINE function after the SINE sequence, the partial SINE sequence or the SINE-like sequence to increase gene editing efficiency; and when the receiving system does not express LINE or its coding protein itself, the coding protein must be added to receiving system or additionally expressed in receiving system) are synthesized. Thereafter, a termination signal of a corresponding kind of RNA polymerase is selectively ligated after a SINE sequence, a partial SINE sequence or a SINE-like sequence (if the termination signal is polyA sequence, the termination signal can be appropriately extended (within 200bp) to increase ORF2p recruitment, or otherwise, polyA sequence with appropriate length (within 200bp) can be added after SINE sequence, partial SINE sequence or SINE-like sequence, or LINE, ORF1p and/or ORF2p sequences before the termination signal to increase ORF2p recruitment), and the sequence is constructed into a vector. Thereafter, the vector is transferred into cells or tissues cultured in vitro by conventional transfection means such as liposomes or virus transfection, and the like, or administered to organisms via pathways such as blood, lymph, cerebrospinal fluid, or local tissues, and the like, and the constructed vector is transferred into nuclei for expression (the constructed vector can be optionally incubated briefly in physiological liquid containing ORF1p and/or ORF2p (at an appropriate temperature, at normal temperature or 37°C, within 48h) to improve the nuclear localization and import efficiency of the vector). The sequence to be inserted is inserted into a corresponding site to be inserted on the genome.
      If the vector constructed as described above is continued for insertion based on the new site generated after insertion before, the sequence insertion can be continuous and a long fragment insertion without significant length limitation into genome can be achieved.
   **3. LINE-mediated approach:** RNA polymerase II starts the expression of LINE or the protein ORF2p and/or ORF1p coding sequence therein, followed by a sequence designed by the same method in SINE sequence direct connection approach (in order to minimize the influence on the receiving system, the SINE and LINE types in the receiving system can be selected. To improve the efficiency, the SINE sequence therein can be selected to be functionally corresponding to the LINE before), and then the termination signal of the RNA polymerase II used can be selectively ligated at last. Thereafter, the vector is transferred into cells or tissues cultured in vitro by conventional transfection means such as liposomes or virus transfection, or administered to organisms via pathways such as blood, lymph, cerebrospinal fluid, or local tissues, and the like, and the constructed vector is transferred into nuclei for expression (the constructed vector can be optionally incubated briefly in physiological liquid containing ORF1p and/or ORF2p (at an appropriate temperature, at normal temperature or 37°C, within 48h) to improve the nuclear localization and import efficiency of the vector), and the sequence to be inserted is inserted into a corresponding site to be inserted on the genome. If insertion according to the above method is continued based on the new site generated after insertion before, the sequence insertion can be continuous and a long fragment insertion without significant length limitation into genome can be achieved.
   **4. Downstream connecting ORF2p binding sequence approach:** The S2 structure formed by a upstream sequence of target site, a downstream sequence of target site and an intermediate sequence to be inserted in a gene transcription framework on a vector when combining with a genome can replace a reverse transcription functional structure in SINE for initialization of reverse transcription, such that an ORF2p binding sequence (e. g., a polyA sequence) capable of binding ORF2p is connected downstream of the downstream sequence of target site in the gene transcription framework. A LINE sequence, an ORF1p and/or an ORF2p coding sequence can be selectively added on the same vector as the gene transcription framework or another vector to improve efficiency. Thereafter, the constructed vector containing the gene transcription framework connected with the downstream ORF2p binding sequence (e.g., a polyA sequence) is transferred into cells or tissues cultured in vitro by conventional transfection means such as liposomes or virus transfection, and the like, or administered to organisms via pathways such as blood, lymph, cerebrospinal fluid, or local tissues, and the like. The constructed vector is transferred into nuclei for expression (optionally, the constructed vector is temporarily incubated in physiological liquid containing ORF1p and/or ORF2p (at an appropriate temperature, or at normal temperature or 37°C, within 48h) to improve the nuclear localization and import efficiency of the vector), and the sequence to be inserted is inserted into a specific site. If insertion according to the above method is continued based on the new site generated after insertion before, the sequence insertion can be continuous and a long fragment insertion without significant length limitation into genome can be achieved.

### II. DNA-mediated genomic sequence deletion technique, as shown in Fig. 2

**1. Deletion of any region on the genome:** The sequence to be inserted in the vector designed as described above in the insertion technique is changed into a certain sequence (within 2000bp) upstream or downstream (within 100000bp) of the insertion site (target site), and the sequence(s) between two identical sequences can be removed with a certain efficiency through homologous recombination after the sequence is inserted through the DNA, RNP or RNA-mediated insertion pathway described in the invention. Sequences containing recombination sites (GCAGA[A/T]C, CCCA[C/G]GAC/ or CCAGC) may be selected for insertion to improve the efficiency of subsequent homologous recombination.
**2. Deletion from CNV terminal:** The CNV terminal in the cell or tissue is detected by sequencing and alignment (alignment of the junction of the gene sequence and a partial SINE sequence). The gene part in the CNV terminal to be treated (within 2000bp) and the 3' part sequence of the lariat that can be formed in a section of the downstream range (within 20,000 bp) of the terminal in the intact gene (the lariat that can be formed downstream can be predicted or detected by the following method) (or the 3' part sequence of the lariat can be substituted by the cut sequence of the downstream sequence of the CNV terminal in a range within 20,000 bp in the intact gene) are respectively selected, and connected with the sequence immediately upstream (within 100000bp) of the sequence to be deleted at CNV terminal. Then the complete SINE sequence, partial SINE sequence or SINE-like sequence (according to the different insertion techniques described above) are ligated after (which can be optionally followed by ORF1p and ORF2p coding sequence). Then the above-described sequence is synthesize and constructed into vector. Then, by one of the above gene insertion techniques, through the DNA, RNA or RNP pathway, the sequence immediately upstream of the sequence to be deleted at CNV terminal is inserted between the gene part and the SINE sequence part (the SINE sequence used on the vector is the same as or close to the SINE sequence around the insertion site to improve efficiency) at the actual CNV terminal. Then, the sequence to be deleted is deleted by homologous recombination between identical sequences. Sequences containing recombination sites (GCAGA[A/T]C, CCCA[C/G]GAC/ or CCAGC) may be selected for insertion to improve the efficiency of subsequent homologous recombination.

### III. DNA-mediated genome sequence substitution technique

The sequence to be inserted in the vector designed in the insertion technique is changed into a substitution sequence and the surrounding sequence of the sequence to be substituted on the genome (i.e., the sequence to be deleted by homologous recombination between the substitution sequence to be inserted and the genomic corresponding sequence. Whether it is located at 3' or 5' of the substitution sequence when constructing the vector depends on whether the insertion site is upstream or downstream of the sequence to be substituted on the genome) (the substitution sequence should be homologous to the sequence to be substituted on the genome). The substitution sequence and the surrounding sequence of the sequence to be substituted on the genome are inserted into the upstream or downstream of the sequence to be substituted on the genome through the gene editing insertion technique. When the inserted substitution sequence and the sequence to be substituted on the genome undergo homologous recombination, the sequence to be substituted on the genome is substituted with the inserted substitution sequence homologous thereto. At the same time, the surrounding sequence of the sequence to be substituted deleted due to homologous recombination is re-inserted into genome together with the substitution sequence during sequence insertion.

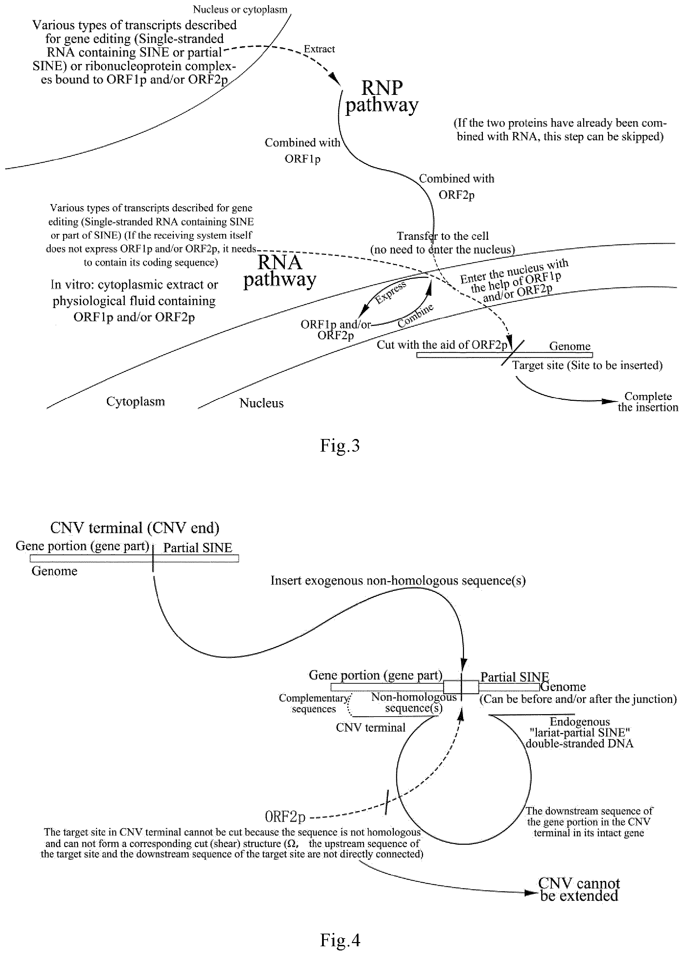

### IV. RNA-mediated genomic sequence editing technique (since RNA-to-DNA conversion is not required, no additional TTAAAA site or TTTTAA site is required to be added), as shown in Fig. 3.

### 1. Ribonucleoprotein (RNP)-mediated pathway:

The synthesized vector as described above is amplified and transferred into an engineering cell line with extra high expression of the protein encoded by LINE (The LINE corresponding to the function of the SINE sequence contained in the synthesized vector can be selected to improve efficiency. For example, if the synthesized vector contains Alu sequence or partial Alu sequence, it corresponds to LINE-1 and its encoding proteins ORF1p and ORF2p.) (An engineering cell permanently or temporarily overexpressing the related protein is obtained by transfection or post-transfection screening with a vector expressing the related protein, wherein the transfected engineering cell does not need to express the related protein if the produced RNA is incubated with the related protein such as ORF1p and ORF2p thereafter). The nuclei and cytoplasm are extracted after a period of time, According to the principle of sequence specificity and the like and applying the corresponding conventional method, a single-stranded plasmid product (single-stranded RNA) or a biologically active ribonucleoprotein (RNP) complex containing the single-stranded plasmid product (single-stranded RNA) is extracted (Alternatively, the extract can be extracted and purified again after being incubated for a second time in the obtained cytoplasm containing ORF1p and/or ORF2p or physiological liquid containing ORF1p and/or ORF2p (at an appropriate temperature, at normal temperature or at 37°C, incubation within 48h). It is noted that RNase inhibitor(s) needs to be added in the in vitro physiological liquid or cytoplasm. If the cells previously transfected the constructed vector do not express related proteins, the extract must be incubated with ORF1p and/or ORF2p). Then, the RNP complex is transferred into cells or tissues cultured in vitro (by transferring into cytoplasm and no need of entering nucleus directly) by conventional transfection means such as liposomes or virus transfection, and the like, or administered to organisms via pathways such as blood, lymph, cerebrospinal fluid, or local tissues, and the like, to complete gene editing. If directional transfer is required, the modification can be carried out on the package outside the vector. Care needs to be taken to avoid RNA degradation throughout the process.

In addition, the application of the vector synthesized in a LINE-mediated approach in the form of RNP requires screening a product containing no front LINE-1 sequence or ORF1p and ORF2p coding sequence from the extracted biologically active ribonucleoprotein (RNP) complex containing a single-stranded plasmid product (single-stranded RNA) (by sequence specificity) (treatment such as an in vitro endonuclease may be additionally added to promote cleavage) to prevent the front sequence from disrupting targeting of gene editing.

### 2. Simple RNA mediated pathway:

As describe above, a sequence containing an upstream and downstream sequence (within 2000bp) of insertion site (i. e., a target site) and an intermediate sequence to be inserted (within 2000bp) at the position corresponding to the insertion site, followed by a SINE sequence, a partial SINE sequence or a SINE-like sequence, followed by a LINE sequence corresponding to the function of the SINE used or protein coding sequence contained thereof (For example, if a partial Alu sequence is used, it corresponds to LINE-1 and ORF1p and ORF2p coding sequence therein. If a partial MIR sequence is used, it corresponds to LINE-2 and the corresponding protein coding sequence therein), is synthesized. The synthesized sequence is constructed into vector, and the transcription is started by an RNA polymerase II/III promoter (or the vector obtained by the various DNA-mediated methods mentioned above can be directly adopted and transferred into engineering cells, and thereafter RNA products usable for gene editing can be extracted by conventional means such as according to sequence specificity). After extraction and purification, the mRNA expressed by the vector is transferred into cells or tissues cultured in vitro (by transferring into cytoplasm and no need of entering nucleus directly) by conventional transfection means such as liposomes or virus transfection, and the like, or administered to organisms via pathways such as blood, lymph, cerebrospinal fluid, or local tissues, and the like, to intact gene editing, such that the purpose of inserting a sequence to be inserted into a corresponding site to be inserted on a genome can be achieved.

If insertion according to the above method is continued based on the new site generated after insertion before, the sequence insertion can be continuous along with cell division and a long fragment insertion without significant length limitation into genome can be achieved (continuous RNA transfer into cells is required).

**V. Hindering the genomic change caused by transposon to stabilize the genome and CNVs thereon** (i.e., inserting a sequence non-homologous to the genome or to the gene part in the CNV terminal and its upstream and downstream sequences in the intact gene between the gene part and the SINE sequence part at the CNV terminal or other regions by the gene editing technique, thereby impeding further extension of CNV. The CNV terminal is defined as the position where the gene sequence is directly connected with a partial SINE sequence, where the gene can be extended, and the specific sequence of the gene part and the partial SINE sequence part of each specific CNV terminal can be obtained by molecular biological means such as gene sequencing or gene chips) (It can be transferred into cells and tissues cultured in vitro by conventional transfection means, such as coating the corresponding vector with fat-soluble substances or substances with cell transfection ability, such as liposomes or viruses, or transferring it into organisms through blood, lymph, cerebrospinal fluid and other pathways or local tissues.) (As shown in Fig. 4)
**1. Intervening a specific CNV** (wherein the upstream sequence used for insertion is the gene part at the CNV terminal of the specific gene): a CNV to be operated is selected, wherein the junction of the 3' end of the gene part and the partial SINE sequence is set as an insertion site (target site), and the upstream sequence of the insertion site in the insertion technique is set as the 3' end of the gene part at the CNV terminal (within 2000bp). The downstream sequence of the insertion site is partial SINE sequence (such that the SINE sequence, partial SINE sequence or SINE-like sequence in the above method connected behind the downstream sequence of the insertion site can be omitted), and the sequence to be inserted is any sequence that is not homologous to the genome, or to a gene part in the CNV terminal, or to an upstream and downstream sequence of the gene part in the CNV terminal in a intact gene (within 2000bp). After the construction of the vector is completed, the vector is transferred into corresponding cells, living tissues or organisms and the like through the DNA, RNA or RNP pathways mentioned above, and the non-homologous sequence is inserted into the corresponding CNV terminal. Since the non-homologous sequence does not exist downstream of the corresponding CNV terminal gene part sequence in the intact gene, no further extension of the CNV terminal according to the intact gene sequence can be performed, thereby blocking further changes in the CNV terminal.
**2. Intervening with a wide range of CNV genomes** (the upstream sequences of the target sites used for insertion must contain all the gene parts at the CNV terminal that may be present):
   **(1) Genome fragmentation sequence method:** cells in organisms, tissues or cell lines to be operated are taken for in vitro culture, or genomes are directly extracted, ultrasonic fragmentation is carried out, and enrichment is carried out through random primers and PCR. A short random sequence (within 20bp) is designed and synthesized, and the partial SINE sequence is connected at the downstream of the short random sequence. The obtained enriched genome fragments and the synthesized short random sequence connecting partial SINE sequence are connected and amplified by PCR to obtain a sequence with different genome fragment sequences connected with the random sequence followed by the partial SINE sequence. The obtained fragment is constructed into a vector, transferred into corresponding cells, living tissues or organisms and the like through the DNA, RNA or RNP pathway, and targets all CNV terminals on a genome through the genome fragment sequence. A non-homologous sequence (i.e., a short random sequence or a partial short random sequence, the portion whereof is not homologous to the gene fragment that is non-homologous to the local gene sequence of the corresponding gene fragment) is inserted between the gene part and the partial SINE part of CNV terminals. Since the non-homologous sequence does not exist downstream of the gene part sequence of the corresponding CNV terminal in its intact gene, further changes in the CNV terminals are blocked.
   **(2) Random sequence method:** A plasmid expressing random sequence with proper length (within 100bp) (including all possible permutations, and combinations that are similar to the SINE sequence can be excluded) and connecting partial SINE sequence after connecting any non-homologous sequence with genome (within 2000bp) is constructed. Alternatively, a plasmid is constructed wherein a random sequence (within 100bp) is connected with the partial SINE sequence wherein any sequence which is not homologous with the genome (within 2000bp) is added at the middle natural cleavage site of the SINE (such as a cleavage site in which a scAlu and a partial Alu can be produced by cleavage for the transcript of Alu element). Alternatively, a vector containing the random sequence followed by any non-homologous sequence with the genome (expressed and the product forms a lariat after that) transcribed by RNA polymerase II can be constructed (the vector needs to contain a SINE sequence or additionally transfer other vectors containing a SINE sequence into the receiving system, and the downstream of the SINE sequence can be connected with or otherwise express a LINE sequence corresponding to the function of SINE or a protein coding sequence thereof). The vector is transferred into corresponding cells, living tissues or organisms through the DNA, RNP or RNA pathways mentioned above, and all CNV terminals on the genome are targeted through random sequences, such that the non-homologous sequences are inserted into corresponding CNV terminals. Since the non-homologous sequence does not exist downstream of the gene part sequence of the corresponding CNV terminal in its intact gene, further changes in the CNV terminals are blocked.
   **(3) According to a lariat terminal sequence method:** All lariat species are detected (A short random sequence (within 100bp) which is not homologous with a genome is inserted into a SINE sequence and the transcript of SINE sequence can still be normally cut into a the partial SINE sequence (i.e., the insertion position of the non-homologous sequence is downstream of the natural cutting site of SINE and not at the natural cutting site). A plasmid capable of expressing the modified SINE sequence is constructed. The plasmid is transferred into the amplified cells taken out from the corresponding organism or cell line of the corresponding species to be operated (the genome of the corresponding species to be tested can also be extracted, the whole genome is cut into fragments with long length (more than 200bp) and certain overlapping with each other (more than 10bp overlapping), and the fragments are constructed into vector and overexpressed by RNA polymerase II in cells in vitro of the corresponding species). After a period of time, the corresponding nucleic acids are specifically extracted through the sequence specificity of the non-homologous sequence inserted into the SINE sequence and sequenced, to obtain sequence information of various generated lariats connected with the partial SINE sequences integrated with the non-homologous sequences.). Alternatively or additionally, according to the sequence rule of pre-mRNA forming lariat, the lariat sequence is predicted (for example, most of them end in AG), and all the lariat sequence information of the specie(s) or individual(s) is obtained. The 3' sequence of all lariats (within 2000bp) is selected, and connected respectively with any sequence non-homologous to the genome (within 2000bp), and integrated into the vector with SINE expression, and then be expressed as lariats (SINE can also be expressed on another vector) (which can be followed by LINE sequence corresponding to SINE function or protein coding sequence thereof to increase efficiency mentioned above). The lariats are connected with partial SINE produced by cell cleavage of SINE transcription product. Alternatively, the 3' sequence of all obtained lariats is respectively connected with any non-homologous sequence with genome (within 2000bp) followed by partial SINE sequence (as mentioned above, which can be followed by LINE sequence corresponding to SINE function or protein coding sequence thereof for increasing efficiency) (the SINE sequence is preferably the same as or similar to the SINE sequence of the gene in which the 3' sequence of lariats is connected with the SINE sequence) and constructed into a vector for expression. The vector is transferred into corresponding cells, tissues or organisms and the like via the DNA, RNP or RNA pathways mentioned above, and the genome-wide CNV terminals are edited.
   **(4) SINE sequence modification method:** by additionally administering the expression of the modified SINE sequence, sequences which are not homologous to the genome or the gene part in the CNV terminal and the upstream and downstream sequences in the intact gene thereof are inserted into each CNV terminal to hinder terminal extension. A vector containing an intact SINE sequence is constructed in which a short sequence is added forehead of the SINE natural cleavage site (which is inconsistent with the conventionally generated 3' sequence of lariats, such as a short sequence spanning the SINE natural cleavage site (within 100bp)) such that the transcription product of SINE can also be naturally cleaved at the added region (LINE sequence corresponding to the function of corresponding SINE species or its protein coding sequence can be added thereafter to increase efficiency). Alternatively, a vector containing an intact SINE sequence in which an arbitrary sequence (within 200bp) non-homologous to the genome is added after the natural cleavage site of SINE transcript is constructed (the LINE sequence corresponding to the function of the corresponding kind of SINE or a protein coding sequence thereof can be added thereafter to increase efficiency), and administered to the corresponding cell, living tissue or organism and the like. The SINE sequence used covers, as far as possible, all SINE sequences of the species or individual (obtainable by methods such as sequencing or array chip and the like) to allow accurate modification of all CNV terminals across the genome.

The whole genome can also be cut into long fragments which are overlapped with each other to a certain extent (the overlapped length is more than the length of a lariat structure), and the long fragments are constructed into a vector to be overexpressed in an in vitro cell line of a corresponding species and generate a lariat structure, and then the vector prepared above for expressing and modifying SINE sequences (if LINE sequences corresponding to the function of SINE of corresponding species or protein coding sequences thereof are added downstream, the method then can be mediated by an RNA pathway) can be transferred into the in vitro cell line transfected with a vector expressing the long fragment. The biologically active single-stranded RNA ribonucleoprotein complex (RNP) or RNA containing the partial SINE (produced by the modified SINE sequence) connected with the generated lariat is extracted and purified by properties such as sequence specificity and conventional means, followed by action of obtained RNP or RNA via the corresponding RNA or RNP pathway.
**3. Modification of SINE element and LINE on genome:** Any sequence (within 500bp) is inserted into a promoter of SINE on genome, natural cleavage site of transcription product or other sequences on SINE and/or promoter of LINE, protein coding sequence of LINE or other sequences of LINE and/or other sequences on genome through the invention, such that SINE sequence on genome cannot be transcribed or cannot be cut naturally after transcription and/or LINE sequence on genome cannot be transcribed or protein thereof with normal function cannot be produced. The method comprises the following steps of: firstly obtaining a sequence of an individual whole genome SINE sequence or LINE sequence to be operated by sequencing, selecting a promoter, a natural cleavage site of the transcription production product, a protein coding sequence and/or other sequences thereon as a site to be inserted (target site), wherein the upstream sequence of target site and downstream sequence of target site in the invention are upstream sequence and downstream sequence of the SINE sequence or LINE sequence relative to the site to be inserted, and the sequence to be inserted is an arbitrary sequence. Any sequence is inserted into the corresponding site on the SINE sequence or LINE sequence on the genome by the insertion technique described above. In addition, the goal can also be achieved by substituting or deleting SINE sequence or LINE sequences on the genome through the above gene editing methods to inactivate them.
**4. Deleting and fixing the CNV terminal:** The CNV terminal to be operated is selected. The junction of the 3' end of the gene part and the partial SINE sequence are set as an insertion site (target site), and the upstream sequence of target site in the insertion techniques are set as the 3' end of the gene part at the CNV terminal (within 2000bp), wherein the downstream sequence of target site is the partial SINE sequence (such that the partial SINE sequence connected behind the downstream sequence in the gene editing method can be omitted). The sequence to be inserted is the sequence (within 2000bp) immediately upstream of the sequence to be deleted (within 100000bp) on the genome followed by any sequence non-homologous to the genome (within 2000bp). After the construction of the vector is completed, the vector is transferred into corresponding cells, living tissues or organisms and the like through the DNA, RNA or RNP pathways mentioned above, such that the sequence immediately upstream of the sequence to be deleted followed by a non-homologous sequence is inserted into corresponding CNV terminal on the genome. When two identical sequences undergo homologous recombination to cause the deletion of an intermediate sequence between the two sequences, the non-homologous sequence simultaneously blocks the further extension of the CNV.
**5. Inhibiting inherent mechanism method:** It can also directly inhibit the CNV extension mechanism inherent in cells or organisms, such as the inhibition of the transcription of SINE sequence or LINE sequence and the like or the production of their RNA and encoding protein proteins such as ORF1p and ORF2p by RNA interference and the like, or hindering the function of proteins related to the CNV extension mechanism by binding specific proteins to the functional structures of proteins related to the CNV extension mechanism, such as ORF1p, ORF2p, or spliceosome, or complexes. By the above gene editing technique and the like, SINEs such as Alu element, various MIRs, and other various SINEs and the like, various LINEs and/or corresponding protein coding sequences thereof in the genome are modified to inactivate or reduce their corresponding activity, functions of related proteins of homologous recombination or mismatch repair mechanism are inhibited, or modified nucleoside is administered to inhibit reverse transcription, such that the effects of inhibiting genome change and stabilizing CNVs are achieved by inhibiting an internal CNV extension mechanism.

As SINE, LINE and their expressed proteins are widely distributed in eukaryotes, gene editing operations can be performed on a wide range of eukaryotes through this technique. In addition, it can also be applied to the treatment of diseases with gene changes and to change or stabilize the state of cells or organisms and the like associated with gene changes.

In the present invention, a definite sequence or site (e.g., a sequence to be insert) is defined along the direction 5'→3', upstream is before the 5' end of the definite sequence or site, downstream is after the 3' end of the definite sequence or site, upstream sequence is before the 5' end of the definite sequence or site and downstream sequence is after the 3' end of the definite sequence or site.

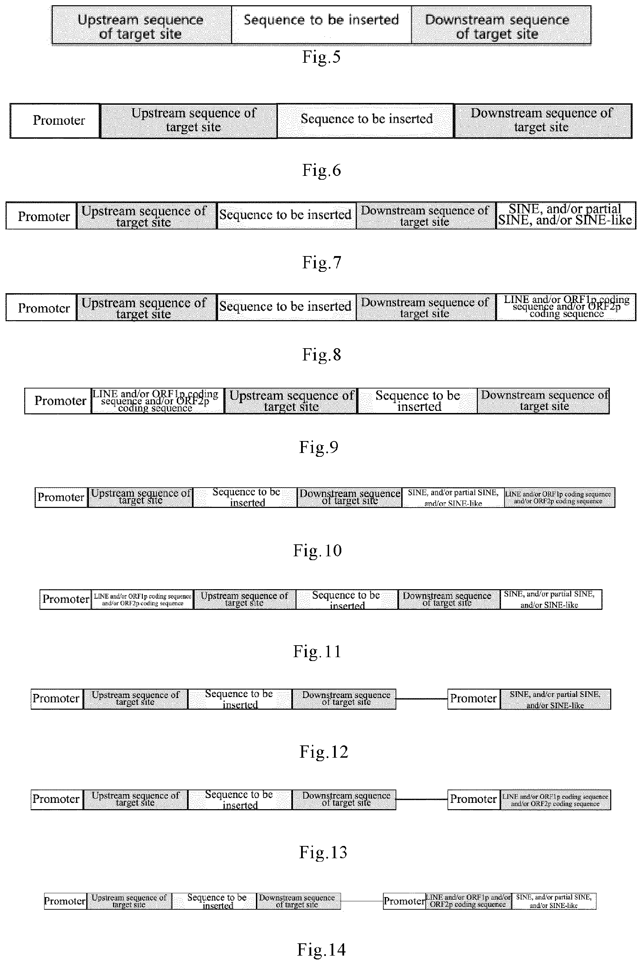

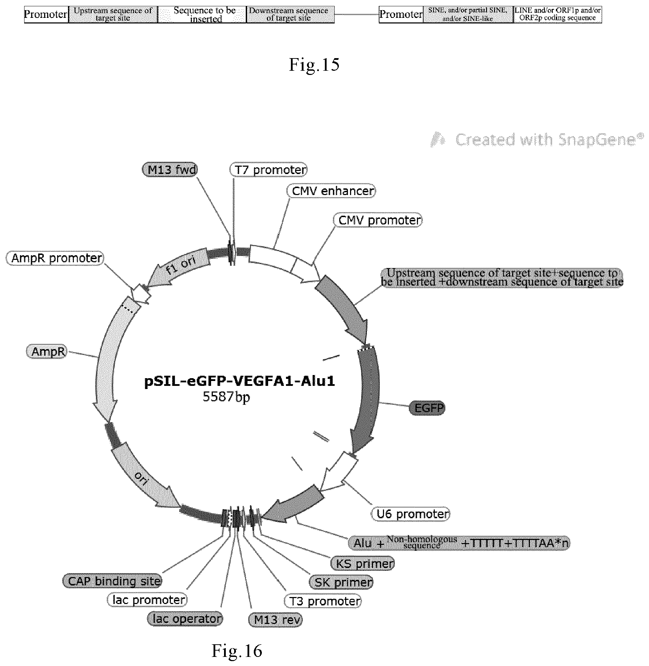

The gene transcription framework provided by the invention, as shown in Fig. 5, comprises a upstream sequence of target site, a sequence to be inserted and a downstream sequence of target site along the 5' to 3' direction. In order to better understand the positional relationship between the gene transcription framework and the short interspersed element, and the long interspersed element, and the promoter, several different connecting forms are listed for understanding. As shown in Fig. 6, there is a schematic structural diagram showing the promoter attached to the front of the gene transcription framework. The promoter may be an RNA polymerase I promoter, an RNA polymerase II promoter, an RNA polymerase III promoter. The promoter can be located on a vector, and a gene transcription framework, short interspersed element, and/or long interspersed element, etc. is/are inserted into the downstream of the promoter through an enzyme cutting site of the vector, and expressed after being transfected into cells. Alternatively, the promoter can be directly synthesized with the gene transcription framework, the short interspersed element, the long interspersed element, and the like by a direct synthesis method and inserted into the vector. Fig. 7 is a structural schematic diagram of a gene transcription framework connected downstream of the promoter and connected upstream of the short interspersed element, partial short interspersed element or short interspersed-like element. Fig. 8 is a structural schematic diagram of an upstream connecting promoter of the gene transcription framework, and a downstream connecting the long interspersed element or ORF1p coding sequence or ORF2p coding sequence. Fig. 9 is a structural schematic diagram of a gene transcription framework, wherein the gene transcription framework is connected with a long interspersed element or an ORF1p coding sequence or an ORF2p coding sequence in the upstream, and a promotor is connected upstream of the long interspersed element or ORF1p coding sequence or ORF2p coding sequence. Fig. 10 is a structural schematic diagram of a gene transcription framework, wherein a promoter is connected upstream, a short interspersed element or a partial short interspersed element or a short interspersed-like element is connected downstream, and then a long interspersed element or an ORF1p coding sequence or an ORF2p coding sequence is connected downstream. Fig. 11 is a structural schematic diagram of a gene transcription framework, wherein the gene transcription framework is connected with a short interspersed element, a partial short interspersed element or a short interspersed-like element in the downstream, a long interspersed element or an ORF1p coding sequence or an ORF2p coding sequence is connected with the upstream of the gene transcription framework, and a promoter is connected with the upstream of the long interspersed element or the ORF1p coding sequence or the ORF2p coding sequence. Fig. 12 is a structural schematic diagram of a gene transcription framework, not sharing a promoter with the short interspersed element, partial short interspersed element or short interspersed-like element. Fig. 13 is a structural schematic diagram of a gene transcription framework, not sharing a promoter with a long interspersed element or an ORF1p coding sequence or an ORF2p coding sequence. Fig. 14 is a structural schematic diagram of a gene transcription framework, not sharing a promoter with a short interspersed element, a partial short interspersed element or a short interspersed-like element, and a long interspersed element or an ORF1p coding sequence or an ORF2p coding sequence, while the short interspersed element, the partial short interspersed element or the short interspersed-like element, and the long interspersed element or ORF1p coding sequence or ORF2p coding sequence share a promoter, and the short interspersed element, the partial short interspersed element or the short interspersed-like element is located downstream of the long interspersed element or ORF1p coding sequence or ORF2p coding sequence. Fig. 15 is a structural schematic diagram of a gene transcription framework, not sharing a promoter with a short interspersed element, a partial short interspersed element or a short interspersed-like element, and a long interspersed element or an ORF1p coding sequence or an ORF2p coding sequence, while the short interspersed element, the partial short interspersed element or the short interspersed-like element, and the long interspersed element or the ORF1p coding sequence or the ORF2p coding sequence share a promoter, and the short interspersed element, the partial short interspersed element or the short interspersed-like element is located upstream of the long interspersed element or ORF1p coding sequence or ORF2p coding sequence. All of the above are the forms of the gene transcription framework on the same vector as short interspersed element, partial short interspersed element or short interspersed-like element and long interspersed elements, ORF1p coding sequence and/or ORF2p coding sequence; It can also be located on different vectors and co-transfected into cells for expression by different promoters.

In the following Examples, the SINE used is a primate-specific short interspersed element Alu element due to the material used being human derived cells. The complete sequence of the Alu element is shown in Seq ID No.1, and a partial Alu sequence is shown in Seq ID No.2. When that material used is of another species, the short interspersed element may be replaced with a short interspersed element of the corresponding specie to facilitate expression, and maybe the gene editing efficiency.

### Material

1. pSIL-eGFP plasmid vector was purchased from Addgene, Plasmid 52675. pBS-L1PA1-CH-mneo plasmid vector was purchased from Addgene, Plasmid 51288.
2. 10× enzyme digestion buffer (required for NheI enzyme digestion): 330 mM Tris-acetate, 100 mM magnesium acetate, 660mM potassium acetate, 1 mg/mL BSA. 10× enzyme digestion buffer (required for SalI restriction enzyme digestion): 500mM Tris-HCl, 100mM MgCl₂, 1000mM NaCl, 1mg/mL BSA.
3. Restriction endonucleases NheI and SalI were purchased from ThermoFisher.
4. T4 DNA ligase and 10×ligation buffer for its application were purchased from Promega.
5. Entranster-H4000 transfection reagent was purchased from Beijing Engreen Biosystem, Ltd.
6. The blood/cell/tissue genomic DNA extraction kit was purchased from Tiangen Biochemical Technology (Beijing) Co., Ltd., with product catalog number of DP304.
7. SuperReal PreMix Plus (SYBR Green) was purchased from Tiangen Biochemical (Beijing) Co., Ltd., with product catalog number of FP205.
8. The magnetic bead method tissue/cell/blood total RNA extraction kit was purchased from Tiangen Biochemical Technology (Beijing) Co., Ltd., with product catalog number of DP761.
9. The FastKing cDNA first strand synthesis kit was purchased from Tiangen Biochemical Technology (Beijing) Co., Ltd., with product catalog number of KR116.
10. TIANSeq mRNA capture kit is from Tiangen Biochemical Technology (Beijing) Co., Ltd., product catalog number: NR105.
11. Lipofectamine^{™} MessengerMAX^{™}mRNA transfection reagent was purchased from ThermoFisher.
12. Trypsase was purchased from Sigma-Aldrich under catalog number T1426.
13. Dnase I was purchased from Tiangen Biotech (Beijing) Co., Ltd., catalog No. RT411.
14. Chemical synthesis of primers and sequences was completed by Biosune Biotechnique (Shanghai) Co., Ltd.

### Example 1 DNA-mediated insertion of exogenous sequence to be inserted into designated sites of the genome

The vascular endothelial growth factor *(VEGFA)* is a member of the PDGF/VEGF growth factor family. It encodes a heparin-binding protein that exists as a disulfide-linked homodimer. This growth factor plays a role in angiogenesis, endothelial cell growth, inducing endothelial cell proliferation, promoting cell migration, inhibiting apoptosis, and inducing vascular permeability, which are necessary for both physiological and pathological angiogenesis.

In this Example, exogenous sequences were inserted into that *VEGFA* gene to confirm the DNA-mediated genomic sequence insertion technique of the present invention.

A 459-bp sequence of the gene *VEGFA* in the human genome was selected, and the sequence is shown in Seq ID No.3: ATTATGCGGATCAAACCTCACCAAGGCCAGCACATAGGAGAGATGAGCTT CCTACAGCACAACAAATGTGAATGCAGGTGAGGATGTAGTCACGGATTCA TTATCAGCAAGTGGCTGCAGGGTGCCTGATCTGTGCCAGGGTTAAGCATGC TGTACTTTTTGGCCCCCGTCCAGCTTCCCGCTATGTGACCTTTGGCATTTTA CTTCAATGTGCCTCAGTTTCTACATCTGTAAAATGGGCAC*AATAGTAGTA TACTTCATAGCATTGTTATAATGATTAAACAAGTTATATATGAAAAGATTA AAACAGTGTTGCTCCATAATAAATGCTGTTTTTACTGTGATTATTATTGTTG TTATCCCTATCATTATCATCACCATCTTAACCCTTCCCTGTTTTGCTCTTTTC TCTCTCCCTACCCATTGCAGACCAAAGAAAGATAGAGCAAGACAAGAAAA, wherein * was a selected insertion site (target site). The sequence upstream of the insertion site in the *VEGFA* gene (upstream sequence of target site) was before the insertion site, and the sequence downstream of the insertion site in the *VEGFA* gene (downstream sequence of target site) was after the insertion site. A non-homologous sequence which was designed randomly was added at an insertion site as a sequence to be inserted to form a gene transcription framework, and restriction endonuclease NheI enzyme cutting sites and protective bases (overhangs) were added at two ends of gene transcription framework in order to enable the gene transcription framework to be inserted into an expression vector. The complete sequence is shown in Seq ID No.4: ***CTAGCTAGCTAG***ATTATGCGGATCAAACCTCACCAAGGCCAGCACATAGG AGAGATGAGCTTCCTACAGCACAACAAATGTGAATGCAGGTGAGGATGTA GTCACGGATTCATTATCAGCAAGTGGCTGCAGGGTGCCTGATCTGTGCCAG GGTTAAGCATGCTGTACTTTTTGGCCCCCGTCCAGCTTCCCGCTATGTGAC CTTTGGCATTTTACTTCAATGTGCCTCAGTTTCTACATCTGTAAAATGGGCA CGCTACGGAATAAGAGGAGGCCACAACAGTCGTGGGTCGAATAGTAGTAT ACTTCATAGCATTGTTATAATGATTAAACAAGTTATATATGAAAAGATTAA AACAGTGTTGCTCCATAATAAATGCTGTTTTTACTGTGATTATTATTGTTGT TATCCCTATCATTATCATCACCATCTTAACCCTTCCCTGTTTTGCTCTTTTCT CTCTCCCTACCCATTGCAGACCAAAGAAAGATAGAGCAAGACAAGAAAA***C TAGCTAGCTAG,*** wherein, the underlined part was a randomly designed exogenous non-homologous sequence (i.e., a sequence to be inserted), the length was 38bp, and two ends of the sequence were NheI enzyme cutting sites and protective bases (bold italic). The sequence was obtained by chemical synthesis and named as VEGFA1. The exogenous sequence to be inserted was designed as a sequence that was non-homologous to that sequence of the *VEGFA* gene so that insertion at a target site on its genome could be specifically detect in later experiments.

At the same time, the short sequences of the upstream sequence and the downstream sequence of the insertion site in the *VEGFA* gene were added before and after the insertion site on the vector to verify the effect of the upstream sequence of target site and the downstream sequence of target site with different lengths on the insertion effect.

10bp before and 10 bp after the insertion site of the *VEGFA* sequence were selected to design the sequence with a non-homologous sequence, NheI enzyme cutting sites and a protective bases into a short sequence, as shown in Seq ID No.5: ***CTAGCTAGCTAG***AAATGGGCACGCTACGGAATAAGAGGAGGCCACAACAG TCGTGGGTCGAATAGTAGTA***CTAGCTAGCTAG*.** Among them, the underlined part represented a randomly designed exogenous non-homologous sequence (i.e., a sequence to be inserted) with a length of 38bp, NheI enzyme cutting site and protective base (bold italic) at both ends of the sequence, and the sequence was obtained by chemical synthesis and named as VEGFA2.

The selection of restriction enzyme cutting site was only for the convenience of plasmid construction and could be changed according to different vectors.

VEGFA1 and VEGFA2 were inserted into plasmid vector pSIL-eGFP, respectively, and plasmids pSIL-eGFP-VEGFA1 and pSIL-eGFP-VEGFA2 were constructed, wherein the specific process was as follows:
Enzyme digestion was performed on VEGFA1, VEGFA2 and plasmid vector pSIL-eGFP, respectively. The reaction system is shown in Table 1.

**Table 1 Enzyme digestion reaction system**

| Component | Addition Amount |
|---|---|
| VEGFA1 or VEGFA2 or pSIL-eGFP | 1µg |
| NheI | 1µL |
| 10 x restriction enzyme buffer | 5µL |
| ddH2O | Make up to 50µL |
| Total | 50µL |

The reaction conditions were as follows: incubation at 37°C for 1h, followed by heating to 65°C for 20min to inactivate the endonuclease, electrophoresis and recovery of the digestion product.

The enzyme-cleaved VEGFA1 or VEGFA2 was connected to the enzyme-cleaved linear plasmid vector pSIL-eGFP, respectively, and the reaction system is shown in Table 2:

**Table 2 ligation reaction system**

| Component | Addition Amount |
|---|---|
| VEGFA1 or VEGFA2 | 20ng |
| pSIL-eGFP | 100ng |
| T4 DNA ligase | 1 Weiss unit |
| 10× ligation buffer | 1µL |
| Nuclease-free water | Make up to 10µL |

The reaction conditions were as follows: incubation at 16°C for 16h, followed by heating to 70°C for 10min to inactivate the ligase, followed by electrophoresis and purification to obtain the plasmid pSIL-eGFP-VEGFA1 and the plasmid pSIL-eGFP-VEGFA2. The plasmid was verified to be correct by sequencing.

Since the pSIL-eGFP plasmid itself carried the CMV promoter, transcription could be initiated by RNA polymerase II once the gene transcription framework was inserted into the CMV promoter.

Alu expression sequence was designed, by connecting Alu sequence, non-homologous sequence (18bp), TTTTT, and TTTTAA * n together, wherein n is 6, and adding SalI enzyme cutting site and corresponding protective base at two ends of that sequence to obtain the sequence shown in Seq ID No.6: ***ACGCGTCGACGTCGGCCATAGCGGCCGCGGAA***GGGCCGGGCGCGGTGGCT CACGCCTGTAATCCCAGCACTTTGGGAGGCCGAGGCGGGCGGATCACGAG GTCAGGAGATCGAGACCATCCCGGCTAAAACGGTGAAACCCCGTCTCTAC TAAAAATACAAAAAATTAGCCGGGCGTGGTGGCGGGCGCCTGTAGTCCCA GCTACTCGGGAGGCTGAGGCAGGAGAATGGCGTGAACCCGGGAGGCGGA GCTTGCAGTGAGCCGAGATCACGCCGCTGCACTCCACCCTGGGCGACAGA GCGAGACTCCGTCTCAAAAAAAAAAAAAAAAAAAAAAAAAAAAAAGATT AATAACTGCTGGAGATCCTATCAGGCGAGCCCGTATTTTTTTTTAATTTTA ATTTTAATTTTAATTTTAATTTTAA***ACGCGTCGACGTCGGCCATAGCGGCCG CGGAA,*** wherein italic at two end are SalI enzyme cutting site and corresponding protective base. The upstream SalI cutting site and the corresponding protective base were followed by Alu sequence, and the underlined part was the non-homologous sequence (18bp) added after Alu sequence for marking Alu sequence so as to facilitate the detection of the connection of its transcription product with the lariat generated by transcription of the gene transcription framework (including the sequence to be inserted) in later experiments to verify the action mechanism of the experiment. The wavy line was the terminator of transcription, and the double underlined part was the six TTTTAA repeat sequences for converting RNA into DNA, which belong to the common repeat sequences in the genome, and the repeat sequence can be added more than one or none. In this embodiment, six repeat sequences were optionally added. The sequence, named Alu1, was obtained by chemical synthesis.

Alu1, plasmid pSIL-eGFP- VEGF A 1 and plasmid pSIL-eGFP-VEGFA2 were digested with SalI, respectively. The digestion reaction system was shown in Table 3.

**Table 3 Enzyme digestion reaction system**

| Component | Addition Amount |
|---|---|
| Alu1 or pSIL-eGFP-VEGFA1 or pSIL-eGFP-VEGFA2 | 1µg |
| SalI | 1µL |
| 10× enzyme digestion buffer | 2µL |
| ddH2O | Make up to 20µL |
| Total | 20µL |

The reaction conditions were as follows: incubation at 37°C for 3h, followed by heating to 80°C for 10min to inactivate the endonuclease, followed by electrophoresis, and recovery of the digestion product.

The enzyme-cleaved Alul was ligated with the enzyme-cleaved linear plasmid pSIL-eGFP-VEGFA1 and plasmid pSIL-eGFP-VEGFA2, respectively, and the reaction system is shown in Table 4.

**Table 4 ligation reaction system**

| Component | Addition Amount |
|---|---|
| Alu1 | 20ng |
| pSIL-eGFP-VEGFA1 or pSIL-eGFP-VEGFA2 | 100ng |
| T4 DNA ligase | 1 Weiss unit |
| 10× ligation buffer | 1µL |
| Nuclease-free water | Add to 10µL |

The reaction conditions were as follows: incubation at 16°C for 16h, followed by incubation at 70°C for 10min to inactivate the ligase, followed by electrophoresis and recovery, to obtain the plasmids pSIL-eGFP-VEGFA1-Alu1 (as shown in Fig. 16) and pSIL-eGFP-VEGFA2-Alu1. The plasmid was verified to be correct by sequencing.

Since the pSIL-eGFP plasmid itself carries the U6 promoter, which was a promoter dependent on RNA polymerase III, the transcription of Alu sequence (Alu element) could be initiated by RNA polymerase III once the Alu sequence was inserted after the U6 promoter.

The pSIL-eGFP-VEGFA1-Alu1 or pSIL-eGFP-VEGFA2-Alu1 was transfected into Hela cells to test the insertion efficiency of the randomly designed exogenous sequence. In order to improve the insertion efficiency, pBS-L1PA1-CH-mneo, a plasmid expressing ORF1p and ORF2p (LINE), was co-transfected into Hela cells, and the corresponding control group was designed. The co-transfected plasmid in that experimental group and the control group are shown in Table 5.

**Table 5 Grouping**

| Group | Co-transfected plasmid |
|---|---|
| Control group 1 | pSIL-eGFP+pBS-L1PA1-CH-mneo |
| Experimental group 1 | pSIL-eGFP-VEGFA1-Alu1+pBS-L1PA1-CH-mneo |
| Experimental group 2 | pSIL-eGFP-VEGFA1+pBS-L1PA1-CH-mneo |
| Experimental group 3 | pSIL-eGFP-VEGFA2-Alu1+pBS-L1PA1-CH-mneo |
| Experimental group 4 | pSIL-eGFP-VEGFA1-Alu1 |

As shown from the grouping, the control group 1 was co-transfected with original pSIL-eGFP and pBS-L1PA1-CH-mneo, which did not contain gene transcription framework sequence and Alul sequence. The experimental group 1 was co-transfected with pSIL-eGFP-VEGFA1-Alu1 and pBS-L1PA1-CH-mneo, which contained gene transcription framework containing long sequence upstream and downstream of the target site of *VEGFA* gene, Alul sequence and pBS-L1PA1-CH-mneo. In experimental group 2, pSIL-eGFP-VEGFA1 and pBS-L1PA1-CH-mneo were co-transfected, which included a gene transcription framework containing long sequence upstream and downstream of target site on the *VEGFA* gene, and pBS-L1PA1-CH-mneo, but did not include an Alul sequence. In experimental group 3, pSIL-eGFP-VEGFA2-Alu1 and pBS-L1PA1-CH-mneo were co-transfected, including the gene transcription framework containing the upstream and downstream short sequences of target site of *VEGFA* gene, Alul sequence and pBS-L1PA1-CH-mneo; In experimental group 4, pSIL-eGFP-VEGFA1-Alu1 was transfected without pBS-L1PA1-CH-mneo, which contained the gene transcription framework with long sequence upstream and downstream of the target site on the *VEGFA* gene and Alul sequence, but did not contain pBS-L1PA1-CH-mneo. Three parallels were set in each group, and each parallel was a 6-well plate into which Hela cells were cultured.

The transfection steps were as follows: Hela cells were passaged and spread into 6-well plates. On the next day of passage, transfection was performed using Entranster-H4000 transfection reagent. For transfection of each plate of cells, 48µg or 96µg (according to the experimental grouping, 48 µg if only one plasmid was transfected; If the two plasmids were co-transfected, 48µg of each plasmid, and a total of 96µg of two plasmids, were used) constructed plasmid was diluted with 300µL serum-free DMEM and mixed thoroughly. At the same time, 120µL of Entranster-H4000 reagent was diluted with 300µL of serum-free DMEM, and after fully mixed, it was allowed to stand for 5min at room temperature. Then the prepared two liquids were mixed and fully mixed, and allowed to stand for 15min at room temperature to prepare the transfection complex. The transfection complex was added to 6-well plate with Hela cells, in which contained 2ml DMEM containing 10% fetal bovine serum per well, for transfection. After the cells grew to about 90% confluence, they were passaged, and the above operations were repeated after passage. After the cells grew to about 90% confluence again, samples were taken for subsequent operations.

Extraction of transfected cell DNA: After the cell culture medium was aspirated away, the cells were rinsed twice with PBS, digested with an appropriate amount of 0.25% trypsin, and digested at 37°C for 20min, with 15 times of pipetting every 5min. After the cells were suspended, complete medium containing serum was added to stop the reaction (digestion). Thereafter, extraction of cellular DNA was performed according to the product instruction of the blood/cell/tissue genomic DNA extraction kit, and the DNA concentration was determined by an ultraviolet spectrophotometer.

### qPCR detection:

Since the *GAPDH* gene do not contain an Alu sequence and its copy number is stable, the *GAPDH* gene is used as an reference gene.

The upstream primer sequence for detecting the *GAPDH* gene is shown in Seq ID No.7 as follows: 5' -CACTGCCACCCAGAAGACTG-3'. The downstream primer sequence is shown in Seq ID No.8: 5'-CCTGCTTCACCACCTTCTTG-3'.

A primer pair 1 and a primer pair 2 were designed, wherein an upstream primer sequence of the primer pair 1 is shown as Seq ID No.9: 5'-CCCAGGGTTGTCCCATCT-3'; and the downstream primer sequence is shown in Seq ID No. 10: 5'-CCTCCTCTTATTCCGTAGC-3'. The upstream primer sequence of primer pair 1 is located in the complete *VEGFA* gene, further upstream of the upstream sequence of the insertion site (target site) used on the plasmid, not in the plasmid, but only in the genome, and the downstream primer sequence of primer pair 1 is located in the 19bp sequence at the 5' end of the randomly designed non-homologous sequence to be inserted (the sequence to be inserted). The upstream sequence of the primer pair 2 is shown in Seq ID No. 11: 5'-CACAACAGTCGTGGGTCG-3'; The downstream primer sequence is shown in Seq ID No. 12: 5'-GAGGGAGAAGTGCTAAAGTCAG-3'. The upstream primer sequence of primer pair 2 is located at the 18bp sequence at the 3' end of the randomly designed non-homologous sequence to be inserted (the sequence to be inserted), the downstream primer sequence is located in the complete *VEGFA* gene, further downstream from the downstream sequence of the insertion site (target site) used on the plasmid, not in the plasmid, but only in the genome.

The primers were all obtained through chemical synthesis.

The qPCR reaction system is shown in Table 6.

**Table 6 qPCR reaction system**

| Component | Addition Amount |
|---|---|
| Upstream primer (10µM) | 1.5µL |
| Downstream primer (10µM) | 1.5µL |
| 2×SuperReal PreMix Plus | 25µL |
| Cellular DNA template (25ng/µL) | 4µL |
| ddH2O without RNase | Make up to 50µL |

The cellular DNA template was DNA extracted from the aforementioned control group 1, and experimental group 1 to 4 after co-transfection or transfection.

The reaction system was prepared on ice, put the lid of the reaction tube on after preparation, mixed gently, and centrifuged briefly to ensure that all the components were at the bottom of the tube. Each 6-well plate cell sample was repeated 3 times simultaneously.

### qPCR reaction cycle:

Primer pair 1: pre-denaturation at 95°C for 15min; (denaturation at 95°C for 10s, annealing at 50°C for 20s, and extension at 72°C for 20s) 40 cycles. The GAPDH primers were reacted under the same conditions.

Primer pair 2: pre-denaturation at 95°C for 15min; (denaturation at 95°C for 10s, annealing at 54°C for 20s, and extension at 72°C for 20s) 40 cycles. The GAPDH primers were reacted under the same conditions.

The exponential growth phases in the amplification curves of GAPDH and the detection of the insertion of the sequence to be inserted were observed and were confirmed to be approximately parallel, the obtained data were analyzed by the 2^{-ΔΔCt} method, and the results are shown in Table 7 and Table 8, where Table 7 is the result of primer pair 1 and Table 8 is the result of primer pair 2. The PCR products were verified to be correct by sequencing.

**Table 7 Results of primer pair 1 (n=3, x̅±s)**

| Ct value | *GAPD H* | Inserti on of the sequen ce to be inserte d | ΔCt | ΔΔCt (mean ΔCt of the experimental group - mean ΔCt of the control group 1) | Relative amount of copy number (2^{-ΔΔCt}) |
|---|---|---|---|---|---|
| Control group 1 plate 1 | 25.63 | N/A | 14.37 | - | - |
| Control group 1 plate 2 | 25.21 | N/A | 14.79 | - | - |
| Control group 1 plate 3 | 26.28 | N/A | 13.72 | - | - |
| Control group 1 mean | | | 14.29±0.44 | 0.00±0.44 | 1.00 (0.74-1.36) |
| Experimental group 1 plate 1 | 26.12 | 25.93 | -0.19 | - | - |
| Experimental group 1 plate 2 | 25.54 | 25.42 | -0.12 | - | - |
| Experimental group 1 plate 3 | 25.89 | 25.97 | 0.08 | - | - |
| Experimental group 1 mean | | | -0.08±0.11 | -14.37±0.11 | 21173.91(19619.49-22851.48) |
| Experimental group 2 plate 1 | 25.87 | 38.92 | 13.05 | - | - |
| Experimental group 2 plate 2 | 26.33 | 39.16 | 12.83 | - | - |
| Experimental group 2 plate 3 | 25.94 | 38.47 | 12.53 | - | - |
| Experimental group 2 mean | | | 12.80±0.21 | -1.49±0.21 | 2.81 (2.43-3.25) |
| Experimental group 3 plate 1 | 26.75 | 39.34 | 12.59 | - | - |
| Experimental group 3 plate 2 | 25.91 | 38.99 | 13.08 | - | - |
| Experimental group 3 plate 3 | 25.68 | 38.94 | 13.26 | - | - |
| Experimental group 3 mean | | | 12.98±0.28 | -1.31±0.28 | 2.48 (2.04-3.01) |
| Experimental group 4 plate 1 | 25.92 | 33.21 | 7.29 | - | - |
| Experimental group 4 plate 2 | 25.71 | 32.65 | 6.94 | - | - |
| Experimental group 4 plate 3 | 26.34 | 33.12 | 6.78 | - | - |
| Experimental group 4 mean | | | 7.00±0.21 | -7.29±0.21 | 156.50 (135.30-181.02) |

Compared with other groups (N/A was calculated according to 40.00), the relative amount of copy number of Experimental group 1 was significantly higher than those of other groups, with statistical significance (P < 0.05), suggesting that gene editing is the most efficient with the longer upstream or downstream sequence of the insertion site (target site) in the gene transcription framework and the adequate expression of Alu element (SINE), ORF1p and ORF2p (LINE). The relative amounts of copy number in Experimental group 2, 3, and 4 were higher than that in Control Group 1 (N/A was calculated according to 40.00) and all results had statistical significance (P < 0.05), suggesting that gene editing was still effective but less efficient under the conditions of shorter upstream or downstream sequence of the insertion site (target site) in the gene transcription framework, low or no expression of Alu element (SINE) of the cell itself, or low or no expression of ORF1p and ORF2p (LINE). A sequence of sufficient length on both sides of the insertion site (target site) in the gene transcription framework is required to facilitate the formation of the lariat in order to ensure efficient insertion. Longer upstream and/or downstream sequence(s) of target site(s), or additional expression of ORF1p, ORF2p (LINE) and/or Alu element (SINE) can improve editing efficiency.

**Table 8 Results of primer pair 2 (n=3, x̅±s)**

| Ct value | *GAPD H* | Inserti on of the sequen ce to be inserte d | ΔCt | ΔΔCt (mean ΔCt of the experimental group - mean ΔCt of the control group 1) | Relative amount of copy number (2^{-ΔΔCt}) |
|---|---|---|---|---|---|
| Control group 1 plate 1 | 25.43 | N/A | 14.57 | - | - |
| Control group 1 plate 2 | 25.77 | N/A | 14.23 | - | - |
| Control group 1 plate 3 | 25.98 | N/A | 14.02 | - | - |
| Control group 1 mean | | | 14.27±0.23 | 0.00±0.23 | 1.00 (0.85-1.17) |
| Experimental group 1 plate 1 | 25.12 | 25.30 | 0.18 | - | - |
| Experimental group 1 plate 2 | 25.84 | 25.61 | -0.23 | - | - |
| Experimental group 1 plate 3 | 25.36 | 25.76 | 0.40 | - | - |
| Experimental group 1 mean | | | 0.12±0.26 | -14.15±0.26 | 18179.19 ( 15181.22-21769.19) |
| Experimental group 2 plate 1 | 26.54 | 39.02 | 12.48 | - | - |
| Experimental group 2 plate 2 | 25.89 | 38.75 | 12.86 | - | - |
| Experimental group 2 plate 3 | 26.30 | 39.43 | 13.13 | - | - |
| Experimental group 2 mean | | | 12.82±0.27 | -1.45±0.27 | 2.73 (2.27-3.29) |
| Experimental group 3 plate 1 | 25.35 | 38.19 | 12.84 | - | - |
| Experimental group 3 plate 2 | 25.09 | 38.48 | 13.39 | - | - |
| Experimental group 3 plate 3 | 25.47 | 38.75 | 13.28 | - | - |
| Experimental group 3 mean | | | 13.17±0.24 | -1.10±0.24 | 2.14 (1.82-2.53) |
| Experimental group 4 plate 1 | 25.74 | 33.34 | 7.60 | - | - |
| Experimental group 4 plate 2 | 26.03 | 32.82 | 6.79 | - | - |
| Experimental group 4 plate 3 | 25.29 | 33.37 | 8.08 | - | - |
| Experimental group 4 mean | | | 7.49±0.53 | -6.78±0.53 | 109.90 (76.11-158.68) |

Compared with other groups (N/A was calculated according to 40.00), the relative amount of copy number of Experimental group 1 was significantly higher than those of other groups, with statistical significance (P < 0.05), suggesting that gene editing is the most efficient with the longer upstream or downstream sequence of the insertion site (target site) in the gene transcription framework and the adequate expression of Alu element (SINE), ORF1p and ORF2p (LINE). The relative amounts of copy number in Experimental group 2, 3, and 4 were higher than that in Control group 1 (N/A was calculated according to 40.00) and all results had statistical significance (P < 0.05), suggesting that gene editing was still effective but less efficient under the conditions of shorter upstream or downstream sequence of the insertion site (target site) in the gene transcription framework, low or no expression of Alu element (SINE) of the cell itself, or low or no expression of ORF1p and ORF2p (LINE). A sequence of sufficient length on both sides of the insertion site (target site) in the gene transcription framework is required to facilitate the formation of the lariat in order to ensure efficient insertion. Longer upstream and/or downstream sequence(s) of target site(s), or additional expression of ORF1p, ORF2p (LINE) and/or Alu element (SINE) can improve editing efficiency.

According to the results in Table 7 and Table 8, both ends of the sequence to be inserted are inserted into the site to be inserted (target site) on genome, which means that the sequence to be inserted was completely inserted into the site to be inserted (target site). Experimental group 1, 2, 3 and 4 could insert non-homologous sequence into the genome completely, and experimental group 1 has the highest efficiency.

### Example 2 Detect the connection between the lariat structure formed by the gene transcription framework (containing exogenous sequence to be inserted) and the RNA fragment containing partial SINE sequence (e.g., Alu element) (the transcript of Alu element cut at the natural splicing site)

1.Extraction of transfected cell total RNA of the experimental group 1 and the control group 1 in Example 1: After transfection, the cell culture medium was aspirated away, then the cells were rinsed twice with PBS, digested with an appropriate amount of 0.25% trypsin, and digested at 37°C for 20min, with 15 times of pipetting every 5min. After the cells were suspended, complete medium containing serum was added to stop the reaction (digestion). Thereafter, the solution containing cells was transferred to a RNase-free centrifuge tube, centrifuged at 300 × g for 5min, the sediment was collected and all the supernatant was aspirated away. Extraction of cellular total RNA was performed according to the product instruction of the magnetic bead method tissue/cell/blood total RNA extraction kit.
2.Synthesis of cDNA template by reverse transcription: The genomic DNA in the extracted total RNA was removed according to the instruction of the FastKing cDNA first strand synthesis kit, and then the cDNA was synthesized, and the concentration of the synthesized cDNA was determined by ultraviolet spectrophotometer.
3.qPCR detection:
   The upstream primer and downstream primer used in detection of *GAPDH* gene as internal reference are shown in Seq ID No.7 and Seq ID No.8.

The primer pair 3 was designed to detect the linkage between the transcriptional lariat structure containing the exogenous sequence to be inserted and the RNA fragment containing partial Alu sequence produced by the transcriptional product of the Alu element. The upstream primer sequence is shown in Seq ID No. 11: 5'-CACAACAGTCGTGGGTCG-3', and the upstream primer is located on the exogenous sequence to be inserted; The downstream primer sequence is shown in Seq ID No. 13: 5'-TACGGGCTCGCCTGATAG-3'. The downstream primer is located at the non-homologous sequence (18bp) after the Alu sequence constructed into the plasmid.

The primers were all obtained through chemical synthesis.

The qPCR reaction system is shown in Table 9.

**Table 9 qPCR reaction system**

| Component | Addition Amount |
|---|---|
| Upstream primer (10µM) | 1.5µL |
| Downstream primer (10µM) | 1.5 µL |
| 2 × SuperReal PreMix Plus | 25µE |
| cDNA template (25ng/µL) | 8µL |
| ddH₂O without RNase | Make up to 50µL |

The reaction system was prepared on ice, put the lid of the reaction tube on after preparation, mixed gently, and centrifuged briefly to ensure that all the components were at the bottom of the tube. Each 6-well plate cell sample was repeated 3 times simultaneously.

### qPCR reaction cycle:

Pre-denaturation at 95°C for 15min; (denaturation at 95°C for 10s, annealing at 54°C for 20s, and extension at 72°C for 20s) 40 cycles. The GAPDH primers were reacted under the same conditions.

The exponential growth phases in the amplification curves of the detection of GAPDH and the linkage between the lariat structure containing the sequence to be inserted and the RNA fragment containing partial Alu were observed and were confirmed to be approximately parallel, the obtained data were analyzed by the 2^{-ΔΔCt} method, and the result is shown in Table 10. The PCR products were verified to be correct by sequencing.

**Table 10 Results of primer pair 3 (n=3, x̅±s)**

| Ct value | *GAPDH* | linkage betwee n the lariat structu re contain ing the sequen ce to be inserte d and the RNA fragme nt contain ing partial Alu sequen ce | ΔCt | ΔΔCt (mean ΔCt of the experimental group - mean ΔCt of the control group 1) | Relative amount of copy number (2^{-ΔΔCt}) |
|---|---|---|---|---|---|
| Control group 1 plate 1 | 18.74 | N/A | 21.26 | - | - |
| Control group 1 plate 2 | 19.56 | N/A | 20.44 | - | - |
| Control group 1 plate 3 | 19.17 | N/A | 20.83 | - | - |
| Control group 1 mean | | | 20.84±0.33 | 0.00±0.33 | 1.00 (0.80-1.26) |
| Experimental group 1 plate 1 | 19.53 | 29.49 | 9.96 | - | - |
| Experimental group 1 plate 2 | 18.82 | 28.21 | 9.39 | - | - |
| Experimental group 1 plate 3 | 18.67 | 28.58 | 9.91 | - | - |
| Experimental group 1 mean | | | 9.75±0.26 | -11.09±0.26 | 2179.83 ( 1820.35-2610.30) |

Compared with Control Group 1 (N/A was calculated according to 40.00), the relative amount of copy number of Experimental group 1 was significantly higher, with statistical significance (P < 0.05), suggesting that the lariat structure containing the exogenous sequence to be inserted was indeed connected with the transcript of Alu sequence (that is, the RNA fragment containing partial Alu).

It can be seen from Table 10 that transcript of Alu sequence (i.e. RNA fragment containing partial Alu) was indeed connected with the transcript containing the sequence to be inserted.

### Example 3. DNA-mediated insertion of exogenous sequence to be inserted into designated sites of the genome

The *MMP2* gene, a member of the matrix metalloproteinase (MMP) gene family, is a zinc-dependent enzyme capable of cleaving components of the extracellular matrix and molecules involved in signal transduction. The protein encoded by this gene is a collagenase A, type IV collagenase, which contains three fibronectin type II repeats at its catalytic site, allowing denatured type IV and V collagen and elastin to bind. Unlike most MMP family members, activation of this protein can occur on cell membranes. This enzyme can be activated extracellular by proteases or intracellular via S-glutathione without the need for proteolysis to remove the protodomain. This protein is thought to be involved in a variety of pathways, including roles in the nervous system, endometrial menstrual rupture, vascularization regulation, and metastasis. Mutations in this gene are associated with Winchester syndrome and nodular arthropathy osteolysis (NAO) syndrome. Alternative splicing results in the encoding of multiple transcriptional variants of different subtypes.

In this Example, exogenous sequences were inserted into that *MMP2* gene to confirm the DNA-mediated genomic sequence insertion technique of the present invention.

A 479-bp sequence of the gene *MMP2* in the human genome was selected, and the sequence is shown in Seq ID No. 14: AGCATGGCGATGGATACCCCTTTGACGGTAAGGACGGACTCCTGGCTCAT GCCTTCGCCCCAGGCACTGGTGTTGGGGGAGACTCCCATTTTGATGACGAT GAGCTATGGACCTTGGGAGAAGGCCAAGGTGAGAAAGGGGCCCTCTGCAT GCCCCAGACCTTCTCTCCTGTCCTCTCTCCACTCCATTTGCTTGGACCAGAG AG*GTGGGAGGGGAGGAAAGTCACACATCTGGGTGAGTCAGAATCTTGGT CTCCAAAGAAGGCCTGGAGAAGTCCAACCTCCCCCTTCCATGTCACTCTTT AGTGGTCCGTGTGAAGTATGGGAACGCCGATGGGGAGTACTGCAAGTTCC CCTTCTTGTTCAATGGCAAGGAGTACAACAGCTGCACTGATACCGGCCGCA GCGATGGCTTCCTCTGGTGCTCCACCACCTACAACTTTGAGAAGGATGGCA AGTACGGCTTCTGTCCCCATGAAG, wherein * was a selected insertion site (target site). The sequence upstream of the insertion site in the *MMP2* gene (upstream sequence of target site) was before the insertion site, and the sequence downstream of the insertion site in the *MMP2* gene (downstream sequence of target site) was after the insertion site. A non-homologous sequence which was designed randomly was added at an insertion site as a sequence to be inserted to form a gene transcription framework, and restriction endonuclease NheI enzyme cutting sites and protective bases (overhangs) were added at two ends of gene transcription framework in order to enable the gene transcription framework to be inserted into an expression vector. The complete sequence is shown in Seq ID No. 15: ***CTAGCTAGCTAG***AGCATGGCGATGGATACCCCTTTGACGGTAAGGACGGA CTCCTGGCTCATGCCTTCGCCCCAGGCACTGGTGTTGGGGGAGACTCCCAT TTTGATGACGATGAGCTATGGACCTTGGGAGAAGGCCAAGGTGAGAAAGG GGCCCTCTGCATGCCCCAGACCTTCTCTCCTGTCCTCTCTCCACTCCATTTG CTTGGACCAGAGAGCCTGTGGGCCTTGCTCAGAGCGGAGAAAGCATGGCA TAATGATGTGGCTGTTTTGTTTGTACAAGATCCGCAGACGTGTAAATGTTC CTGCAAAAACACAGACGTGGGAGGGGAGGAAAGTCACACATCTGGGTGA GTCAGAATCTTGGTCTCCAAAGAAGGCCTGGAGAAGTCCAACCTCCCCCTT CCATGTCACTCTTTAGTGGTCCGTGTGAAGTATGGGAACGCCGATGGGGA GTACTGCAAGTTCCCCTTCTTGTTCAATGGCAAGGAGTACAACAGCTGCAC TGATACCGGCCGCAGCGATGGCTTCCTCTGGTGCTCCACCACCTACAACTT TGAGAAGGATGGCAAGTACGGCTTCTGTCCCCATGAAGCTA GCTA GCTA G, wherein, the underlined part was a randomly designed exogenous non-homologous sequence (i.e., a sequence to be inserted), the length was 103bp, and two ends of the sequence were NheI enzyme cutting sites and protective bases (bold italic). The sequence was obtained by chemical synthesis and named as MMP2-1. The exogenous sequence to be inserted was designed as a sequence that was non-homologous to that sequence of the *MMP2* gene so that insertion at a target site on its genome could be specifically detect in later experiments.

At the same time, the short sequences of the upstream sequence and the downstream sequence of the insertion site in the *MMP2* gene were added before and after the insertion site on the vector to verify the effect of the upstream sequence of target site and the downstream sequence of target site with different lengths on the insertion effect.

10bp before and 10 bp after the insertion site of the *MMP2* sequence were selected to design the sequence with a non-homologous sequence, NheI enzyme cutting sites and a protective bases into a short sequence, as shown in Seq ID No.16: ***CTAGCTAGCTAG***GACCAGAGAGCCTGTGGGCCTTGCTCAGAGCGGAGAAA GCATGGCATAATGATGTGGCTGTTTTGTTTGTACAAGATCCGCAGACGTGT AAATGTTCCTGCAAAAACACAGACGTGGGAGGGG***CTAGCTAGCTAG***.

Among them, the underlined part represented a randomly designed exogenous non-homologous sequence (i.e., a sequence to be inserted) with a length of 103bp, NheI enzyme cutting site and protective base (bold italic) at both ends of the sequence, and the sequence was obtained by chemical synthesis and named as MMP2-2.

The selection of restriction enzyme cutting site was only for the convenience of plasmid construction and could be changed according to different vectors.

MMP2-1 and MMP2-2 were inserted into plasmid vector pSIL-eGFP, respectively, and plasmids pSIL-eGFP-MMP2-1 and pSIL-eGFP-MMP2-2 were constructed, wherein the specific process was as follows:
Enzyme digestion was performed on MMP2-1, MMP2-2 and plasmid vector pSIL-eGFP, respectively. The reaction system is shown in Table 11.

**Table 11 Enzyme digestion reaction system**

| Component | Addition Amount |
|---|---|
| MMP2-1 or MMP2-2 or pSIL-eGFP | 1 µg |
| NheI | 1µL |
| 10 x restriction enzyme buffer | 5µL |
| ddH2O | Make up to 50µL |
| Total | 50µL |

The reaction conditions were as follows: incubation at 37°C for 1h, followed by heating to 65°C for 20min to inactivate the endonuclease, electrophoresis and recovery of the digestion product.

The enzyme-cleaved MMP2-1 or MMP2-2 was connected to the enzyme-cleaved linear plasmid vector pSIL-eGFP, respectively, and the reaction system is shown in Table 12:

**Table 12 ligation reaction system**

| Component | Addition Amount |
|---|---|
| MMP2-1 or MMP2-2 | 20ng |
| pSIL-eGFP | 100ng |
| T4 DNA ligase | 1 Weiss unit |
| 10× ligation buffer | 1µL |
| Nuclease-free water | Make up to 10µL |

The reaction conditions were as follows: incubation at 16°C for 16h, followed by heating to 70°C for 10min to inactivate the ligase, followed by electrophoresis and purification to obtain the plasmid pSIL-eGFP-MMP2-1 and the plasmid pSIL-eGFP-MMP2-2. The plasmid was verified to be correct by sequencing.

Since the pSIL-eGFP plasmid itself carried the CMV promoter, transcription could be initiated by RNA polymerase II once the gene transcription framework was inserted into the CMV promoter.

Alu expression sequence was designed, by connecting Alu sequence, non-homologous sequence (18bp), TTTTT, and TTTTAA * n together, wherein n is 6, and adding SalI enzyme cutting site and corresponding protective base at two ends of that sequence to obtain the sequence shown in Seq ID No. 17: ***ACGCGTCGACGTCGGCCATAGCGGCCGCGGAA***GGGCCGGGCGCGGTGGCT CACGCCTGTAATCCCAGCACTTTGGGAGGCCGAGGCGGGCGGATCACGAG GTCAGGAGATCGAGACCATCCCGGCTAAAACGGTGAAACCCCGTCTCTAC TAAAAATACAAAAAATTAGCCGGGCGTGGTGGCGGGCGCCTGTAGTCCCA GCTACTCGGGAGGCTGAGGCAGGAGAATGGCGTGAACCCGGGAGGCGGA GCTTGCAGTGAGCCGAGATCACGCCGCTGCACTCCACCCTGGGCGACAGA GCGAGACTCCGTCTCAAAAAAAAAAAAAAAAAAAAAAAAAAAAAAGATT AATAACTGCTGGAGATCCAAGAGCGAGCCAACAGATTTTTTTTTAATTTTA ATTTTAATTTTAATTTTAATTTTA***AACGCGTCGACGTCGGCCATAGCGGCCG CGGAA***, wherein italic at two end are SalI enzyme cutting site and corresponding protective base. The upstream SalI cutting site and the corresponding protective base were followed by Alu sequence, and the underlined part was the non-homologous sequence (18bp) added after Alu sequence for marking Alu sequence so as to facilitate the detection of the connection of its transcription product with the lariat generated by transcription of the gene transcription framework (including the sequence to be inserted) in later experiments to verify the action mechanism of the experiment. The wavy line was the terminator of transcription, and the double underlined part was the six TTTTAA repeat sequences for converting RNA into DNA, which belong to the common repeat sequences in the genome, and the repeat sequence can be added more than one or none. In this embodiment, six repeat sequences were optionally added. The sequence, named Alu2, was obtained by chemical synthesis.

Alu2, plasmid pSIL-eGFP-MMP2-1 and plasmid pSIL-eGFP-MMP2-2 were digested with SalI, respectively. The digestion reaction system was shown in Table 13.

**Table 13 Enzyme digestion reaction system**

| Component | Addition Amount |
|---|---|
| Alu2 or pSIL-eGFP-MMP2-1 or pSIL-eGFP-MMP2-2 | 1µg |
| SalI | 1µL |
| 10× enzyme digestion buffer | 2µL |
| ddH₂O | Make up to 20µL |
| Total | 20µL |

The reaction conditions were as follows: incubation at 37°C for 3h, followed by heating to 80°C for 10min to inactivate the endonuclease, followed by electrophoresis, and recovery of the digestion product.

The enzyme-cleaved Alu2 was ligated with the enzyme-cleaved linear plasmid pSIL-eGFP-MMP2-1 and plasmid pSIL-eGFP-MMP2-2, respectively, and the reaction system is shown in Table 14.

**Table 14 ligation reaction system**

| Component | Addition Amount |
|---|---|
| Alu2 | 20ng |
| pSIL-eGFP-MMP2-1 or pSIL-eGFP-MMP2-2 | 100ng |
| T4 DNA ligase | 1 Weiss unit |
| 10× ligation buffer | 1µL |
| Nuclease-free water | Add to 10µL |

The reaction conditions were as follows: incubation at 16°C for 16h, followed by incubation at 70°C for 10min to inactivate the ligase, followed by electrophoresis and recovery, to obtain the plasmids pSIL-eGFP-MMP2-1-Alu2 (as shown in Fig. 16) and pSIL-eGFP-MMP2-2-Alu2. The plasmid was verified to be correct by sequencing.

Since the pSIL-eGFP plasmid itself carries the U6 promoter, which was a promoter dependent on RNA polymerase III, the transcription of Alu sequence (Alu element) could be initiated by RNA polymerase III once the Alu sequence was inserted after the U6 promoter.

pSIL-eGFP-MMP2-1-Alu2 or pSIL-eGFP-MMP2-2-Alu2 was transfected into U251 cells (human glioma) to test the insertion efficiency of the randomly designed exogenous sequence. In order to improve the insertion efficiency, pBS-L1PA1-CH-mneo, a plasmid expressing ORF1p and ORF2p (LINE), was co-transfected into U251 cells (human glioma), and the corresponding control group was designed. The co-transfected plasmid in that experimental group and the control group are shown in Table 15.

**Table 15 Grouping**

| Group | Co-transfected plasmid |
|---|---|
| Control group 1 | pSIL-eGFP+pBS-L1PA1-CH-mneo |
| Experimental group 5 | pSIL-eGFP-MMP2-1-Alu2+pBS-L1PA1-CH-mneo |
| Experimental group 6 | pSIL-eGFP-MMP2-1+pBS-L1PA1-CH-mneo |
| Experimental group 7 | pSIL-eGFP-MMP2-2-Alu2+pBS-L1PA1-CH-mneo |
| Experimental group 8 | pSIL-eGFP-MMP2-1-Alu2 |

As shown from the grouping, the control group 1 was co-transfected with original pSIL-eGFP and pBS-L1PA1-CH-mneo, which did not contain gene transcription framework sequence and Alu2 sequence. The experimental group 5 was co-transfected with pSIL-eGFP-MMP2-1-Alu2 and pBS-L1PA1-CH-mneo, which contained gene transcription framework containing long sequence upstream and downstream of the target site of *MMP2* gene, Alu2 sequence and pBS-L1PA1-CH-mneo. In experimental group 6, pSIL-eGFP-MMP2-1 and pBS-L1PA1-CH-mneo were co-transfected, which included a gene transcription framework containing long sequence upstream and downstream of target site on the *MMP2* gene, and pBS-L1PA1-CH-mneo, but did not include an Alu2 sequence. In experimental group 7, pSIL-eGFP-MMP2-2-Alu2 and pBS-L1PA1-CH-mneo were co-transfected, including the gene transcription framework containing the upstream and downstream short sequences of target site of *MMP2* gene, Alu2 sequence and pBS-L1PA1-CH-mneo; In experimental group 8, pSIL-eGFP-MMP2-1-Alu2 was transfected without pBS-L1PA1-CH-mneo, which contained the gene transcription framework with long sequence upstream and downstream of the target site on the *MMP2* gene and Alu2 sequence, but did not contain pBS-L1PA1-CH-mneo. Three parallels were set in each group, and each parallel was a 6-well plate into which U251 cells (human glioma) were cultured.

The transfection steps were as follows: U251 cells were passaged and spread into 6-well plates. On the next day of passage, transfection was performed using Entranster-H4000 transfection reagent. For transfection of each plate of cells, 48µg or 96µg (according to the experimental grouping, 48 µg if only one plasmid was transfected; If the two plasmids were co-transfected, 48µg of each plasmid, and a total of 96µg of two plasmids, were used) constructed plasmid was diluted with 300µL serum-free DMEM and mixed thoroughly. At the same time, 120µL of Entranster-H4000 reagent was diluted with 300µL of serum-free DMEM, and after fully mixed, it was allowed to stand for 5min at room temperature. Then the prepared two liquids were mixed and fully mixed, and allowed to stand for 15min at room temperature to prepare the transfection complex. The transfection complex was added to 6-well plate with U251 cells, in which contained 2ml DMEM containing 10% fetal bovine serum per well, for transfection. After the cells grew to about 90% confluence, they were passaged, and the above operations were repeated after passage. After the cells grew to about 90% confluence again, samples were taken for subsequent operations.

Extraction of transfected cell DNA: After the cell culture medium was aspirated away, the cells were rinsed twice with PBS, digested with an appropriate amount of 0.25% trypsin, and digested at 37°C for 20min, with 15 times of pipetting every 5min. After the cells were suspended, complete medium containing serum was added to stop the reaction (digestion). Thereafter, extraction of cellular DNA was performed according to the product instruction of the blood/cell/tissue genomic DNA extraction kit, and the DNA concentration was determined by an ultraviolet spectrophotometer.

### qPCR detection:

Since the *GAPDH* gene do not contain an Alu sequence and its copy number is stable, the *GAPDH* gene is used as an reference gene.

The upstream primer sequence for detecting the *GAPDH* gene is shown in Seq ID No.7 as follows: 5' -CACTGCCACCCAGAAGACTG-3'. The downstream primer sequence is shown in Seq ID No.8: 5'-CCTGCTTCACCACCTTCTTG-3'.

A primer pair 4 was designed, wherein an upstream primer sequence of the primer pair 4 is shown as Seq ID No.18: 5'-TTTCAGGGTCTAGGTGGC-3'; and the downstream primer sequence is shown in Seq ID No. 19: 5'-AAATGCTTTCTCCGCTCT-3'. The upstream primer sequence of primer pair 4 is located in the complete *MMP2* gene, further upstream of the upstream sequence of the insertion site (target site) used on the plasmid, not in the plasmid, but only in the genome, and the downstream primer sequence of primer pair 4 is located in the randomly designed non-homologous sequence to be inserted (the sequence to be inserted).

The primers were all obtained through chemical synthesis.

The qPCR reaction system is shown in Table 16.

**Table 16 qPCR reaction system**

| Component | Addition Amount |
|---|---|
| Upstream primer (10µM) | 1.5µL |
| Downstream primer (10µM) | 1.5 µL |
| 2 × SuperReal PreMix Plus | 25µL |
| Cellular DNA template (25ng/µL) | 4µL |
| ddH₂O without RNase | Make up to 50µL |

The cellular DNA template was DNA extracted from the aforementioned control group 1, and experimental group 5 to 8 after co-transfection or transfection.

The reaction system was prepared on ice, put the lid of the reaction tube on after preparation, mixed gently, and centrifuged briefly to ensure that all the components were at the bottom of the tube. Each 6-well plate cell sample was repeated 3 times simultaneously.

### qPCR reaction cycle:

Primer pair 4: pre-denaturation at 95°C for 15min; (denaturation at 95°C for 10s, annealing at 50°C for 20s, and extension at 72°C for 20s) 40 cycles. The GAPDH primers were reacted under the same conditions.

The exponential growth phases in the amplification curves of GAPDH and the detection of the insertion of the sequence to be inserted were observed and were confirmed to be approximately parallel, the obtained data were analyzed by the 2^{-ΔΔCt} method, and the results are shown in Table 17. The PCR products were verified to be correct by sequencing.

**Table 17 Results of primer pair 4 (n=3, x̅±s)**

| Ct value | *GAPD H* | Inserti on of the sequen ce to be inserte d | ΔCt | ΔΔCt (mean ΔCt of the experimental group - mean ΔCt of the control group 1) | Relative amount of copy number (2^{-ΔΔCt}) |
|---|---|---|---|---|---|
| Control group 1 plate 1 | 25.14 | N/A | 14.86 | - | - |
| Control group 1 plate 2 | 25.77 | N/A | 14.23 | - | - |
| Control group 1 plate 3 | 25.49 | N/A | 14.51 | - | - |
| Control group 1 mean | | | 14.53±0.26 | 0.00±0.26 | 1.00 (0.84-1.20) |
| Experimental group 5 plate 1 | 25.98 | 25.37 | -0.61 | - | - |
| Experimental group 5 plate 2 | 25.35 | 25.19 | -0.16 | - | - |
| Experimental group 5 plate 3 | 26.06 | 25.77 | -0.29 | - | - |
| Experimental group 5 mean | | | -0.35±0.19 | -14.88±0.19 | 30152.71 ( 26432.04-34397.12) |
| Experimental group 6 plate 1 | 25.33 | 39.10 | 13.77 | - | - |
| Experimental group 6 plate 2 | 25.84 | 39.25 | 13.41 | - | - |
| Experimental group 6 plate 3 | 25.12 | 38.37 | 13.25 | - | - |
| Experimental group 6 mean | | | 13.48±0.22 | -1.05±0.22 | 2.07 ( 1.78-2.41 ) |
| Experimental group 7 plate 1 | 26.39 | 38.89 | 12.50 | - | - |
| Experimental group 7 plate 2 | 26.02 | 38.10 | 12.08 | - | - |
| Experimental group 7 plate 3 | 25.75 | 38.04 | 12.29 | - | - |
| Experimental group 7 mean | | | 12.29±0.17 | -2.24±0.17 | 4.72± (4.20-5.31) |
| Experimental group 8 plate 1 | 25.30 | 30.51 | 5.21 | - | - |
| Experimental group 8 plate 2 | 25.88 | 31.34 | 5.46 | - | - |
| Experimental group 8 plate 3 | 25.21 | 31.26 | 6.05 | - | - |
| Experimental group 8 mean | | | 5.57±0.35 | -8.96±0.35 | 498.00 ( 390.72-634.73) |

Compared with other groups (N/A was calculated according to 40.00), the relative amount of copy number of Experimental group 5 was significantly higher than those of other groups, with statistical significance (P < 0.05), suggesting that gene editing is the most efficient with the longer upstream or downstream sequence of the insertion site (target site) in the gene transcription framework and the adequate expression of Alu element (SINE), ORF1p and ORF2p (LINE). The relative amounts of copy number in Experimental group 6, 7, and 8 were higher than that in Control Group 1 (N/A was calculated according to 40.00) and all results had statistical significance (P < 0.05), suggesting that gene editing was still effective but less efficient under the conditions of shorter upstream or downstream sequence of the insertion site (target site) in the gene transcription framework, low or no expression of Alu element (SINE) of the cell itself, or low or no expression of ORF1p and ORF2p (LINE). A sequence of sufficient length on both sides of the insertion site (target site) in the gene transcription framework is required to facilitate the formation of the lariat in order to ensure efficient insertion. The comprehensive experimental results show that by the technique of the present invention, the sequence to be inserted was effectively inserted into the target site on the genome, and longer upstream and/or downstream sequence(s) of target site(s), or additional expression of ORF1p, ORF2p (LINE) and/or Alu element (SINE) can improve editing efficiency.

### Example 4 Detect the connection between the lariat structure formed by the gene transcription framework (containing exogenous sequence to be inserted) and the RNA fragment containing partial SINE sequence (e.g., Alu element) (the transcript of Alu element cut at the natural splicing site)

1.Extraction of transfected cell total RNA of the experimental group 5 and the control group 1 in Example 3: After transfection, the cell culture medium was aspirated away, then the cells were rinsed twice with PBS, digested with an appropriate amount of 0.25% trypsin, and digested at 37°C for 20min, with 15 times of pipetting every 5min. After the cells were suspended, complete medium containing serum was added to stop the reaction (digestion). Thereafter, the solution containing cells was transfered to a RNase-free centrifuge tube, centrifuged at 300 × g for 5min, the sediment was collected and all the supernatant was aspirated away. Extraction of cellular total RNA was performed according to the product instruction of the magnetic bead method tissue/cell/blood total RNA extraction kit.
2.Synthesis of cDNA template by reverse transcription: The genomic DNA in the extracted total RNA was removed according to the instruction of the FastKing cDNA first strand synthesis kit, and then the cDNA was synthesized, and the concentration of the synthesized cDNA was determined by ultraviolet spectrophotometer.
3.qPCR detection:
   The upstream primer and downstream primer used in detection of *GAPDH* gene as internal reference are shown in Seq ID No.7 and Seq ID No.8.

The primer pair 5 was designed to detect the linkage between the transcriptional lariat structure containing the exogenous sequence to be inserted and the RNA fragment containing partial Alu sequence produced by the transcriptional product of the Alu element. The upstream primer sequence is shown in Seq ID No.20: 5'-GGCATAATGATGTGGCTGTT-3', and the upstream primer is located on the exogenous sequence to be inserted; The downstream primer sequence is shown in Seq ID No.21: 5'-TCTGTTGGCTCGCTCTTG-3'. The downstream primer is located at the non-homologous sequence (18bp) after the Alu sequence constructed into the plasmid.

The primers were all obtained through chemical synthesis.

The qPCR reaction system is shown in Table 18.

**Table 18 qPCR reaction system**

| Component | Addition Amount |
|---|---|
| Upstream primer (10µM) | 1.5µL |
| Downstream primer (10µM) | 1.5 µL |
| 2 × SuperReal PreMix Plus | 25µE |
| cDNA template (25ng/µL) | 8µL |
| ddH₂O without RNase | Make up to 50µL |

The reaction system was prepared on ice, put the lid of the reaction tube on after preparation, mixed gently, and centrifuged briefly to ensure that all the components were at the bottom of the tube. Each 6-well plate cell sample was repeated 3 times simultaneously.

### qPCR reaction cycle:

Pre-denaturation at 95°C for 15min; (denaturation at 95°C for 10s, annealing at 52°C for 20s, and extension at 72°C for 20s) 40 cycles. The *GAPDH* primers were reacted under the same conditions.

The exponential growth phases in the amplification curves of the detection of GAPDH and the linkage between the lariat structure containing the sequence to be inserted and the RNA fragment containing partial Alu were observed and were confirmed to be approximately parallel, the obtained data were analyzed by the 2^{-ΔΔCt} method, and the result is shown in Table 19. The PCR products were verified to be correct by sequencing.

**Table 19 Results of primer pair 5 (n=3, x̅±s)**

| Ct value | *GAPDH* | linkage betwee n the lariat structu re contain ing the sequen ce to be inserte d and the RNA fragme nt contain ing partial Alu sequen ce | ΔCt | ΔΔCt (mean ΔCt of the experimental group - mean ΔCt of the control group 1) | Relative amount of copy number (2^{-ΔΔCt}) |
|---|---|---|---|---|---|
| Control group 1 plate 1 | 18.53 | N/A | 21.47 | - | - |
| Control group 1 plate 2 | 19.16 | N/A | 20.84 | - | - |
| Control group 1 plate 3 | 18.92 | N/A | 21.08 | - | - |
| Control group 1 mean | | | 21.13±0.26 | 0.00±0.26 | 1.00 (0.84-1.20) |
| Experimental group 5 plate 1 | 19.68 | 28.76 | 9.08 | - | - |
| Experimental group 5 plate 2 | 18.89 | 27.91 | 9.02 | - | - |
| Experimental group 5 plate 3 | 19.25 | 27.67 | 8.42 | - | - |
| Experimental group 5 mean | | | 8.84±0.30 | -12.29±0.30 | 5007.93 ( 4067.71-6165.49) |

Compared with Control Group 1 (N/A was calculated according to 40.00), the relative amount of copy number of Experimental group 5 was significantly higher, with statistical significance (P < 0.05), suggesting that the lariat structure containing the exogenous sequence to be inserted was indeed connected with the transcript of Alu sequence (that is, the RNA fragment containing partial Alu).

It can be seen from Table 19 that transcript of Alu sequence (i.e. RNA fragment containing partial Alu) was indeed connected with the transcript containing the sequence to be inserted.

### Example 5 Testing the targeting accuracy of the gene editing technique in the present invention

The sequence of 5bp to 10bp with a total of 6bp upstream of the randomly designed non-homologous sequence (i.e., the sequence to be inserted) in the sequence MMP2-1 shown in Seq ID No. 15 is replaced with CGATGA to obtain the sequence shown in Seq ID No.22: ***CTAGCTAGCTAG***AGCATGGCGATGGATACCCCTTTGACGGTAAGGACGGA CTCCTGGCTCATGCCTTCGCCCCAGGCACTGGTGTTGGGGGAGACTCCCAT TTTGATGACGATGAGCTATGGACCTTGGGAGAAGGCCAAGGTGAGAAAGG GGCCCTCTGCATGCCCCAGACCTTCTCTCCTGTCCTCTCTCCACTCCATTTG CTTGCGATGAAGAGCCTGTGGGCCTTGCTCAGAGCGGAGAAAGCATGGCA TAATGATGTGGCTGTTTTGTTTGTACAAGATCCGCAGACGTGTAAATGTTC CTGCAAAAACACAGACGTGGGAGGGGAGGAAAGTCACACATCTGGGTGA GTCAGAATCTTGGTCTCCAAAGAAGGCCTGGAGAAGTCCAACCTCCCCCTT CCATGTCACTCTTTAGTGGTCCGTGTGAAGTATGGGAACGCCGATGGGGA GTACTGCAAGTTCCCCTTCTTGTTCAATGGCAAGGAGTACAACAGCTGCAC TGATACCGGCCGCAGCGATGGCTTCCTCTGGTGCTCCACCACCTACAACTT TGAGAAGGATGGCAAGTACGGCTTCTGTCCCCATGAAGCTA GCTA GCTA G, the wavy part was originally GACCAG, replaced with CGATGA, and the rest of the sequence was the same as Seq ID No. 15, which was obtained by chemical synthesis and named MMP2-3.

MMP2-3 was prepared using the method in Example 3 to obtain plasmid pSIL-eGFP-MMP2-3-Alu2.

The plasmid pSIL-eGFP-MMP2-3-Alu2 was transfected into U251 cells (human glioma), and plasmid pBS-L1PA1-CH-mneo expressing ORF1p and ORF2p (LINE) was also co-transfected into U251 cells as the experimental group, and the aforementioned cells co-transfected with pSIL-eGFP-MMP2-1-Alu2 and pBS-L1PA1-CH-mneo as the control group. The specific grouping is shown in Table 20.

**Table 20 Grouping**

| Group | Co-transfected plasmid |
|---|---|
| Control group 2 | pSIL-eGFP-MMP2-1-Alu2+pBS-L1PA1-CH-mneo |
| Experimental group 9 | pSIL-eGFP-MMP2-3-Alu2+pBS-L1PA1-CH-mneo |

Three parallels were set in each group, and each parallel was a 6-well plate into which U251 cells (human glioma) were cultured. The method of transfection and extraction of cell DNA after transfection was the same as Example 3.

### qPCR detection:

Since the *GAPDH* gene do not contain an Alu sequence and its copy number is stable, the *GAPDH* gene was used as a reference gene.

The upstream primer sequence for detecting the *GAPDH* gene is shown in Seq ID No.7 as follows: 5' -CACTGCCACCCAGAAGACTG-3'. The downstream primer sequence is shown in Seq ID No.8: 5'-CCTGCTTCACCACCTTCTTG-3'.

The primer pair 4 detecting the sequence to be inserted was designed. Its upstream primer sequence is shown in Seq ID No. 18 and its downstream primer sequence is shown in Seq ID No. 19.

The qPCR reaction system and reaction cycle of Example 3 were used for qPCR.

The exponential growth phases in the amplification curves of GAPDH and the detection of the insertion of the sequence to be inserted were observed and were confirmed to be approximately parallel, the obtained data were analyzed by the 2^{-ΔΔCt} method, and the result is shown in Table 21. The PCR products were verified to be correct by sequencing.

**Table 21 Results of primer pair 4 (n=3, x̅±s)**

| Ct value | *GAPDH* | Inserti on of the sequen ce to be inserte d | ΔCt | ΔΔCt (mean ΔCt of the experimental group - mean ΔCt of the control group) | Relative amount of copy number (2^{-ΔΔCt}) |
|---|---|---|---|---|---|
| Control group 2 plate 1 | 25.23 | 25.74 | 0.51 | - | - |
| Control group 2 plate 2 | 25.98 | 26.10 | 0.12 | - | - |
| Control group 2 plate 3 | 25.45 | 25.63 | 0.18 | - | - |
| Control group 2 mean | | | 0.27±0.17 | 0.00±0.17 | 1.00 (0.89-1.13) |
| Experimental group 9 plate 1 | 26.79 | N/A | 13.21 | - | - |
| Experimental group 9 plate 2 | 25.97 | N/A | 14.03 | - | - |
| Experimental group 9 plate 3 | 26.26 | N/A | 13.74 | - | - |
| Experimental group 9 mean | | | 13.66±0.34 | 13.39±0.34 | 9.32×10⁻⁵ ( 7.36×10⁻⁵-11.79×10⁻⁵) |

It can be seen from Table 21 that the relative amount of copy number in the experimental group was significantly lower than that in the control group (N/A is calculated according to 40.00), which is statistically significant (P<0.05), which means that when the upstream sequence of the insertion site (target site) on the vector is inconsistent with the upstream sequence of the insertion site (target site) on the genome, it is difficult to insert the sequence to be inserted into the genome. It is explained that the gene editing implemented by the present invention has high targeting accuracy.

### Example 6 Application of SINE sequence (taking Alu sequence as an example) direct connection method to insert exogenous sequence to be inserted

*IT15* gene is the causative gene of Huntington's disease, in the present example, the exogenous sequence is inserted into the *IT15* gene to confirm the DNA-mediated genome sequence insertion technique by SINE sequence (taking the Alu sequence as an example) direct connection method.

A 160bp sequence of gene *IT15* in the human genome was selected as shown in Seq ID No.23:
ATGCTATTCATAATCACATTCGTTTGTTTGAACCTCTTGTTATAAAAGCTTT AAAACAGTACACGACTACAACATGTGTGCAGTTACAG*AAGCAGGTTTTA GATTTGCTGGCGCAGCTGGTTCAGTTACGGGTTAATTACTGTCTTCTGGAT TCAGATCA, where * is the selected insertion site (target site), the upstream sequence of the insertion site in the *IT15* gene (upstream sequence of the target site) before the insertion site, and the downstream sequence of the insertion site in the *IT15* gene (downstream sequence of the target site) after the insertion site.

A randomly designed non-homologous sequence is added at the insertion site as the sequence to be inserted, and the "partial Alu sequence" is connected downstream of the sequence downstream of the target site to become a gene transcription framework, in order to make the gene transcription framework can be inserted into the expression vector, restriction enzyme NheI digestion sites and protection bases are added at both ends, and the complete sequence is shown in Seq ID No.24: 
CTAGCTAGCTAGATGCTATTCATAATCACATTCGTTTGTTTGAACCTCTTGT
TATAAAAGCTTTAAAACAGTACACGACTACAACATGTGTGCAGTTACAGG
AAGTTCATGGATGTCTATCAGCGCAGCTACTGCCATCCAATCGAGACCCTG
GTGGACATAAGCAGGTTTTAGATTTGCTGGCGCAGCTGGTTCAGTTACGGG
TTAATTACTGTCTTCTGGATTCAGATCAACTAAAAATACAAAAAATTAGCC
GGGCGTGGTGGCGGGCGCCTGTAGTCCCAGCTACTCGGGAGGCTGAGGCA
GGAGAATGGCGTGAACCCGGGAGGCGGAGCTTGCAGTGAGCCGAGATCA
CGCCGCTGCACTCCACCCTGGGCGACAGAGCGAGACTCCGTCTCAAAAAA
AAAAAAAAAAAAAAAAAAAAAAAAGATTAATAACTGCTGGAGATCCTAG
CTAGCTAG,
 among them, the underscore indicates a randomly designed exogenous non-homologous sequence (i.e., the sequence to be inserted), with a length of 60 bp, NheI digestion sites and protective bases (italic bold) at both ends of the sequence, and partial Alu sequences (shaded parts) between the downstream sequence of the IT15 gene insertion site (downstream sequence of the target site) and the 3 ' NheI digestion site and protective base sequence. The sequence was obtained by chemical synthesis and named IT15-1.

Here, partial Alu sequence is selected to be attached to downstream behind the sequence downstream of the target site to simulate the state in which the SINE (Alu element) transcript in vivo retains only the reverse transcription functional structure and connects with the lariat structure generated by pre-mRNA after intracellular action (shearing at the natural cutting site in SINE transcript).

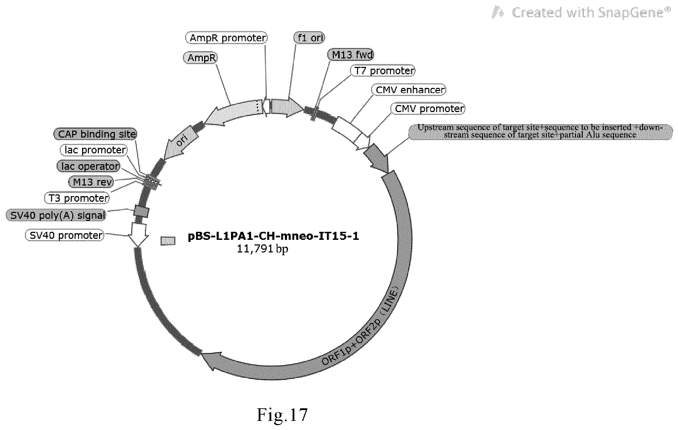

Insert IT15-1 into the plasmid vector pBS-L1PA1-CH-mneo, and construct the plasmid pBS-L1PA1-CH-mneo-IT15-1 as shown in Figure 17, the specific process is:
IT15-1 and pBS-L1PA1-CH-mneo were digested separately, and the reaction system is shown in Table 22:

**Table 22 Digestion reaction system**

| Component | Added amount |
|---|---|
| IT15-1 or pBS-L1PA1-CH-mneo | 1µg |
| NheI | 1µL |
| 10×digestion buffer | 5µL |
| ddH₂O | made up to 50µL |
| Total | 50µL |

The reaction conditions were: incubation at 37°C for 1h, then temperature to 65°C incubation for 20min to inactivate the endonuclease enzyme, electrophoresis, recovery of enzyme digestion products.

The digested IT15-1 was ligated with the plasmid vector pBS-L1PA1-CH-mneo, and the reaction system was shown in Table 23:

**Table 23 Ligation reaction system**

| Component | Amount added |
|---|---|
| IT15-1 | 20ng |
| pBS-L1PA1-CH-mneo | 100ng |
| T4 DNA ligase | 1 Weiss unit |
| 10×ligation buffer | 1µL |
| Nuclease-free water | made up to 10µL |

The reaction conditions were: incubation at 16 °C for 16h, then temperature to 70 °C, incubation for 10min to inactivate ligase, electrophoresis and purification to obtain plasmid pBS-L1PA1-CH-mneo-IT15-1. The plasmid was sequenced to verify that it was correct.

Since the pBS-L1PA1-CH-mneo plasmid carries its own CMV promoter, transcription can be initiated by RNA polymerase II as long as the expression framework is inserted into the CMV promoter.

Experimental group: the group of transfected pBS-L1PA1-CH-mneo-IT15-1 plasmid was set as experimental group 10; The group transfected with unengineered pBS-L1PA1-CH-mneo plasmid was set as control group 3. Three parallels were set in each group, and each parallel was a 6-well plate cultured with Hela cells.

The transfection procedure is to passage Hela cells and spread them in 6-well plates.

The next day, the Entranster-H4000 transfection reagent was used for transfection. For transfection of cells per plate, take 48 µg of the constructed plasmid diluted with 300 µL of serum-free DMEM and mix well; At the same time, 120 µL of Entranster-H4000 reagent was diluted with 300 µL of serum-free DMEM, mixed well, and allowed to stand at room temperature for 5 min. After that, the two prepared liquids were mixed and well mixed and allowed to stand at room temperature for 15 min to make transfection complexes. Transfection complexes are added to six-well plates containing 2 ml of DMEM culture medium containing 10% fetal bovine serum per well for transfection. When the cells are about 90% fused, the above operations are repeated after passage, and the cells are about 90% fused and then taken for subsequent operations.

Extract transfected cell DNA: After aspirating the cell culture medium, rinse the cells twice with PBS, add an appropriate amount of 0.25% trypsin for digestion, digest at 37 °C for a total of 20 min, and pipette 15 times every 5 min. When the cells are in suspension, complete medium containing serum is added to terminate the reaction. After that, the cell DNA was extracted according to the product instructions of the blood/cell/tissue genomic DNA extraction kit, and the DNA concentration was determined by ultraviolet spectrophotometer.

### qPCR test:

Since the GAPDH gene does not contain an Alu sequence and the copy number is stable, the GAPDH gene is used as an internal reference gene.

The upstream primer sequence of the GAPDH gene is shown in Seq ID No.7, and the downstream primer sequence is shown in Seq ID No. 8.

Design primer pair 6 with upstream primer sequences as shown in Seq ID No.25: 5 ' -GAAATTGGTTTGAGCAGGAG-3' ; The downstream primer sequence is shown in Seq ID No.26: 5' -CGATTGGATGGCAGTAGC-3 ' . The upstream primer sequence of primer pair 6 is located in the intact IT15 gene, further upstream in front of the upstream sequence of the insertion site (target site) used on the plasmid, not in the plasmid, only in the genome, and the downstream primer sequence of primer pair 6 is located on the randomly designed non-homologous sequence (sequence to be inserted) to be inserted.

The above primers are obtained by chemical synthesis.

The qPCR reaction system is shown in Table 24.

**Table 24 qPCR reaction system**

| Component | Amount added |
|---|---|
| Upstream primer (10µM) | 1.5µL |
| Downstream primer (10µM) | 1.5µL |
| 2×SuperReal PreMix Plus | 25µL |
| Cell DNA template ( 25ng/µL ) | 4µL |
| ddH₂O without RNase | Make up to 50µL |

The cellular DNA template was DNA extracted from the aforementioned control group 3, and experimental group 10 after transfection.

The reaction system was prepared on ice, put the lid of the reaction tube on after preparation, mixed gently, and centrifuged briefly to ensure that all the components were at the bottom of the tube. Each 6-well plate cell sample was repeated 3 times simultaneously.

### qPCR reaction cycle:

Primer pair 6: pre-denaturation at 95°C for 15min; (denaturation at 95°C for 10s, annealing at 50°C for 20s, and extension at 72°C for 20s) 40 cycles. The GAPDH primers were reacted under the same conditions.

The exponential growth phases in the amplification curves of GAPDH and the detection of the insertion of the sequence to be inserted were observed and were confirmed to be approximately parallel, the obtained data were analyzed by the 2^{-ΔΔct} method, and the results are shown in Table 25. The PCR products were verified to be correct by sequencing.

**Table 25 Results for primer pair 6 < n=3 x±s)**

| Ct value | *GAPDH* | Inserti on of the sequen ce to be inserte d | ΔCt | ΔΔCt (mean ΔCt of the experimental group - mean ΔCt of the control group) | Relative amount of copy number (2^{-ΔΔCt}) |
|---|---|---|---|---|---|
| Control group 3 plate 1 | 26.72 | N/A | 13.28 | - | - |
| Control group 3 plate 2 | 27.49 | N/A | 12.51 | - | - |
| Control group 3 plate 3 | 27.65 | N/A | 12.35 | - | - |
| Control group 3 mean | | | 12.71±0.41 | 0.00±0.41 | 1.00 (0.75-1.33) |
| Experimental group 10 plate 1 | 25.94 | 25.31 | -0.63 | - | - |
| Experimental group 10 plate 2 | 26.67 | 25.59 | -1.08 | - | - |
| Experimental group 10 plate 3 | 25.85 | 25.10 | -0.75 | - | - |
| Experimental group 10 mean | | | -0.82±0.19 | -13.53±0.19 | 11828.67 ( 10369.08-13493.72) |

It can be seen from Table 25 that the relative amount of copy number in the experimental group 10 was significantly higher than that in the control group 3 (N/A is calculated according to 40.00), which is statistically significant (P<0.05), which indicates that the sequence to be inserted is efficiently inserted into a target site on the genome.

### Example 7 testing the targeting accuracy of the gene editing technology in the present invention

Replace the 10bp to 15bp (6bp in total) upstream of the randomly designed non-homologous sequence (i.e., the sequence to be inserted) in the sequence shown in Seq ID No.24 with GGACAT, resulting in the sequence shown in Seq ID No.27: 
CTAGCTAGCTAGATGCTATTCATAATCACATTCGTTTGTTTGAACCTCTTGT
TATAAAAGCTTTAAAACAGTACACGACTACAACAGGACATCAGTTACAGG
AAGTTCATGGATGTCTATCAGCGCAGCTACTGCCATCCAATCGAGACCCTG
GTGGACATAAGCAGGTTTTAGATTTGCTGGCGCAGCTGGTTCAGTTACGGG
TTAATTACTGTCTTCTGGATTCAGATCAACTAAAAATACAAAAAATTAGCC
GGGCGTGGTGGCGGGCGCCTGTAGTCCCAGCTACTCGGGAGGCTGAGGCA
GGAGAATGGCGTGAACCCGGGAGGCGGAGCTTGCAGTGAGCCGAGATCA
CGCCGCTGCACTCCACCCTGGGCGACAGAGCGAGACTCCGTCTCAAAAAA
AAAAAAAAAAAAAAAAAAAAAAAAGATTAATAACTGCTGGAGATCCTAG
CTAGCTAG,
 in this sequence, the wavy part was originally TGTGTG and replaced with GGACAT, and the rest of the sequence was the same as Seq ID No.24, which was obtained by chemical synthesis and named IT15-2.

IT15-2 was inserted into the plasmid vector pBS-L1PA1-CH-mneo, and the plasmid pBS-L1PA1-CH-mneo-IT15-2 was constructed, and the method refers to Example 6.

The Hela cell group transfected with pBS-L1PA1-CH-mneo-IT15-2 plasmid was experimental group 11; The Hela cell group transfected with the pBS-L1PA1-CH-mneo-IT15-1 plasmid was the control group 4. Three parallels were set in each group, and each parallel was a 6-well plate cultured with Hela cells.

Example 6 method was used for transfection and extraction of transfected cell DNA for qPCR detection.

Since the GAPDH gene does not contain an Alu sequence and the copy number is stable, the GAPDH gene is used as an internal reference gene.

The upstream primer sequence of the GAPDH gene is shown in Seq ID No.7, and the downstream primer sequence is shown in Seq ID No. 8.

Use primer pair 6 for qPCR, reaction system and reaction cycle as in Example 6.

The exponential growth phases in the amplification curves of GAPDH and the detection of the insertion of the sequence to be inserted were observed and were confirmed to be approximately parallel, the obtained data were analyzed by the 2^{-ΔΔct} method, and the results are shown in Table 26. The PCR products were verified to be correct by sequencing.

**Table 26 Results for primer pair 6 (n=3, x±s)**

| Ct value | *GAPDH* | Inserti on of the sequen ce to be inserte d | ΔCt | ΔΔCt (mean ΔCt of the experimental group - mean ΔCt of the control group) | Relative amount of copy number (2^{-ΔΔCt}) |
|---|---|---|---|---|---|
| Control group 4 plate 1 | 25.49 | 24.73 | -0.76 | - | - |
| Control group 4 plate 2 | 25.66 | 25.04 | -0.62 | - | - |
| Control group 4 plate 3 | 25.89 | 25.67 | -0.22 | - | - |
| Control group 4 mean | | | -0.53±0.23 | 0.00±0.23 | 1.00 (0.85-1.17) |
| Experimental group 11 plate 1 | 25.52 | N/A | 14.48 | - | - |
| Experimental group 11 plate 2 | 25.14 | N/A | 14.86 | - | - |
| Experimental group 11 plate 3 | 24.78 | N/A | 15.22 | - | - |
| Experimental group 11 mean | | | 14.85±0.30 | 15.38±0.30 | 2.35 × 10⁻⁵ ( 1.90×10⁻⁵-2.89×10⁻⁵) |

The relative amount of copy number in the experimental group was significantly lower than that in the control group (N/A was calculated according to 40.00), which was statistically significant (P<0.05), which meant that when the upstream sequence of the insertion site (target site) on the vector was inconsistent with the upstream sequence of the insertion site (target site) on the genome, it was difficult for the sequence to be inserted into the target site on the genome.

Conclusion: It is stated that the genome sequence insertion implemented by the present invention has high targeting accuracy.

From Example 1 to Example 6, it can be seen that the method of DNA-mediated exogenous sequence insertion into the genome designated site can be effective gene editing of eukaryotic cells (such as cell lines or primary cells), and the sequence to be inserted is targeted to the target site with high efficiency and accuracy. From the feasibility of editing different tissue cells, it can be seen that this method can be applied to various cells, tissues and organisms (living organisms).

### Example 8 DNA-mediated deletion of sequence (sequence to be deleted) on the genome

A sequence of gene *MINK1* in the human genome is randomly selected, as shown in Seq ID No.28: 
AGAGAATGAGGGGCCCCTTTTTCTCTCTGGTGGCTCAGGCCCAACTCCCTT
CCTACTGGGGAGGCTCACTCCCTCCCCTTTCCCCTCTCCCCCTGGAATGCC
CTGCCTCCTGCTGAAAATCCCTCAGGAAGCTCTTCACCTGTCACCTGTTAC
GGGCCAGGTGCTCTGCAGGTTGCTCTGGGGAGATGGGATCTGATGGCCCT
CCTGCCTGGGATGCTGTCCGTGATCCTTTTACCTGGGTTTTTCTCTAAGATG
CTGGAAGATGGAATCGGGTTCTTCAGGATGGTGGTGGGGTAAAGGAGGGT
GCTGGGGTGTCTGGGTCGGGCCAGGACCACAGCTGGCTCAGGCAAGTCCT
GTGTGTGCACGCAGGGATGTGAGGCAAGGGAGCAGAGGTGACTCCCCACA
CTGACCCCTCCCTCTGTGTCTTCACAGTGGATTTTGGGGTGAGTGCTCAGC
TGGACCGCACCGTGGGCAGACGGAACACTTTCATTGGGACTCCCTACTGG
ATGGCTCCAGAGGTCATCGCCTGTGATGAGAACCCTGATGCCACCTATGAT
TACAGGGTATGGAGTGGAAAGTTGGGAGCATGGGGGCTGCCAAGGGCGG
GAAGCAATATGGGGACCACGGGGCCTGAGCAGGCTGGGGAACAGAGGAA
GGTCAGATGATGTTAGCAGTGAGGGGCTGGGGAACATCTTACGGCAAGGC
AAGTGTGGGTGGGAAGATGGGATGGGTTGGAAGGCACTGCTGCAGGAATG
GGTGTGGCCCAGGAAGGCTCCTGAGAGGCCAGGATGGTGGGTGAAGAGA
 
GGTTGCAGGGCAGAGTTGTCAGGAATATTCACTTGTTCCTTCTTTCCCGTCT
ATAGAGTGATATTTGGTCTCTAGGAATCACAGCCATCGAGATGGCAGAGG
GAGCCCCCCGTAAGTTCTGAGTCTGCCGGGAGTGGGAGGGGAGGGAAAGG
AAGGGCCCAGAGAGTGGCTGTAGGGAGGAGGTGGGTCCTGGGACCCTGCC
GAGGAAGGGTCCTGTAGCTCCCAGTGCAGTGAAAGGGACTGAGGGTGTCT
CCTCTGTGTCCAGCTCTGTGTGACATGCACCCCATGCGAGCCCTCTTCCTC
ATTCCTCGGAACCCTCCGCCCAGGCTCAAGTCCAAGAAGTG,
 where the underlined part is the sequence to be deleted on the genome, the upstream sequence immediately adjacent to the 5' end of the sequence to be deleted on the genome before the sequence to be deleted, the downstream sequence immediately adjacent to the 3' end of the sequence to be deleted on the genome after the sequence to be deleted, and the shadowed part is the 3' sequence of the sequence to be deleted on the genome.

The sequence is constructed in the order of the 3' sequence of the sequence to be deleted + the upstream sequence immediately adjacent to the 5' end of the sequence to be deleted + the downstream sequence immediately adjacent to the 3' end of the sequence to be deleted, and the NheI digestion site and the corresponding protective base are added at both ends, the sequence is shown in Seq ID No.29:
***CTAGCTAGCTAG***AGTGATATTTGGTCTCTAGGAATCACAGCCATCGAGATG GCAGAGGGAGCCCCCCGTAAGTTCTGAGTCTGCCAGAGAATGAGGGGCCC CTTTTTCTCTCTGGTGGCTCAGGCCCAACTCCCTTCCTACTGGGGAGGCTCA CTCCCTCCCCTTTCCCCTCTCCCCCTGGAATGCCCTGCCTCCTGCTGAAAAT CCCTCAGGAAGCTCTTCACCTGTCACCTGTTACGGGCCAGGTGCTCTGCAG GTTGCTCTGGGGAGTGGGAGGGGAGGGAAAGGAAGGGCCCAGAGAGTGG CTGTAGGGAGGAGGTGGGTCCTGGGACCCTGCCGAGGAAGGGTCCTGTAG CTCCCAGTGCAGTGAAAGGGACTGAGGGTGTCTCCTCTGTGTCCAGCTCTG TGTGACATGCACCCCATGCGAGCCCTCTTCCTCATTCCTCGGAACCCTCCG CCCAGGCTCAAGTCCAAGAAGTG***CTAGCTAGCTAG***, in which the italic and bold parts at both ends are NheI digestion sites and corresponding protective bases. The sequence was obtained by chemical synthesis and named MINK1-1.

At the same time, a sequence that is not homologous to the MINK1 gene is used to replace the upstream sequence immediately adjacent to the 5' end of the sequence to be deleted in Seq ID No. 29, and the sequence is obtained as shown in Seq ID No. 30: ***CTAGCTAGCTAG***AGTGATATTTGGTCTCTAGGAATCACAGCCATCGAGATG GCAGAGGGAGCC*C*CCCGTAAGTTCTGAGTCTGCCCATTGTTATAATGATTA AACAAGTTATATATGAAAAGATTAAAACAGTGTTGCTCCATAATAAATGC TGTTTTTACTGTGATTATTATTGTTGTTATCCCTATCATTATCATCACCATCT TAACCCTTCCCTGTTTTGCTCTTTTCTCTCTCCCTACCCATTGCAGACCAAA GAAAGATAGGGAGTGGGAGGGGAGGGAAAGGAAGGGCCCAGAGAGTGGC TGTAGGGAGGAGGTGGGTCCTGGGACCCTGCCGAGGAAGGGTCCTGTAGC TCCCAGTGCAGTGAAAGGGACTGAGGGTGTCTCCTCTGTGTCCAGCTCTGT GTGACATGCACCCCATGCGAGCCCTCTTCCTCATTCCTCGGAACCCTCCGC CCAGGCTCAAGTCCAAGAAGTG***CTAGCTAGCTAG***, where the underscore is a substituted sequence that is not homologous to the MINK1 gene. The sequence was obtained by chemical synthesis and named MINK1-2.

MINK1-1 and MINK1-2 were inserted into the plasmid vector pSIL-eGFP, respectively, to construct plasmids pSIL-eGFP-MINK1-1 and pSIL-eGFP-MINK1-2, as follows:
MINK1-1, MINK1-2, and plasmid pSIL-eGFP were digested separately, and the reaction system was shown in Table 27:

**Table 22 Digestion reaction system**

| Component | Added amount |
|---|---|
| MINK1-1 or MINK1-2 or pSIL-eGFP | 1µg |
| NheI | 1µL |
| 10×digestion buffer | 5µL |
| ddH₂O | made up to 50µL |
| Total | 50µL |

The reaction conditions were: incubation at 37°C for 1h, then temperature to 65°C incubation for 20min to inactivate the endonuclease enzyme, electrophoresis, recovery of enzyme digestion products.

The digested MINK1-1 or MINK1-2 was ligated with the plasmid vector pSIL-eGFP, and the reaction system was shown in Table 28:

**Table 28 Ligation reaction system**

| Component | Amount added |
|---|---|
| MINK1-1 or MINK1-2 | 20ng |
| pSIL-eGFP | 100ng |
| T4 DNA ligase | 1 Weiss unit |
| 10×ligation buffer | 1µL |
| Nuclease-free water | made up to 10µL |

The reaction conditions were: incubation at 16 °C for 16h, then temperature to 70 °C, incubation for 10min to inactivate ligase, electrophoresis and purification to obtain plasmid pSIL-eGFP-MINK1-1 and pSIL-eGFP-MINK1-2. The plasmid was sequenced to verify that it was correct.

The Alu1 prepared in Example 1, plasmid pSIL-eGFP-MINK1-1 and plasmid pSIL-eGFP-MINK1-2 were digested with SalI, and then linked, and the reaction system and conditions were the same as Example 1 to obtain pSIL-eGFP-MINK1-1-Alu1 and pSIL-eGFP-MINK1-2-Alu1.

pSIL-eGFP-MINK1-1-Alu1 or pSIL-eGFP-MINK1-2-Alu1 was transfected into Hela cells to test the effect of deleting the "sequence to be deleted", and in order to improve the deletion efficiency, plasmid pBS-L1PA1-CH-mneo expressing ORF1p and ORF2p (LINE) was co-transfected in Hela cells, and the corresponding control group was designed. Among them, the group transfected pSIL-eGFP-MINK1-1-Alu1+pBS-L1PA1-CH-mneo was set as experimental group 12, and the group transfected with pSIL-eGFP-MINK1-2-Alu1+pBS-L1PA1-CH-mneo was set as control group 5. Three parallels were set in each group, and each parallel was a 6-well plate cultured with Hela cells.

According to the method of Example 1, plasmid transfection is performed and the transfected cell DNA is extracted.

### qPCR test:

Since the GAPDH gene does not contain an Alu sequence and the copy number is stable, the GAPDH gene is used as an internal reference gene.

The upstream primer sequence of the GAPDH gene is shown in Seq ID No.7, and the downstream primer sequence is shown in Seq ID No. 8.

Design primer pair 7 with upstream primer sequences as shown in Seq ID No.31: 5' -ACAGGGTATGGAGTGGAAAG-3' ; The downstream primer sequence is shown in Seq ID No.32: 5' -ATAGACGGGAAAGAAGGAAC-3' . The upstream primer of primer pair 7 is located on the sequence to be deleted in the MINK1 gene on the genome and is not present in the plasmid, and the downstream primer is located on the sequence to be deleted in the MINK1 gene on the genome and is not present in the plasmid.

The above primers are obtained by chemical synthesis.

The qPCR reaction system is shown in Table 29.

**Table 29 qPCR reaction system**

| Component | Amount added |
|---|---|
| Upstream primer (10µM) | 1.5µL |
| Downstream primer (10µM) | 1.5µL |
| 2 × SuperReal PreMix Plus | 25µE |
| Cell DNA template ( 25ng/µL ) | 4µL |
| ddH₂O without RNase | Make up to 50µL |

The cellular DNA template was DNA extracted from the aforementioned control group 5, and experimental group 12 after transfection.

The reaction system was prepared on ice, put the lid of the reaction tube on after preparation, mixed gently, and centrifuged briefly to ensure that all the components were at the bottom of the tube. Each 6-well plate cell sample was repeated 3 times simultaneously.

### qPCR reaction cycle:

Primer pair 7: pre-denaturation at 95°C for 15min; (denaturation at 95°C for 10s, annealing at 50°C for 20s, and extension at 72°C for 20s) 40 cycles. The GAPDH primers were reacted under the same conditions.

After observing the exponential growth period in the amplification curve of GAPDH and detecting sequence to be deleted, and confirming that it is approximately parallel, the obtained data were analyzed by the 2^{-ΔΔct} method, and the results are shown in Table 30. The PCR products were verified to be correct by sequencing.

**Table 30 Results for primer pair 7 (n=3, x̅±s)**

| Ct value | *GAPDH* | The sequen ce to be deleted | ΔCt | ΔΔCt (mean ΔCt of the experimental group - mean ΔCt of the control group) | Relative amount of copy number (2^{-ΔΔCt}) |
|---|---|---|---|---|---|
| Control group 5 plate 1 | 24.13 | 23.74 | -0.39 | - | - |
| Control group 5 plate 2 | 24.90 | 24.22 | -0.68 | - | - |
| Control group 5 plate 3 | 24.67 | 23.91 | -0.76 | - | - |
| Control group 5 mean | | | -0.61±0.16 | 0.00±0.16 | 1.00 (0.90-1.12) |
| Experimental group 12 plate 1 | 25.26 | 39.13 | 13.87 | - | - |
| Experimental group 12 plate 2 | 25.44 | 39.06 | 13.62 | - | - |
| Experimental group 12 plate 3 | 25.85 | N/A | 14.15 | - | - |
| Experimental group 12 mean | | | 13.88±0.22 | 14.49±0.22 | 4.35×10⁻⁵ (3.73×10⁻⁵-5.06×10⁻⁵) |

The relative amount of copy number in the experimental group 12 (N/A calculated according to 40.00) was significantly lower than that in the control group 5, which was statistically significant (P<0.05), indicating that the sequence to be deleted on the genome was deleted.

### Example 9 Deleting the sequence by SINE sequence (taking Alu sequence as an example) direct connection method

The *FMR1* gene is associated with the hereditary mental retardation - fragile X syndrome, and one of the sequences was selected, as shown in Seq ID No. 33: 
ACTTGCTGAGTACCCAAGGAAAGTGTGCTTGTATTTATGGGCGTCTATTTT
CAGAGCACTAATTATTGCTGAATTAGAACAGAAATATAGGAAAACTGATT
 
TTTACAAGGAGCTTCAAAGCAATCTCAGGTAGTTTCTGATTATGTATCTCT
GCCTACCTCGGGGTACATAGACAGGGTTACAATTTGGTTGAGGATATATG
ACATGTGGTTTTTAAAGACACCTAGGGGCATTTTAAGAAAATTTCCTCGAT
ATCTGAAAATCTGTAGATTTCAAAATTATGTTAATCATGAAATATTCTGTG
TTGTAATTTTTGTGTAGGTGTATTCCAGAGCAAATGAAAAAGAGCCTTGCT
GTTGGTGGTTAGCTAAAGTGAGGATGATAAAGGGTGAGGTAGGAAAATGC
CTATTTAAATTTTTTTCTTATATTGTTTCCTTTTTTTAAACCCAGGTTGTACA
TTCCCGTGTGGATTTCTATTTTGAAGTAATATCTAATTTTGAGTAATTTAAT
TAAAATGTTTTCACTATGTGTTCAGTATGTTTCTGTTGGTCATAAATTTTTT
CACATAGATTATTTATTTTAAAATAACTGAATAGGGAGAACTTCTTATTCT
TACTTTAAAAATTGTGATTAGAAGTGACTTTTATTTATTTCTCAGTTTTATG
TGATAGAATATGCAGCATGTGATGCAACTTACAATGAAATTGTCACAATTG
AACGTCTAAGATCTGTTAATCCCAACAAACCTGCCACAAAAGATACTTTCC
ATAAGATCAAGCTGGATGTGCCAGAAGACTTACGGCAAA,
 where the underlined part is the sequence to be deleted on the genome, the upstream sequence immediately adjacent to the 5' end of the sequence to be deleted on the genome before the sequence to be deleted, the downstream sequence immediately adjacent to the 3' end of the sequence to be deleted on the genome after the sequence to be deleted, and the shadowed part is the 3' sequence of the sequence to be deleted on the genome. The sequence is constructed in the order of the 3' sequence of the sequence to be deleted + the upstream sequence immediately adjacent to the 5' end of the sequence to be deleted + the downstream sequence immediately adjacent to the 3' end of the sequence to be deleted + partial Alu sequence, and the NheI digestion site and the corresponding protective base are added at both ends, the sequence is shown in Seq ID No.34: 
CTAGCTAGCTAGTTTATGTGATAGAATATGCAGCATGTGATGCAACTTACA
ATGAAATTGTCACAATTGAACGTCTAAGATCTGTTAATCCCAACAAACCTG
CCACAAAAGACTTGCTGAGTACCCAAGGAAAGTGTGCTTGTATTTATGGG
CGTCTATTTTCAGAGCACTAATTATTGCTGAATTAGAACAGAAATATAGGA
AAACTGATATACTTTCCATAAGATCAAGCTGGATGTGCCAGAAGACTTAC
GGCAAAACTAAAAATACAAAAAATTAGCCGGGCGTGGTGGCGGGCGCCTG
TAGTCCCAGCTACTCGGGAGGCTGAGGCAGGAGAATGGCGTGAACCCGGG
 
AGGCGGAGCTTGCAGTGAGCCGAGATCACGCCGCTGCACTCCACCCTGGG
CGACAGAGCGAGACTCCGTCTCAAAAAAAAAAAAAAAAAAAAAAAAAAAA
AAGATTAATAACTGCTGGAGATCCTAGCTAGCTAG,
 in which the italic and bold parts at both ends are NheI digestion sites and corresponding protective bases. The shaded part is a partial Alu sequence. The sequence was obtained by chemical synthesis and named FMR1-1.

At the same time, a sequence that is not homologous to the *FMR1* gene is used to replace the upstream sequence immediately adjacent to the 5' end of the sequence to be deleted in Seq ID No. 34, and the sequence is obtained as shown in Seq ID No. 35: 
CTAGCTAGCTAGTTTATGTGATAGAATATGCAGCATGTGATGCAACTTACA
ATGAAATTGTCACAATTGAACGTCTAAGATCTGTTAATCCCAACAAACCTG
CCACAAAAGAGTGTTGCTCCATAATAAATGCTGTTTTTACTGTGATTATTA
TTGTTGTTATCCCTATCATTATCATCACCATCTTAACCCTTCCCTGTTTTGCT
CTTTTATACTTTCCATAAGATCAAGCTGGATGTGCCAGAAGACTTACGGCA
AAACTAAAAATACAAAAAATTAGCCGGGCGTGGTGGCGGGCGCCTGTAGT
CCCAGCTACTCGGGAGGCTGAGGCAGGAGAATGGCGTGAACCCGGGAGG
CGGAGCTTGCAGTGAGCCGAGATCACGCCGCTGCACTCCACCCTGGGCGA
CAGAGCGAGACTCCGTCTCAAAAAAAAAAAAAAAAAAAAAAAAAAAAAA
GATTAATAACTGCTGGAGATCCTAGCTAGCTAG,
 where the underscore is a substituted sequence that is not homologous to the *FMR1* gene. The sequence was obtained by chemical synthesis and named FMR1-2.

FMR1-1 and FMR1-2 were inserted into the plasmid vector pBS-L1PA1-CH-mneo, respectively, to construct plasmids pBS-L1PA1-CH-mneo-FMR1-1 and pBS-L1PA1-CH-mneo-FMR1-2, as follows:
FMR1-1, FMR1-2, and plasmid pBS-L1PA1-CH-mneo were digested separately, and the reaction system was shown in Table 31:

**Table 31 Digestion reaction system**

| Component | Added amount |
|---|---|
| FMR1-1 or FMR1-2 or pBS-L1PA1-CH-mneo | 1µg |
| NheI | 1µL |
| 10×digestion buffer | 5µL |
| ddH₂O | made up to 50µL |
| Total | 50µL |

The reaction conditions were: incubation at 37°C for 1h, then temperature to 65°C incubation for 20min to inactivate the endonuclease enzyme, electrophoresis, recovery of enzyme digestion products.

The digested FMR1-1 or FMR1-2 was ligated with the plasmid vector pBS-L1PA1-CH-mneo, and the reaction system was shown in Table 32:

**Table 32 Ligation reaction system**

| Component | Amount added |
|---|---|
| FMR1-1 or FMR1-2 | 20ng |
| pBS-L1PA1-CH-mneo | 100ng |
| T4 DNA ligase | 1 Weiss unit |
| 10×ligation buffer | 1µL |
| Nuclease-free water | made up to 10µL |

The reaction conditions were: incubation at 16 °C for 16h, then temperature to 70 °C, incubation for 10min to inactivate ligase, electrophoresis and purification to obtain plasmids pBS-L1PA1-CH-mneo-FMR1-1 and pBS-L1PA1-CH-mneo-FMR1-2. The plasmid was sequenced to verify that it was correct.

The group transfected pBS-L1PA1-CH-mneo-FMR1-1 was set as experimental group 13, and the group transfected with pBS-L1PA1-CH-mneo-FMR1-2 was set as control group 6. Three parallels were set in each group, and each parallel was a 6-well plate cultured with Hela cells.

The transfection steps were as follows: Hela cells were passaged and spread into 6-well plates. On the next day of passage, transfection was performed using Entranster-H4000 transfection reagent. For transfection of each plate of cells, 48µg constructed plasmid was diluted with 300µL serum-free DMEM and mixed thoroughly. At the same time, 120µL of Entranster-H4000 reagent was diluted with 300µL of serum-free DMEM, and after fully mixed, it was allowed to stand for 5min at room temperature. Then the prepared two liquids were mixed and fully mixed, and allowed to stand for 15min at room temperature to prepare the transfection complex. The transfection complex was added to 6-well plate with Hela cells, in which contained 2ml DMEM containing 10% fetal bovine serum per well, for transfection. After the cells grew to about 90% confluence, they were passaged, and the above operations were repeated after passage. After the cells grew to about 90% confluence again, samples were taken for subsequent operations.

Extraction of transfected cell DNA: After the cell culture medium was aspirated away, the cells were rinsed twice with PBS, digested with an appropriate amount of 0.25% trypsin, and digested at 37°C for 20min, with 15 times of pipetting every 5min. After the cells were suspended, complete medium containing serum was added to stop the reaction (digestion). Thereafter, extraction of cellular DNA was performed according to the product instruction of the blood/cell/tissue genomic DNA extraction kit, and the DNA concentration was determined by an ultraviolet spectrophotometer.

### qPCR test:

Since the GAPDH gene does not contain an Alu sequence and the copy number is stable, the GAPDH gene is used as an internal reference gene.

The upstream primer sequence of the GAPDH gene is shown in Seq ID No.7, and the downstream primer sequence is shown in Seq ID No. 8.

Design primer pair 8 with upstream primer sequences as shown in Seq ID No.36: 5' -ACAGGGTTACAATTTGGT-3 ' ; The downstream primer sequence is shown in Seq ID No.37: 5' -CATTTGCTCTGGAATACAC-3 ' . The upstream primer of primer pair 8 is located on the sequence to be deleted in the *FMR1* gene on the genome and is not present in the plasmid, and the downstream primer is located on the sequence to be deleted in the *FMR1* gene on the genome and is not present in the plasmid.

The above primers are obtained by chemical synthesis.

The qPCR reaction system is shown in Table 33.

**Table 33 qPCR reaction system**

| Component | Amount added |
|---|---|
| Upstream primer (10µM) | 1.5µL |
| Downstream primer (10µM) | 1.5µL |
| 2×SuperReal PreMix Plus | 25µE |
| Cell DNA template ( 25ng/µL ) | 4µL |
| ddH₂O without RNase | Make up to 50µL |

The cellular DNA template was DNA extracted from the aforementioned control group 6, and experimental group 13 after transfection.

The reaction system was prepared on ice, put the lid of the reaction tube on after preparation, mixed gently, and centrifuged briefly to ensure that all the components were at the bottom of the tube. Each 6-well plate cell sample was repeated 3 times simultaneously.

### qPCR reaction cycle:

Primer pair 8: pre-denaturation at 95°C for 15min; (denaturation at 95°C for 10s, annealing at 45°C for 20s, and extension at 72°C for 20s) 40 cycles. The GAPDH primers were reacted under the same conditions.

After observing the exponential growth period in the amplification curve of GAPDH and detecting sequence to be deleted, and confirming that it is approximately parallel, the obtained data were analyzed by the 2^{-ΔΔct} method, and the results are shown in Table 34. The PCR products were verified to be correct by sequencing.

**Table 34 Results for primer pair 8 (n=3, x±s)**

| Ct value | *GAPDH* | The sequen ce to be deleted | ΔCt | ΔΔCt (mean ΔCt of the experimental group - mean ΔCt of the control group) | Relative amount of copy number (2^{-ΔΔCt}) |
|---|---|---|---|---|---|
| Control group 6 plate 1 | 25.07 | 25.44 | 0.37 | - | - |
| Control group 6 plate 2 | 25.69 | 26.10 | 0.41 | - | - |
| Control group 6 plate 3 | 25.49 | 25.44 | -0.05 | - | - |
| Control group 6 mean | | | 0.24±0.21 | 0.00±0.21 | 1.00 (0.86-1.16) |
| Experimental group 13 plate 1 | 26.40 | N/A | 13.60 | - | - |
| Experimental group 13 plate 2 | 25.26 | 39.48 | 14.22 | - | - |
| Experimental group 13 plate 3 | 26.51 | N/A | 13.49 | - | - |
| Experimental group 13 mean | | | 13.77±0.32 | 13.53±0.32 | 8.45×10⁻⁵ ( 6.77×10⁻⁵-10.55×10⁻⁵) |

The relative amount of copy number in the experimental group 13 (N/A calculated according to 40.00) was significantly lower than that in the control group 6, which was statistically significant (P<0.05), indicating that the sequence to be deleted on the genome was deleted.

From the above embodiments, it can be seen that the deletion of any sequence in any region of the genome using the present invention is feasible. At the same time, when sequencing obtains the CNVs end of each gene (that is, the corresponding gene sequence connects the partial SINE sequence), the CNVs end can also be edited: insert or delete to edit the CNVs (CNVs end) and modify the state of cells, tissues or living organisms by the expression changes brought by them.

### Example 10 RNA-mediated insertion of exogenous sequence to be inserted into the genome

A sequence of gene *IT15* in the human genome was selected and the corresponding sequence was designed in the following order: NheI digestion recognition site and protective bases + upstream sequence of insertion site (target site) + sequence to be inserted + downstream sequence of insertion site (target site) + partial Alu sequence + NheI digestion recognition site and protection bases, as shown in Seq ID No.38: 
CTAGCTAGCTAGATGCTATTCATAATCACATTCGTTTGTTTGAACCTCTTGT
TATAAAAGCTTTAAAACAGTACACGACTACGAAGTTCATGGATGTCTATCA
GCGCAGCTACTGCCATCCAATCGAGACCCTGGTGGACATAACATGTGTGC
AGTTACAGAAGCAGGTTTTAGATTTGCTGGCGCAGCTGGTTCAGTTACGGG
TTAATTACTGTCTTCTGGATTCAGATCAACTAAAAATACAAAAAATTAGCC
 
GGGCGTGGTGGCGGGCGCCTGTAGTCCCAGCTACTCGGGAGGCTGAGGCA
GGAGAATGGCGTGAACCCGGGAGGCGGAGCTTGCAGTGAGCCGAGATCA
CGCCGCTGCACTCCACCCTGGGCGACAGAGCGAGACTCCGTCTCAAAAAA
AAAAAAAAAAAAAAAAAAAAAAAAGATTAATAACTGCTGGAGATCCTAG
CTAGCTAG,
 wherein the underscore indicates a randomly designed sequence to be inserted that is non-homologous to the *IT15* gene, with a length of 60 bp, a NheI digestion site and protective bases (italic bold) at both ends of the sequence, and a partial Alu sequence (shaded part) between the downstream sequence of the IT15 gene insertion site (target site) and the 3' NheI digestion site and the protective base sequence. The sequence was obtained by chemical synthesis and named IT15-3.

IT15-3 was inserted into the plasmid vector pBS-L1PA1-CH-mneo, respectively, to construct plasmids pBS-L1PA1-CH-mneo-IT15-3, as follows:
IT15-3 and plasmid pBS-L1PA1-CH-mneo were digested separately, and the reaction system was shown in Table 35:

**Table 35 Digestion reaction system**

| Component | Added amount |
|---|---|
| IT15-3 or pBS-L1PA1-CH-mneo | 1µg |
| NheI | 1µL |
| 10×digestion buffer | 5µL |
| ddH₂O | made up to 50µL |
| Total | 50µL |

The reaction conditions were: incubation at 37°C for 1h, then temperature to 65°C incubation for 20min to inactivate the endonuclease enzyme, electrophoresis, recovery of enzyme digestion products.

The digested IT15-3 was ligated with the plasmid vector pBS-L1PA1-CH-mneo, and the reaction system was shown in Table 36:

**Table 36 Ligation reaction system**

| Component | Amount added |
|---|---|
| IT15-3 | 20ng |
| pBS-L1PA1-CH-mneo | 100ng |
| T4 DNA ligase | 1 Weiss unit |
| 10×ligation buffer | 1µL |
| Nuclease-free water | made up to 10µL |

The reaction conditions were: incubation at 16 °C for 16h, then temperature to 70 °C, incubation for 10min to inactivate ligase, electrophoresis and purification to obtain plasmids pBS-L1PA1-CH-mneo-IT15-3. The plasmid was sequenced to verify that it was correct.

The pBS-L1PA1-CH-mneo-IT15-3 and pBS-L1PA1-CH-mneo plasmids were then transfected into Hela cells, respectively.

The transfection steps were as follows: Hela cells were passaged and spread into 6-well plates. On the next day of passage, transfection was performed using Entranster-H4000 transfection reagent. For transfection of each plate of cells, 48 µg constructed plasmid was diluted with 300µL serum-free DMEM and mixed thoroughly. At the same time, 120µL of Entranster-H4000 reagent was diluted with 300µL of serum-free DMEM, and after fully mixed, it was allowed to stand for 5min at room temperature. Then the prepared two liquids were mixed and fully mixed, and allowed to stand for 15min at room temperature to prepare the transfection complex. The transfection complex was added to 6-well plate with Hela cells, in which contained 2ml DMEM containing 10% fetal bovine serum per well, for transfection. After the cells grew to about 90% confluence, they were passaged, and the above operations were repeated after passage. After the cells grew to about 90% confluence again, samples were taken for subsequent operations.

Extraction of transfected cell RNA: After the cell culture medium was aspirated away, the cells were rinsed twice with PBS, digested with an appropriate amount of 0.25% trypsin, and digested at 37°C for 20min, with 15 times of pipetting every 5min. After the cells were suspended, complete medium containing serum was added to stop the reaction (digestion). Transfer the solution containing the cells to a centrifuge tube of RNase-Free, centrifuge at 300 g for 5 min, collect the pellet and aspirate all the supernatant. Perform total RNA extraction according to the instructions for the Magnetic Bead Method Tissue/Cell/Blood Total RNA Extraction Kit.

Extraction of mRNA from total RNA:
The total RNA concentration previously extracted was detected by the UV spectrophotometer, and 1000 ng of total RNA was diluted to 50 µL with nuclease-free ddH2O, mRNA was extracted according to the instructions of the TIANSeq mRNA capture kit, and part of the sample was taken to detect the mRNA content (repeat the above experimental steps several times to obtain enough mRNA for transfection).

Experimental group: The group given mRNA extracted from "Hela cells transfected with pBS-L1PA1-CH-mneo-IT15-3 plasmid" was set as experimental group 14; The group given mRNA extracted from "Hela cells transfected with pBS-L1PA1-CH-mneo plasmid" was set to control group 7. Three parallels were set in each group, and each parallel was a 6-well plate cultured with Hela cells.

The resulting mRNA was transfected into cells with Lipofectamine MessengerMAX transfection reagent, and Hela cells cultured in 6-well plates in experimental group 14 and control group 7 were transfected. The first transfection is performed when cells grew to 40% confluency. For each well of transfected cells, 7.5 µL of Lipofectamine MessengerMAX transfection reagent is diluted with 125 µL of serum-free DMEM solution and incubated for 10 min at room temperature. After mixing 5 µg of the prepared mRNA with 125 µL of serum-free DMEM solution, mixing it with previously diluted 125 µL of Lipofectamine MessengerMAX transfection reagent and incubating for 5 min at room temperature. Add the resulting mixing solution to the culture medium of each well of cultured cells and mix gently. When the cells are 70% confluent, repeat the above operation.

Extraction of transfected cell DNA: After the cell culture medium was aspirated away, the cells were rinsed twice with PBS, digested with an appropriate amount of 0.25% trypsin, and digested at 37°C for 20min, with 15 times of pipetting every 5min. After the cells were suspended, complete medium containing serum was added to stop the reaction (digestion). Thereafter, extraction of cellular DNA was performed according to the product instruction of the blood/cell/tissue genomic DNA extraction kit, and the DNA concentration was determined by an ultraviolet spectrophotometer.

### qPCR test:

Since the GAPDH gene does not contain an Alu sequence and the copy number is stable, the GAPDH gene is used as an internal reference gene.

The upstream primer sequence of the GAPDH gene is shown in Seq ID No.7, and the downstream primer sequence is shown in Seq ID No. 8.

Design primer pair 6 with upstream primer sequences as shown in Seq ID No.25: 5' -GAAATTGGTTTGAGCAGGAG-3' ; The downstream primer sequence is shown in Seq ID No.26: 5'-CGATTGGATGGCAGTAGC-3'. The upstream primer sequence of primer pair 6 is located in the intact *IT15* gene, further upstream in front of the upstream sequence of the insertion site (target site) used on the plasmid, not in the plasmid, only in the genome, and the downstream primer sequence of primer pair 6 is located on the randomly designed non-homologous sequence (sequence to be inserted) to be inserted.

The above primers are obtained by chemical synthesis.

The qPCR reaction system is shown in Table 37.

**Table 37 qPCR reaction system**

| Component | Amount added |
|---|---|
| Upstream primer (10µM) | 1.5µL |
| Downstream primer (10µM) | 1.5µL |
| 2×SuperReal PreMix Plus | 25µE |
| Cell DNA template ( 25ng/µL ) | 4µL |
| ddH₂O without RNase | Make up to 50µL |

The cellular DNA template was DNA extracted from the aforementioned control group 7, and experimental group 14 after transfection.

The reaction system was prepared on ice, put the lid of the reaction tube on after preparation, mixed gently, and centrifuged briefly to ensure that all the components were at the bottom of the tube. Each 6-well plate cell sample was repeated 3 times simultaneously.

### qPCR reaction cycle:

Primer pair 6: pre-denaturation at 95°C for 15min; (denaturation at 95°C for 10s, annealing at 50°C for 20s, and extension at 72°C for 20s) 40 cycles. The GAPDH primers were reacted under the same conditions.

The exponential growth phases in the amplification curves of GAPDH and the detection of the insertion of the sequence to be inserted were observed and were confirmed to be approximately parallel, the obtained data were analyzed by the 2^{-ΔΔct} method, and the results are shown in Table 38. The PCR products were verified to be correct by sequencing.

**Table 38 Results for primer pair 6 < n=3 x±s)**

| Ct value | *GAPDH* | Inserti on of the sequen ce to be inserte d | ΔCt | ΔΔCt (mean ΔCt of the experimental group - mean ΔCt of the control group) | Relative amount of copy number (2^{-ΔΔCt}) |
|---|---|---|---|---|---|
| Control group 7 plate 1 | 26.05 | N/A | 13.95 | - | - |
| Control group 7 plate 2 | 26.41 | N/A | 13.59 | - | - |
| Control group 7 plate 3 | 26.49 | N/A | 13.51 | - | - |
| Control group 7 mean | | | 13.68±0.19 | 0.00±0.19 | 1.00 (0.88-1.14) |
| Experimental group 14 plate 1 | 25.55 | 25.03 | -0.52 | - | - |
| Experimental group 14 plate 2 | 24.90 | 24.67 | -0.23 | - | - |
| Experimental group 14 plate 3 | 25.12 | 24.99 | -0.13 | - | - |
| Experimental group 14 mean | | | -0.29±0.17 | -13.97±0.17 | 16046.82 ( 14263.10-18053.61) |

It can be seen from Table 38 that the relative amount of copy number in the experimental group 14 was significantly higher than that in the control group 7 (N/A is calculated according to 40.00), which is statistically significant (P<0.05), which indicates that the sequence to be inserted is efficiently inserted into a target site on the genome, indicating that gene editing by the RNA pathway of the present invention is effective.

Due to the feasibility of complete RNA mediation, it can be seen that if the coding sequence of ORF2p and/or ORF1p is not added to the sequence to be imported into the system to be edited, RNA products containing insertion site (target site) upstream sequence + sequence to be inserted + insertion site (target site) downstream sequence + SINE sequence (such as Alu sequence), partial SINE sequence or SINE-like sequence can bind ORF2p in vitro or bind ORF1p and ORF2p at the same time and then transfer into cells (cytoplasm) by RNP-mediated pathway.

### Example 11 the editing and fixing of CNVs ends by present invention

Based on the widespread phenomenon of CNVs extension in organisms, it can be deduced that the CNVs of each gene should have an end, specifically a certain sequence in the corresponding gene is connected downstream to the partial SINE (Alu) sequence, and the CNV is gradually extended by different lariat-partial SINE sequence (double-stranded DNA) continuously inserted in front of the partial SINE sequence in the CNV end. Since exons need to be cut from pre-mRNA, the end of the intron must form a lariat, and the probability of producing a lariat containing exons that overlap with it is low, so for genes with relatively low expression, there must be a relatively long period of time, with the CNV end at the end of the intron in the gene and connected to the partial SINE (Alu) sequence.

The present embodiment randomly selects the 3' sequence of an intron in the *BRCA1* gene, and selects it as the sequence to be deleted in the next embodiment; The 3 ' sequence of the *BRCA1* gene intron is shown in Seq ID No. 39:
CCCAGCTACTTGAGAGGCTGAGGCAGGGAGAATTGCTTGAACCAGGTAGG CGGAGGTTGCAGTGAGCCAAGATCGCACCACTGCACTCCAGCCTGGGGCA ACAGAGCAAGACTGTCTCAAAAAAAATAAATAAATAAAATAAATTCTTAA GAAGGATATTTTGGAAAACTCCTTACATACCTAAATTCTTTGTTTATCAAA TACTTGGACTTAGCACACTCTTCTTTGAAATGGACCAATAAACAACAGGAG CCCATAAGCAAAAAGAACTCATTATTTTAAAAACAGTAACTATCCTTACAG GCTTTCTCAGGGCTCTTTCTGTTGGATCCTTCCCTCTCACAGGTCCTTGCTA ATGATCTCTAGGTGGACACATTCTAGATGAGATGTCCCTGTCTAGAATGGC AGCACCATGAGGGCTATATCCTCAGTACTAGGACAGCGCCTGGTGCTTAAT AGATAGTAAATAGTTGTCTAATTAACTGAGCAAACAGATAGATTCATGAA TTAGCTTTTTGCTTTTTCTGTTAGAAACTAAAGGTTCAGGTCAGGCACAAT GGCGCATGTCTCTAATCCCAGCACTTTGGGAGGCCGAGGCGGGCTGATCA CTTGAGGTCAGGAGTTCAAGACCAGCCTGGCCAACATAGTAAAACCCTGT TTCTACAAAAATTACCAAAATTAGCCGGGCGTCTTGGCAAGCACCTGTAAT GCCAGCTACTTGAGAGGCTGAGGTGGGAGAATCGCTTGAACCTGGGAGGA AGAGGTTGCAGTGAGCCGAGATGGTGCCAACCTGGGTGACAGAGGGAGA CTTAAAAAAAAAAAGAAAGAAAGAAAGAAAAGAAACTAAAGGTTCAAAG AATCCCAGAAAAGGAAGAGTCCTCACAAGCCAGTAATCTAGGCAGGATTA CTGATAGTATTTTTATATTTGTTGTATTTTTATAAAATGCCATAGATAGAGG GCTTTTTTCAACATTACATCAGTCTAAAAATCACACATTTTTATATGAACTA ACCTAAATGTCTGATGAATCTCACAACACCAAGTCTTTGAAATGTGCCCAT ATAAATAAAATGTTAACAGATTCATGCTAATTTTAAATATCGATAGTGTTT AAATGCCTTAATTATTTTTTCACTCCCTAGCTTTAAAAGAAAATAACCAAC TTCAAAAGGACATCACAATAACATCAAGTCTATTTGGGGGAATTTGAGGA TTTTTTCCCTCACTAACATCATTTGGAAATAATTTCATGGGCATTAATTGCA TGAATGTGGTTAGATTAAAAGGTGTTCAGCTAGAACTTGTAGTTCCATACT AGGTGATTTCAATTCCTGTGCTAAAATTAATTTGTATGATATATTTTCATTT AATGGAAAGCTTCTCAAAGTATTTCATTTTCTTGGTGCCATTTATCGTTTTT GAAG, wherein the underscore part is the upstream sequence immediately adjacent to the 5' end of the sequence to be deleted, the sequence after the underscore is the sequence to be deleted (specifically the 3' sequence of an intron in the BRCA1 gene), and the wavy line is the 3' end sequence of the sequence to be deleted (deletion is carried out in the next embodiment, hence the name).

The sequence is constructed in the following order: the 3' end sequence of the sequence to be deleted + the randomly designed sequence that is not homologous to the BRCA1 gene + partial Alu sequence, and the NheI digestion site and protective bases are added at both ends of the sequence to construct the sequence.

Since the natural shear site that causes the Alu element transcript to produce RNA containing only a partial Alu sequence is inconsistently reported in different literatures, in order to prevent the inability to insert due to a mismatch with the partial Alu sequence in the CNV terminal, three possible partial Alu sequences were synthesized and introduced (the difference is due to their 5' end sequences), and the construction sequences are shown in Seq ID No.40, Seq ID No.41, Seq ID No.42.

The sequence Seq ID No.40 is: 
CTAGCTAGCTAGACTAACATCATTTGGAAATAATTTCATGGGCATTAATTG
CATGAATGTGGTTAGATTAAAAGGTGTTCAGCTAGAACTTGTAGTTCCATA
CTAGGTGATTTCAATTCCTGTGCTAAAATTAATTTGTATGATATATTTTCAT
TTAATGGAAAGCTTCTCAAAGTATTTCATTTTCTTGGTGCCATTTATCGTTT
TTGAAGGGGGGGAAAAATACAAAAAATTAGCCGGGCGTGATGGTGGGCG
CCTGTAGTCCCAGCTACACGGGAGGCTGAGACAGGAGAATGGCGTGAACC
CGGGAGGCGGAGCTTGCAGTGAGCCGAGATCGCACCACTGCACTGCAGCC
TGGGCGACAGAGCGAGACTCTGTCTCAAAAAAAAAAAAAAAGAAAGAAA
AAAAACTAGCTAGCTAG,
 where the underscore indicates a randomly designed non-homologous sequence (a sequence that is not homologous to the *BRCA1* gene), with NheI cleavage sites and protective bases (italic bold) at both ends of the sequence, and partial Alu sequence-Alu3 (shaded part) between the non-homologous sequence and the 3'-end NheI cleavage site and protective base sequence. The sequence was obtained by chemical synthesis and named BRCA1-1-Alu3.

The sequence Seq ID No.41 is: 
CTAGCTAGCTAGACTAACATCATTTGGAAATAATTTCATGGGCATTAATTG
CATGAATGTGGTTAGATTAAAAGGTGTTCAGCTAGAACTTGTAGTTCCATA
CTAGGTGATTTCAATTCCTGTGCTAAAATTAATTTGTATGATATATTTTCAT
TTAATGGAAAGCTTCTCAAAGTATTTCATTTTCTTGGTGCCATTTATCGTTT
TTGAAGGGGGGGCTAAAAATACAAAAAATTAGCCGGGCGTGATGGTGGGC
 
GCCTGTAGTCCCAGCTACACGGGAGGCTGAGACAGGAGAATGGCGTGAAC
CCGGGAGGCGGAGCTTGCAGTGAGCCGAGATCGCACCACTGCACTGCAGC
CTGGGCGACAGAGCGAGACTCTGTCTCAAAAAAAAAAAAAAAGAAAGAA
AAAAAACTAGCTAGCTAG,
 where the underscore indicates a randomly designed non-homologous sequence (a sequence that is not homologous to the *BRCA1* gene), with NheI cleavage sites and protective bases (italic bold) at both ends of the sequence, and partial Alu sequence-Alu4 (shaded part) between the non-homologous sequence and the 3'-end NheI cleavage site and protective base sequence. The sequence was obtained by chemical synthesis and named BRCA1-1-Alu4.

The sequence Seq ID No.42 is: 
CTAGCTAGCTAGACTAACATCATTTGGAAATAATTTCATGGGCATTAATTG
CATGAATGTGGTTAGATTAAAAGGTGTTCAGCTAGAACTTGTAGTTCCATA
CTAGGTGATTTCAATTCCTGTGCTAAAATTAATTTGTATGATATATTTTCAT
TTAATGGAAAGCTTCTCAAAGTATTTCATTTTCTTGGTGCCATTTATCGTTT
TTGAAGGGGGGGACTAAAAATACAAAAAATTAGCCGGGCGTGATGGTGG
GCGCCTGTAGTCCCAGCTACACGGGAGGCTGAGACAGGAGAATGGCGTGA
ACCCGGGAGGCGGAGCTTGCAGTGAGCCGAGATCGCACCACTGCACTGCA
GCCTGGGCGACAGAGCGAGACTCTGTCTCAAAAAAAAAAAAAAAGAAAG
AAAAAAAACTAGCTAGCTAG,
 where the underscore indicates a randomly designed non-homologous sequence (a sequence that is not homologous to the *BRCA1* gene), with NheI cleavage sites and protective bases (italic bold) at both ends of the sequence, and partial Alu sequence-Alu5 (shaded part) between the non-homologous sequence and the 3'-end NheI cleavage site and protective base sequence. The sequence was obtained by chemical synthesis and named BRCA1-1-Alu5.

Sequences that do not contain non-homologous sequences (sequences that are not homologous to the BRCA1 gene) are also designed, as shown in Seq ID No. 43, Seq ID No. 44 and Seq ID No. 45.

Seq ID No.43 sequence is: CTAGCTAGCTAGACTAACATCATTTGGAAATAATTTCATGGGCATTAATTG 
CATGAATGTGGTTAGATTAAAAGGTGTTCAGCTAGAACTTGTAGTTCCATA
CTAGGTGATTTCAATTCCTGTGCTAAAATTAATTTGTATGATATATTTTCAT
TTAATGGAAAGCTTCTCAAAGTATTTCATTTTCTTGGTGCCATTTATCGTTT
TTGAAGAAAAATACAAAAAATTAGCCGGGCGTGATGGTGGGCGCCTGTAG
TCCCAGCTACACGGGAGGCTGAGACAGGAGAATGGCGTGAACCCGGGAG
GCGGAGCTTGCAGTGAGCCGAGATCGCACCACTGCACTGCAGCCTGGGCG
ACAGAGCGAGACTCTGTCTCAAAAAAAAAAAAAAAGAAAGAAAAAAAAC
TAGCTAGCTAG,
 where Seq ID No.43 is BRCA1-1-Alu3 without non-homologous sequence. The sequence was obtained by chemical synthesis and named BRCA1-2-Alu3.

The sequence Seq ID No.44 is: 
CTAGCTAGCTAGACTAACATCATTTGGAAATAATTTCATGGGCATTAATTG
CATGAATGTGGTTAGATTAAAAGGTGTTCAGCTAGAACTTGTAGTTCCATA
CTAGGTGATTTCAATTCCTGTGCTAAAATTAATTTGTATGATATATTTTCAT
TTAATGGAAAGCTTCTCAAAGTATTTCATTTTCTTGGTGCCATTTATCGTTT
TTGAAGCTAAAAATACAAAAAATTAGCCGGGCGTGATGGTGGGCGCCTGT
AGTCCCAGCTACACGGGAGGCTGAGACAGGAGAATGGCGTGAACCCGGG
AGGCGGAGCTTGCAGTGAGCCGAGATCGCACCACTGCACTGCAGCCTGGG
CGACAGAGCGAGACTCTGTCTCAAAAAAAAAAAAAAAGAAAGAAAAAAA
ACTAGCTAGCTAG,
 where Seq ID No.44 is BRCA1-1-Alu4 without non-homologous sequence. The sequence was obtained by chemical synthesis and named BRCA 1-2-Alu4.

The sequence Seq ID No.45 is: 
CTAGCTAGCTAGACTAACATCATTTGGAAATAATTTCATGGGCATTAATTG
CATGAATGTGGTTAGATTAAAAGGTGTTCAGCTAGAACTTGTAGTTCCATA
CTAGGTGATTTCAATTCCTGTGCTAAAATTAATTTGTATGATATATTTTCAT
TTAATGGAAAGCTTCTCAAAGTATTTCATTTTCTTGGTGCCATTTATCGTTT
TTGAAGACTAAAAATACAAAAAATTAGCCGGGCGTGATGGTGGGCGCCTG
TAGTCCCAGCTACACGGGAGGCTGAGACAGGAGAATGGCGTGAACCCGGG
AGGCGGAGCTTGCAGTGAGCCGAGATCGCACCACTGCACTGCAGCCTGGG
 
CGACAGAGCGAGACTCTGTCTCAAAAAAAAAAAAAAAGAAAGAAAAAAA
ACTAGCTAGCTAG,
 where Seq ID No.45 is BRCA1-1-Alu5 without non-homologous sequence. The sequence was obtained by chemical synthesis and named BRCA 1-2-AluS.

BRCA1-1-Alu3, BRCA1-1-Alu4, BRCA1-1-Alu5, BRCA1-2-Alu3, BRCA1-2-Alu4, BRCA1-2-Alu5 were inserted into the plasmid vector pBS-L1PA1-CH-mneo, respectively, to construct plasmids pBS-L1PA1-CH-mneo-BRCA1-1-Alu3 , pBS-L1PA1-CH-mneo-BRCA1-1-Alu4, pBS-L1PA1-CH-mneo-BRCA1-1-Alu5 , pBS-L1PA1-CH-mneo-BRCA1-2-Alu3, pBS-L1PA1-CH-mneo-BRCA1-2-Alu4 andpBS-LlPAl-CH-mneo-BRCA1-2-Alu5, as follows:
BRCA1-1-Alu3, BRCA1-1-Alu4, BRCA1-1-Alu5, BRCA1-2-Alu3, BRCA1-2-Alu4, BRCA1-2-Alu5, and plasmid pBS-L1PA1-CH-mneo were digested separately, and the reaction system was shown in Table 39:

**Table 39 Digestion reaction system**

| Component | Added amount |
|---|---|
| BRCA1-1-Alu3 or BRCA1-1-Alu4 or BRCA1-1-Alu5 or BRCA1-2-Alu3 or BRCA1-2-Alu4 or BRCA1-2-Alu5 or pBS-L1PA1-CH-mneo | 1µg |
| NheI | 1µL |
| 10×digestion buffer | 5µL |
| ddH₂O | made up to 50µL |
| Total | 50µL |

The reaction conditions were: incubation at 37°C for 1h, then temperature to 65°C incubation for 20min to inactivate the endonuclease enzyme, electrophoresis, recovery of enzyme digestion products.

The digested BRCA1-1-Alu3, BRCA1-1-Alu4, BRCA1-1-Alu5, BRCA1-2-Alu3, BRCA1-2-Alu4 or BRCA1-2-Alu5 was ligated with the plasmid vector pBS-L1PA1-CH-mneo, and the reaction system was shown in Table 40:

**Table 40 Ligation reaction system**

| Component | Amount added |
|---|---|
| BRCA1-1-Alu3 or BRCA1-1-Alu4 or BRCA1-1-Alu5 or BRCA1-2-Alu3 or BRCA1-2-Alu4 or | 20ng |
| BRCA 1-2-AluS | |
| pBS-L1PA1-CH-mneo | 100ng |
| T4 DNA ligase | 1 Weiss unit |
| 10×ligation buffer | 1µL |
| Nuclease-free water | made up to 10µL |

The reaction conditions were: incubation at 16 °C for 16h, then temperature to 70 °C, incubation for 10min to inactivate ligase, electrophoresis and purification to obtain plasmids pBS-L1PA1-CH-mneo-BRCA1-1-Alu3, pBS-L1PA1-CH-mneo-BRCA1-1-Alu4, pBS-L1PA1-CH-mneo-BRCA1-1-Alu5 , pBS-L1PA1-CH-mneo-BRCA1-2-Alu3, pBS-L1PA1-CH-mneo-BRCA1-2-Alu4 andpBS-L1PA1-CH-mneo-BRCA1-2-AluS. The plasmid was sequenced to verify that it was correct.

The group transfected pBS-L1PA1-CH-mneo-BRCA1-1-Alu3 , pBS-L1PA1-CH-mneo-BRCA1-1-Alu4 and pBS-L1PA1-CH-mneo-BRCA1-1-Alu5 was set as experimental group 15, and the group transfected with pBS-L1PA1-CH-mneo-BRCA1-2-Alu3, pBS-L1PA1-CH-mneo-BRCA1-2-Alu4 andpBS-L1PA1-CH-mneo-BRCA1-2-Alu5 was set as control group 8. Three parallels were set in each group, and each parallel was a 6-well plate cultured with Hela cells.

The transfection steps were as follows: Hela cells were passaged and spread into 6-well plates. On the next day of passage, transfection was performed using Entranster-H4000 transfection reagent. For transfection of each plate of cells, 96µg (32µg for each plasmid) constructed plasmid was diluted with 300µL serum-free DMEM and mixed thoroughly. At the same time, 120µL of Entranster-H4000 reagent was diluted with 300µL of serum-free DMEM, and after fully mixed, it was allowed to stand for 5min at room temperature. Then the prepared two liquids were mixed and fully mixed, and allowed to stand for 15min at room temperature to prepare the transfection complex. The transfection complex was added to 6-wellplate with Hela cells, in which contained 2ml DMEM containing 10% fetal bovine serum per well, for transfection. After the cells grew to about 90% confluence, they were passaged, and the above operations were repeated after passage. After the cells grew to about 90% confluence again, samples were taken for subsequent operations.

Extraction of transfected cell DNA: After the cell culture medium was aspirated away, the cells were rinsed twice with PBS, digested with an appropriate amount of 0.25% trypsin, and digested at 37°C for 20min, with 15 times of pipetting every 5min. After the cells were suspended, complete medium containing serum was added to stop the reaction (digestion). Thereafter, extraction of cellular DNA was performed according to the product instruction of the blood/cell/tissue genomic DNA extraction kit, and the DNA concentration was determined by an ultraviolet spectrophotometer.

### qPCR test:

Since the GAPDH gene does not contain an Alu sequence and the copy number is stable, the GAPDH gene is used as an internal reference gene.

The upstream primer sequence of the GAPDH gene is shown in Seq ID No.7, and the downstream primer sequence is shown in Seq ID No. 8.

Design primer pair 9 with upstream primer sequences as shown in Seq ID No.46: 5'-CCCCTTTATCTCCTTCTG-3'; The downstream primer sequence is shown in Seq ID No.47: 5'- ATTTCTCCCATTCCACTT-3'. The upstream primer sequence of primer pair 9 is located downstream of the 3' end sequence of the sequence to be deleted in the intact *BRCA1* gene, not present in the plasmid and exists only on the genome, and the downstream primer sequence of primer pair 9 is located downstream of the 3' end sequence of the sequence to be deleted in the intact *BRCA1* gene, not in the plasmid, only on the genome.

The above primers are obtained by chemical synthesis.

The qPCR reaction system is shown in Table 41.

**Table 41 qPCR reaction system**

| Component | Amount added |
|---|---|
| Upstream primer (10µM) | 1.5µL |
| Downstream primer (10µM) | 1.5µL |
| 2 × SuperReal PreMix Plus | 25µL |
| Cell DNA template (25ng/µL) | 4µL |
| ddH₂O without RNase | Make up to 50µL |

The cellular DNA template was DNA extracted from the aforementioned control group 8, and experimental group 15 after transfection.

The reaction system was prepared on ice, put the lid of the reaction tube on after preparation, mixed gently, and centrifuged briefly to ensure that all the components were at the bottom of the tube. Each 6-well plate cell sample was repeated 3 times simultaneously.

### qPCR reaction cycle:

Primer pair 8: pre-denaturation at 95°C for 15min; (denaturation at 95°C for 10s, annealing at 46°C for 20s, and extension at 72°C for 20s) 40 cycles. The GAPDH primers were reacted under the same conditions.

After observing the exponential growth period in the amplification curve of GAPDH and detecting downstream sequence of the 3' end sequence of the sequence to be deleted, and confirming that it is approximately parallel, the obtained data were analyzed by the 2^{-ΔΔCt} method, and the results are shown in Table 42. The PCR products were verified to be correct by sequencing.

**Table 42 Results for primer pair 9 (n=3, x̅±s)**

| Ct value | *GAPDH* | Upstre am sequen ce | ΔCt | ΔΔCt (mean ΔCt of the experimental group - mean ΔCt of the control group) | Relative amount of copy number (2^{-ΔΔCt}) |
|---|---|---|---|---|---|
| Control group 8 plate 1 | 24.16 | 23.30 | -0.86 | - | - |
| Control group 8 plate 2 | 23.52 | 22.44 | -1.08 | - | - |
| Control group 8 plate 3 | 24.34 | 23.65 | -0.69 | - | - |
| Control group 8 mean | | | -0.88±0.16 | 0.00±0.16 | 1.00 (0.90-1.12) |
| Experimental group 15 plate 1 | 26.07 | 25.69 | -0.38 | - | - |
| Experimental group 15 plate 2 | 25.47 | 25.18 | -0.29 | - | - |
| Experimental group 15 plate 3 | 25.83 | 25.43 | -0.40 | - | - |
| Experimental group 15 mean | | | -0.36±0.05 | 0.52±0.05 | 0.70 (0.67-0.72) |

The relative amount of copy number in the experimental group 15 (N/A calculated according to 40.00) was significantly lower than that in the control group 8, which was statistically significant (P<0.05), this means that the copy number of the downstream sequence of the gene part of the CNV end in the corresponding intact gene in the experimental group 15 is less than that of the control group 8, indicating that the insertion of non-homologous sequences into the CNV terminal hinders the downstream extension of the gene part of the CNV end.

Conclusion: It can be seen that the insertion of non-homologous sequences in the CNV end hinders the extension of the corresponding CNV.

### Example 12 Cutting (shortening) the CNV end

The 3' sequence of one of the introns of the *BRCA1* gene was selected (located in Seq ID No. 39 in Example 11), and the sequence was synthesized in the order of the 3' end sequence of the sequence to be deleted + non-homologous sequence + upstream sequence immediately adjacent to the 5' end of the sequence to be deleted + partial Alu sequence and NheI digestion sites and protective bases were added at both ends, as shown in Seq ID No. 48, Seq ID No. 49 and Seq ID No. 50.

Seq ID No.48 sequence is: 
CTAGCTAGCTAGACTAACATCATTTGGAAATAATTTCATGGGCATTAATTG
CATGAATGTGGTTAGATTAAAAGGTGTTCAGCTAGAACTTGTAGTTCCATA
CTAGGTGATTTCAATTCCTGTGCTAAAATTAATTTGTATGATATATTTTCAT
 
TTAATGGAAAGCTTCTCAAAGTATTTCATTTTCTTGGTGCCATTTATCGTTT
TTGAAGGGGGGGCCCAGCTACTTGAGAGGCTGAGGCAGGGAGAATTGCTT
GAACCAGGTAGGCGGAGGTTGCAGTGAGCCAAGATCGCACCACTGCACTC
CAGCCTGGGGCAACAGAGCAAGACTGTCTCAAAAAAAATAAATAAATAA
AATAAATTCTTAAAAAAATACAAAAAATTAGCCGGGCGTGATGGTGGGCG
CCTGTAGTCCCAGCTACACGGGAGGCTGAGACAGGAGAATGGCGTGAACC
CGGGAGGCGGAGCTTGCAGTGAGCCGAGATCGCACCACTGCACTGCAGCC
TGGGCGACAGAGCGAGACTCTGTCTCAAAAAAAAAAAAAAAGAAAGAAA
AAAAACTAGCTAGCTAG,
 wherein the underscore indicates a randomly designed non-homologous sequence, at both ends of the sequence is the NheI digestion site and the protective base (italic bold), between the non-homologous sequence and the shadow sequence is the upstream sequence immediately adjacent to the 5' end of the sequence to be deleted, and the shadow sequence is a partial Alu sequence-Alu3. The sequence was obtained by chemical synthesis and named BRCA1-3-Alu3.

The sequence Seq ID No.49 is: 
CTAGCTAGCTAGACTAACATCATTTGGAAATAATTTCATGGGCATTAATTG
CATGAATGTGGTTAGATTAAAAGGTGTTCAGCTAGAACTTGTAGTTCCATA
CTAGGTGATTTCAATTCCTGTGCTAAAATTAATTTGTATGATATATTTTCAT
TTAATGGAAAGCTTCTCAAAGTATTTCATTTTCTTGGTGCCATTTATCGTTT
TTGAAGGGGGGGCCCAGCTACTTGAGAGGCTGAGGCAGGGAGAATTGCTT
GAACCAGGTAGGCGGAGGTTGCAGTGAGCCAAGATCGCACCACTGCACTC
CAGCCTGGGGCAACAGAGCAAGACTGTCTCAAAAAAAATAAATAAATAA
AATAAATTCTTAACTAAAAATACAAAAAATTAGCCGGGCGTGATGGTGGG
CGCCTGTAGTCCCAGCTACACGGGAGGCTGAGACAGGAGAATGGCGTGAA
CCCGGGAGGCGGAGCTTGCAGTGAGCCGAGATCGCACCACTGCACTGCAG
CCTGGGCGACAGAGCGAGACTCTGTCTCAAAAAAAAAAAAAAAGAAAGA
AAAAAAACTAGCTAGCTAG,
 wherein the underscore indicates a randomly designed non-homologous sequence, at both ends of the sequence is the NheI digestion site and the protective base (italic bold), between the non-homologous sequence and the shadow sequence is the upstream sequence immediately adjacent to the 5' end of the sequence to be deleted, and the shadow sequence is a partial Alu sequence-Alu4.

The sequence was obtained by chemical synthesis and named BRCA1-3-Alu4.

The sequence Seq ID No.50 is: 
CTAGCTAGCTAGACTAACATCATTTGGAAATAATTTCATGGGCATTAATTG
CATGAATGTGGTTAGATTAAAAGGTGTTCAGCTAGAACTTGTAGTTCCATA
CTAGGTGATTTCAATTCCTGTGCTAAAATTAATTTGTATGATATATTTTCAT
TTAATGGAAAGCTTCTCAAAGTATTTCATTTTCTTGGTGCCATTTATCGTTT
TTGAAGGGGGGGCCCAGCTACTTGAGAGGCTGAGGCAGGGAGAATTGCTT
GAACCAGGTAGGCGGAGGTTGCAGTGAGCCAAGATCGCACCACTGCACTC
CAGCCTGGGGCAACAGAGCAAGACTGTCTCAAAAAAAATAAATAAATAA
AATAAATTCTTAAACTAAAAATACAAAAAATTAGCCGGGCGTGATGGTGG
GCGCCTGTAGTCCCAGCTACACGGGAGGCTGAGACAGGAGAATGGCGTGA
ACCCGGGAGGCGGAGCTTGCAGTGAGCCGAGATCGCACCACTGCACTGCA
GCCTGGGCGACAGAGCGAGACTCTGTCTCAAAAAAAAAAAAAAAGAAAG
AAAAAAAACTAGCTAGCTAG,
 wherein the underscore indicates a randomly designed non-homologous sequence, at both ends of the sequence is the NheI digestion site and the protective base (italic bold), between the non-homologous sequence and the shadow sequence is the upstream sequence immediately adjacent to the 5' end of the sequence to be deleted, and the shadow sequence is a partial Alu sequence-Alu5. The sequence was obtained by chemical synthesis and named BRCA1-3-Alu5.

BRCA1-3-Alu3, BRCA1-3-Alu4 and BRCA1-3-Alu5 were inserted into the plasmid vector pBS-L1PA1-CH-mneo, respectively, to construct plasmids pBS-L1PA1-CH-mneo-BRCA1-3-Alu3, pBS-L1PA1-CH-mneo-BRCA1-3-Alu4 andpBS-L1PA1-CH-mneo-BRCA1-3-Alu5, as follows:
BRCA1-3-Alu3, BRCA1-3-Alu4, BRCA1-3-Alu5, and plasmid pBS-L1PA1-CH-mneo were digested separately, and the reaction system was shown in Table 43:

**Table 43 Digestion reaction system**

| Component | Added amount |
|---|---|
| BRCA 1-3 -Alu3 or BRCA1-3-Alu4 or BRCA1-3-Alu5 or pBS-L1PA1-CH-mneo | 1µg |
| NheI | 1µL |
| 10 × digestion buffer | 5µL |
| ddH₂O | made up to 50µL |
| Total | 50µL |

The reaction conditions were: incubation at 37°C for 1h, then temperature to 65°C incubation for 20min to inactivate the endonuclease enzyme, electrophoresis, recovery of enzyme digestion products.

The digested BRCA1-3-Alu3, BRCA1-3-Alu4 and BRCA1-3-Alu5 was ligated with the plasmid vector pBS-L1PA1-CH-mneo, and the reaction system was shown in Table 44:

**Table 44 Ligation reaction system**

| Component | Amount added |
|---|---|
| BRCA1-3-Alu3 or BRCA1-3-Alu4 or BRCA1-3-Alu5 | 20ng |
| pBS-LIPAI-CH-mneo | 100ng |
| T4 DNA ligase | 1 Weiss unit |
| 10×ligation buffer | 1µL |
| Nuclease-free water | made up to 10µL |

The reaction conditions were: incubation at 16 °C for 16h, then temperature to 70 °C, incubation for 10min to inactivate ligase, electrophoresis and purification to obtain plasmids pBS-L1PA1-CH-mneo-BRCA1-3-Alu3, pBS-L1PA1-CH-mneo-BRCA1-3-Alu4 and pBS-L1PA1-CH-mneo-BRCA1-3-Alu5. The plasmid was sequenced to verify that it was correct.

The group transfected pBS-L1PA1-CH-mneo-BRCA1-3-Alu3 , pBS-L1PA1-CH-mneo-BRCA1-3-Alu4 and pBS-L1PA1-CH-mneo-BRCA1-3-Alu5 was set as experimental group 16, and the group transfected with pBS-L1PA1-CH-mneo-BRCA1-1-Alu3, pBS-L1PA1-CH-mneo-BRCA1-1-Alu4 and pBS-L1PA1-CH-mneo-BRCA1-1-Alu5 was set as control group 9. Three parallels were set in each group, and each parallel was a 6-well plate cultured with Hela cells.

The transfection steps were as follows: Hela cells were passaged and spread into 6-well plates. On the next day of passage, transfection was performed using Entranster-H4000 transfection reagent. For transfection of each plate of cells, 96µg (32µg for each plasmid) constructed plasmid was diluted with 300µL serum-free DMEM and mixed thoroughly. At the same time, 120µL of Entranster-H4000 reagent was diluted with 300µL of serum-free DMEM, and after fully mixed, it was allowed to stand for 5min at room temperature. Then the prepared two liquids were mixed and fully mixed, and allowed to stand for 15min at room temperature to prepare the transfection complex. The transfection complex was added to 6-well plate with Hela cells, in which contained 2ml DMEM containing 10% fetal bovine serum per well, for transfection. After the cells grew to about 90% confluence, they were passaged, and the above operations were repeated after passage. After the cells grew to about 90% confluence again, samples were taken for subsequent operations.

Extraction of transfected cell DNA: After the cell culture medium was aspirated away, the cells were rinsed twice with PBS, digested with an appropriate amount of 0.25% trypsin, and digested at 37°C for 20min, with 15 times of pipetting every 5min. After the cells were suspended, complete medium containing serum was added to stop the reaction (digestion). Thereafter, extraction of cellular DNA was performed according to the product instruction of the blood/cell/tissue genomic DNA extraction kit, and the DNA concentration was determined by an ultraviolet spectrophotometer.

### qPCR test:

Since the GAPDH gene does not contain an Alu sequence and the copy number is stable, the GAPDH gene is used as an internal reference gene.

The upstream primer sequence of the GAPDH gene is shown in Seq ID No.7, and the downstream primer sequence is shown in Seq ID No. 8.

Design primer pair 10 with upstream primer sequences as shown in Seq ID No.51: 5'-GCTTTCTCAGGGCTCTTT-3'; The downstream primer sequence is shown in Seq ID No.52: 5'-GCACCATCTCGGCTCACT-3'. The upstream primer of primer pair 10 is located on the sequence to be deleted on the genome and is not present in the plasmid, and the downstream primer is located on the sequence to be deleted on the genome and is not present in the plasmid.

The above primers are obtained by chemical synthesis.

The qPCR reaction system is shown in Table 45.

**Table 45 qPCR reaction system**

| Component | Amount added |
|---|---|
| Upstream primer (10µM) | 1.5µL |
| Downstream primer (10µM) | 1.5µL |
| 2 × SuperReal PreMix Plus | 25µL |
| Cell DNA template (25ng/µL) | 4µL |
| ddH₂O without RNase | Make up to 50µL |

The cellular DNA template was DNA extracted from the aforementioned control group 9, and experimental group 16 after transfection.

The reaction system was prepared on ice, put the lid of the reaction tube on after preparation, mixed gently, and centrifuged briefly to ensure that all the components were at the bottom of the tube. Each 6-well plate cell sample was repeated 3 times simultaneously.

### qPCR reaction cycle:

Primer pair 10: pre-denaturation at 95°C for 15min; (denaturation at 95°C for 10s, annealing at 49°C for 20s, and extension at 72°C for 20s) 40 cycles. The GAPDH primers were reacted under the same conditions.

After observing the exponential growth period in the amplification curve of GAPDH and detecting sequence to be deleted, and confirming that it is approximately parallel, the obtained data were analyzed by the 2^{-ΔΔCt} method, and the results are shown in Table 46. The PCR products were verified to be correct by sequencing.

**Table 46 Results for primer pair 10 (n=3, x̅±s)**

| Ct value | *GAPDH* | The sequen ce to be deleted | ΔCt | ΔΔCt (mean ΔCt of the experimental group - mean ΔCt of the control group) | Relative amount of copy number (2^{-ΔΔCt}) |
|---|---|---|---|---|---|
| Control group 9 plate 1 | 25.35 | 24.68 | -0.67 | - | - |
| Control group 9 plate 2 | 25.87 | 25.14 | -0.73 | - | - |
| Control group 9 plate 3 | 25.40 | 24.31 | -1.09 | - | - |
| Control group 9 mean | | | -0.83±0.19 | 0.00±0.19 | 1.00 (0.88-1.14) |
| Experimental group 16 plate 1 | 26.17 | 25.92 | -0.25 | - | - |
| Experimental group 16 plate 2 | 26.01 | 25.57 | -0.44 | - | - |
| Experimental group 16 plate 3 | 25.46 | 25.16 | -0.30 | - | - |
| Experimental group 16 mean | | | -0.33±0.08 | 0.50±0.08 | 0.71 (0.67-0.75) |

The relative amount of copy number in the experimental group 16 (N/A calculated according to 40.00) was significantly lower than that in the control group 9, which was statistically significant (P<0.05), it was indicated that the sequence to be deleted was deleted, and the gene part sequence of the CNV terminal (i.e., the CNV end) was reduced in the experimental group 16.

From Example 11, it can be seen that a non-homologous sequence may be inserted at the CNV end to hinder its further extension; Example 12 shows that the sequence of the gene part of the CNV terminal is significantly less than that of the control group in the experimental group, indicating that the CNV end is clipped while the CNV is shortened, proving that the CNV end can be modified by the relevant methods in the present invention. Therefore, it is also possible to modify many or all CNVs by changing the guide sequence upstream of the insertion point in the editing method (i.e., the upstream sequence of the target site) (when editing the CNV end, it is the same as the gene part sequence of the CNV end).

### Example 13 The corresponding gene expression changes after CNV end clipping

Total RNA was extracted from cells in the experimental group 16 and control group 9 in Example 12:
Extraction of transfected cell RNA: After the cell culture medium was aspirated away, the cells were rinsed twice with PBS, digested with an appropriate amount of 0.25% trypsin, and digested at 37°C for 20min, with 15 times of pipetting every 5min. After the cells were suspended, complete medium containing serum was added to stop the reaction (digestion). Transfer the solution containing the cells to a centrifuge tube of RNase-Free, centrifuge at 300 g for 5 min, collect the pellet and aspirate all the supernatant. Perform total RNA extraction according to the instructions for the Magnetic Bead Method Tissue/Cell/Blood Total RNA Extraction Kit.

Extraction of mRNA from total RNA:
The total RNA concentration previously extracted was detected by the UV spectrophotometer, and 1000 ng of total RNA was diluted to 50 µL with nuclease-free ddH2O, mRNA was extracted according to the instructions of the TIANSeq mRNA capture kit, and part of the sample was taken to detect the mRNA content (repeat the above experimental steps several times to obtain enough mRNA for transfection).

Reverse transcription synthesis of cDNA templates:
The cDNA is synthesized according to the instructions of the FastKing cDNA First Strand Synthesis Kit, and the concentration of the synthesized cDNA is determined by the UV spectrophotometer for subsequent testing.

### qPCR test:

Since the expression of *GAPDH* gene is relatively stable in various tissues, the *GAPDH* gene is used as the internal reference gene.

The upstream primer sequence of the *GAPDH* gene is shown in Seq ID No.7, and the downstream primer sequence is shown in Seq ID No. 8.

Design primer pair 11 with upstream primer sequences as shown in Seq ID No.53: 5'-CAGAGGACAATGGCTTCCATG-3'; The downstream primer sequence is shown in Seq ID No.54: 5'-CTACACTGTCCAACACCCACTCTC-3'. The upstream primer sequence of primer pair 11 is located on the BRCA1 gene and is not present in the plasmid and exists only on the genome, and the downstream primer sequence of primer pair 11 is located on the BRCA1 gene and is not present in the plasmid and exists only on the genome.

The above primers are obtained by chemical synthesis.

The qPCR reaction system is shown in Table 47.

**Table 47 qPCR reaction system**

| Component | Amount added |
|---|---|
| Upstream primer (10µM) | 1.5µL |
| Downstream primer (10µM) | 1.5µL |
| 2 × SuperReal PreMix Plus | 25µL |
| Cell DNA template (25ng/µL) | 8µL |
| ddH₂O without RNase | Make up to 50µL |

The cell DNA templates were cDNA synthesized from mRNA extracted from control group 9 and experimental group 16 after transfection.

The reaction system was prepared on ice, put the lid of the reaction tube on after preparation, mixed gently, and centrifuged briefly to ensure that all the components were at the bottom of the tube. Each 6-well plate cell sample was repeated 3 times simultaneously.

### qPCR reaction cycle:

Primer pair 11: pre-denaturation at 95°C for 15min; (denaturation at 95°C for 10s, annealing at 55°C for 20s, and extension at 72°C for 20s) 40 cycles. The GAPDH primers were reacted under the same conditions.

After observing the exponential growth period in the amplification curve of expression of *GAPDH* and *BRCA1*, and confirming that it is approximately parallel, the obtained data were analyzed by the 2^{-ΔΔCt} method, and the results are shown in Table 48. The PCR products were verified to be correct by sequencing.

**Table 48 Results for primer pair 11 (n=3, x̅±s)**

| Ct value | *GAPDH* | *BRCA1* | ΔCt | ΔΔCt (mean ΔCt of the experimental group - mean ΔCt of the control group) | Relative amount of copy number (2^{-ΔΔCt}) |
|---|---|---|---|---|---|
| Control group | 19.70 | 23.82 | 4.12 | - | - |
| 9 plate 1 | | | | | |
| Control group 9 plate 2 | 19.11 | 23.05 | 3.94 | - | - |
| Control group 9 plate 3 | 18.60 | 23.07 | 4.47 | - | - |
| Control group 9 mean | | | 4.18±0.22 | 0.00±0.22 | 1.00 (0.86-1.16) |
| Experimental group 16 plate 1 | 20.17 | 24.91 | 4.74 | - | - |
| Experimental group 16 plate 2 | 19.13 | 24.36 | 5.23 | - | - |
| Experimental group 16 plate 3 | 20.35 | 25.18 | 4.83 | - | - |
| Experimental group 16 mean | | | 4.93±0.21 | 0.75±0.21 | 0.59 (0.51-0.69) |

The relative expression of *BRCA1* gene in the experimental group 16 was lower than that in the control group 9, which was statistically significant (P<0.05), it was indicated that the CNV terminal clipping of *BRCA1* gene led to a decrease in its expression.

Conclusion: It can be seen that the expression of *BRCA1* gene is reduced, indicating that the editing of CNV does affect the transcription of the corresponding gene, which in turn can affect the expression of protein and the state of cells, tissues or organisms. As can be seen from examples 11 to 13, based on the present invention can fix, extend and clip the CNV end, and simultaneously affect the gene transcription and protein expression of cells. In addition, CNVs also change with physiological processes such as embryonic and ontogenetic development and tumorigenesis and differ in different cells, tissues and individuals, so editing CNVs can also change the corresponding cell, tissue and organism state.

As can be seen from the above embodiments, the present invention uses the retrotransposons and their reverse transcription function widely present in eukaryotes to edit the genome, wherein the SINE, LINE sequence and related proteins involved are widely present in normal organisms, without producing double-strand breaks, more accurate targeted sequence recognition and cutting, the target fragment is integrated into the genome, and the corresponding fragment can be deleted and replaced. Since there is no double-strand break, there is no need to worry about the danger of genomic double-strand DNA break and the introduction of unexpected random sequences. Taking the Alu element sequence in SINE and its function corresponding to LINE-1 in LINE as an example, Alu element and LINE-1 are widely distributed in the genome of primates, and in a specific embodiment, the sequence to be inserted relies on the sequence on both sides of the sequence to be inserted (upstream sequence of the target site and downstream sequence of the target site) to be guided to the inserted site (target site) on the genome,

Moreover, ORF2p can only slide smoothly from the 3' end of the carrier nucleic acid to the shear site for single-stranded cutting on the genome under the condition that the upstream sequence of the target site is completely matched, which greatly improves its targeting accuracy and avoids the occurrence of unexpected cutting, and its targeting accuracy is theoretically higher than that of other gene editing techniques currently existing.

In addition, the sequence of interest, genes and genomes can be modified through RNA or RNP pathways without introducing DNA fragments and entering the nucleus during transfection by generating the required RNA and corresponding proteins such as ORF1p and ORF2p in vitro.

With the nuclear localization function of ORF1p (and ORF2p), RNA and proteins transfected into cells can be guided into the nucleus, which is beneficial for editing cells that are difficult to manipulate due to the difficulty of entering the nucleus of the vector. At the same time, the present invention can also edit the CNVs on the genome, so that the CNVs are increased, decreased or "stabilized (cannot continue to change)" through the present invention, because CNVs can directly affect protein expression, etc., the operation of CNVs can change or stabilize the expression and state of the corresponding cells, tissues, organs or organisms (individuals). Since various types of SINE and LINE homologous and functionally similar to Alu elements and LINE-1, such as various MIRs and LINE-2, are widely distributed in eukaryotes, the present invention can also be applied to other eukaryotic systems.

Different from other gene editing technologies, the relevant mechanisms used in the present invention exist in normal organisms, without the introduction of foreign mechanisms and systems, reducing the impact on the receiving system for gene editing. Since it does not introduce foreign systems such as proteins derived from prokaryotes, and does not produce double-strand breaks, the present invention is easier to apply to clinical practice than other existing gene editing technologies.

## Claims

1. A gene transcription framework, **characterized in** comprising an upstream sequence of target site, a sequence to be inserted, a downstream sequence of target site along a 5'→3' direction;
wherein the gene transcription framework is a segment of DNA sequence which can be transcribed by RNA polymerase I, RNA polymerase II or RNA polymerase III, and
in the transcription product of the gene transcription framework or the transformation product of the transcription product, the upstream sequence of target site or the complementary sequence thereof can hybridize with the upstream sequence or the complementary sequence thereof of the corresponding target site in the cell genome, and the downstream sequence of target site or the complementary sequence thereof can hybridize with the downstream sequence or the complementary sequence thereof of the corresponding target site in the cell genome, and
the upstream sequence of target site and the downstream sequence of target site are directly connected in the corresponding target gene sequence in the genome, and the site between the upstream sequence of target site and the downstream sequence of target site in the target gene sequence in the genome is the target site of the sequence to be inserted.

2. The gene transcription framework according to claim 1, **characterized in that**, the cell is a eukaryotic cell.

3. A vector system comprising one or more vector(s) comprising:
one or more 1) the gene transcription framework according to claim 1;
one or more 2) short interspersed element(s) and/or partial short interspersed element(s) and/or short interspersed-like element(s), and/or
one or more 3) long interspersed element(s) and/or ORF1p coding sequence(s) and/or ORF2p coding sequence(s);
wherein the components 1), 2) and/or 3) are located on the same or different vectors of the vector system; and
the vector is provided with one or more promoter(s) which is/are an RNA polymerase I promoter, an RNA polymerase II promoter or an RNA polymerase III promoter, and is/are located upstream of the components 1), 2) and/or 3); and
the vector system is mediated by the DNA, RNA and/or RNP pathway.

4. The vector system according to claim 3, **characterized in that**,
the components 1), when more than one exists, are on the same or different vectors of the vector system; and
the components 2), when more than one exists, are on the same or different vectors of the vector system; and
the components 3), when more than one exists, are on the same or different vectors of the vector system.

5. The vector system according to claim 3, **characterized in that**, the vector is a eukaryotic expression vector, a prokaryotic expression vector, a viral vector, a plasmid vector, an artificial chromosome, a phage vector, or a cosmid vector.

6. The vector system according to claim 3, **characterized in that**, the vector is an expression vector, a cloning vector, a sequencing vector, a transformation vector, a shuttle vector, or a multifunctional vector.

7. The vector system according to claim 3, **characterized in that**, when 1) and 2) are located on the same vector, the short interspersed element, and/or the partial short interspersed element, and/or the short interspersed-like element are located downstream of the gene transcription framework, and the gene transcription framework is connected directly or indirectly to the short interspersed element, and/or the partial short interspersed element, and/or the short interspersed-like element;
when directly connected, the gene transcription framework shares a promotor with the short interspersed element, and/or the partial short interspersed element, and/or the short interspersed-like element;
when indirectly connected, the gene transcription framework shares or does not share a promotor with the short interspersed element, and/or the partial short interspersed element, and/or the short interspersed-like element.

8. The vector system according to claim 3, **characterized in that**, when 1) and 3) are located on the same vector, the one or more long interspersed element(s) and/or one or more ORF1p coding sequence(s), and/or the one or more ORF2p coding sequence(s) are located upstream and/or downstream of the gene transcription framework, and the gene transcription framework is linked directly or indirectly to the one or more long interspersed element(s), and/or the one or more ORF1p coding sequence(s), and/or the one or more ORF2p coding sequence(s);
when directly connected, the gene transcription framework shares a promotor with the one or more long interspersed element(s), and/or one or more ORF1p coding sequence(s), and/or one or more ORF2p coding sequence(s);
when indirectly connected, the gene transcription framework shares or does not share a promotor with the one or more long interspersed element(s), and/or one or more ORF1p coding sequence(s), and/or one or more ORF2p coding sequence(s).

9. The vector system according to claim 3, **characterized in that**, when 1), 2) and 3) are located on the same vector, the short interspersed element and/or the partial short interspersed element and/or the short interspersed-like element are located downstream of the gene transcription framework and/or downstream of the long interspersed element and/or the ORF1p coding sequence and/or the ORF2p coding sequence;
when the short interspersed element and/or the partial short interspersed element and/or the short interspersed-like element are located downstream of the gene transcription framework, the long interspersed element and/or the ORF1p coding sequence and/or the ORF2p coding sequence are located upstream of the gene transcription framework, and/or the long interspersed element and/or the ORF1p coding sequence and/or the ORF2p coding sequence are located downstream of the short interspersed element and/or the partial short interspersed element and/or the short interspersed-like element;
when the short interspersed element and/or the partial short interspersed element and/or the short interspersed-like element are located downstream of the long interspersed element and/or the ORF1p coding sequence and/or the ORF2p coding sequence, the long interspersed element and/or the ORF1p coding sequence and/or the ORF2p coding sequence are located downstream of the gene transcription framework; and
the gene transcription framework, the short interspersed element and/or the partial short interspersed element and/or the short interspersed-like element, and the long interspersed element and/or the ORF1p coding sequence and/or the ORF2p coding sequence are directly connected or indirectly connected;
when directly connected, the gene transcription framework, the short interspersed element and/or the partial short interspersed element and/or the short interspersed-like element, and the long interspersed element and/or the ORF1p coding sequence and/or the ORF2p coding sequence share a promoter;
when indirectly connected, the gene transcription framework, the short interspersed element and/or the partial short interspersed element and/or the short interspersed-like element, and the long interspersed element and/or the ORF1p coding sequence and/or the ORF2p coding sequence share or do not share a promoter.

10. The vector system according to claim 3, **characterized in that**, when 2) and 3) are located on the same vector, the long interspersed element and/or the ORF1p coding sequence and/or the ORF2p coding sequence are located upstream and/or downstream of the short interspersed element and/or the partial short interspersed element and/or the short interspersed-like element, and the short interspersed element and/or the partial short interspersed element and/or the short interspersed-like element are directly connected or indirectly connected to the long interspersed element and/or the ORF1p coding sequence and/or the ORF2p coding sequence,
when directly connected, the short interspersed element and/or partially short interspersed element and/or short interspersed-like element share a promoter with the long interspersed element and/or the ORF1p coding sequence and/or the ORF2p coding sequence;
when indirectly connected, the short interspersed element and/or partially short interspersed element and/or short interspersed-like element share or do not share a promoter with the long interspersed element and/or the ORF1p coding sequence and/or the ORF2p coding sequence.

11. A genome sequence editing method, **characterized in** comprising the steps of:
1) selecting a site to be inserted of a target gene to be edited in a genome, and determining an upstream sequence and a downstream sequence of the site to be inserted of the target gene on two sides of the site to be inserted;
2) preparing the vector system according to any of claims 3 to 10;
3) transforming or transfecting the vector system into cells, tissues, or organisms for transcription.

12. Use of the vector system according to any of claims 3 to 10 mediated by the DNA, RNA and/or RNP pathway for insertion, deletion, substitution of DNA sequence(s) in any region of the genome.

13. The use according to claim 12, **characterized in that**, the DNA sequence is one or more CNV sequence(s), CNV terminal sequence(s), short interspersed element(s), partial short interspersed element(s), long interspersed element(s), partial long interspersed element(s), ORF1p coding sequence and/or the ORF2p coding sequence.

14. Use of the vector system according to any of claims 3 to 10 as a medicament for preventing and/or treating cancer, gene related genetic disorder, or neurodegenerative diseases.

15. The use according to claim 14, **characterized in that**, the cancer is a glioma, breast cancer, cervical cancer, lung cancer, stomach cancer, colorectal cancer, duodenal cancer, leukemia, prostate cancer, endometrial cancer, thyroid cancer, lymphoma, pancreatic cancer, liver cancer, melanoma, skin cancer, pituitary tumor, germ cell tumor, meningioma, meningeal carcinoma, glioblastoma, various astrocytomas, various oligodendrogliomas, various ependymomas, choroid plexus papilloma, choroid plexus carcinoma, chordoma, various ganglioneuroma, olfactory neuroblastoma, sympathetic nervous system neuroblastoma, pineal cell tumor, pineal blastoma, medulloblastoma, trigeminal schwannoma, acoustic neuroma, glomus jugular, angioreticuloma, craniopharyngioma, and/or granulosa cell tumor.

16. The use according to claim 14, wherein the gene related genetic disorder is Huntington's disease, fragile X syndrome, phenylketonuria, pseudohypertrophic progressive muscular dystrophy, mitochondrial encephalomyopathy, spinal muscular atrophy, Parkinson plus syndrome, albinism, red-green color blindness, achondroplasia, alkaptonuria, congenital deafness, thalassemia, sickle cell anemia, hemophilia, epilepsy associated with genetic alterations, myoclonus, dystonia, stroke and schizophrenia, vitamin D-resistant rickets, familial colon polyposis, and/or hereditary nephritis.

17. The use according to claim 14, wherein the neurodegenerative disease is Parkinson's disease, Alzheimer's disease, Huntington's disease, amyotrophic lateral sclerosis, spinocerebellar ataxia, multiple system atrophy, primary lateral sclerosis, Pick's disease, frontotemporal dementia, dementia with Lewy bodies and/or progressive supranuclear palsy.
